(19) Europäisches Patentamt
European Patent Office
Office européen des brevets

(11) **EP 3 011 949 B1**

## (12) EUROPÄISCHE PATENTSCHRIFT

(45) Veröffentlichungstag und Bekanntmachung des Hinweises auf die Patenterteilung:
**05.05.2021 Patentblatt 2021/18**

(21) Anmeldenummer: **15190550.2**

(22) Anmeldetag: **20.10.2015**

(51) Int Cl.:
*A61K 6/16* (2020.01)     *A61K 6/17* (2020.01)
*A61K 6/71* (2020.01)     *A61K 6/889* (2020.01)

(54) **HÄRTBARES DENTALMATERIAL**

CURABLE DENTAL MATERIAL

MATERIAU DENTAIRE DURCISSABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.10.2014 DE 102014221603**

(43) Veröffentlichungstag der Anmeldung:
**27.04.2016 Patentblatt 2016/17**

(73) Patentinhaber: **VOCO GmbH**
**27472 Cuxhaven (DE)**

(72) Erfinder:
• **Willner, Alexander**
**27474 Cuxhaven (DE)**
• **Plaumann, Manfred Thomas**
**27472 Cuxhaven (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
**DE-A1-102004 017 124     US-A1- 2010 041 789**
**US-A1- 2012 082 954     US-B2- 8 039 101**

• **DATABASE WPI Week 201439 Thomson Scientific, London, GB; AN 2014-K87600 XP002755276, & WO 2014/083842 A1 (KURARAY NORITAKE DENTAL INC) 5. Juni 2014 (2014-06-05)**

Bemerkungen:
Die Akte enthält technische Angaben, die nach dem Eingang der Anmeldung eingereicht wurden und die nicht in dieser Patentschrift enthalten sind.

EP 3 011 949 B1

**Beschreibung**

Die vorliegende Erfindung betrifft ein härtbares Dentalmaterial, ein daraus durch Polymerisation herstellbares gehärtetes Dentalmaterial, ein Verfahren zur Herstellung des härtbaren bzw. gehärteten Dentalmaterials und die Verwendung des härtbaren bzw. gehärteten Dentalmaterials. Das härtbare bzw. gehärtete Dentalmaterial kann insbesondere als Zahnfüllungsmaterial, Dentalzement, dentales Unterfüllungsmaterial, fließfähiges Kompositmaterial (Flow-Material), Kronen- und Brückenmaterial, zur Herstellung von Inlays, Onlays und Stumpfaufbaumaterialien eingesetzt werden.

In der zahnärztlichen Praxis ist es oft von entscheidender Bedeutung, nicht-natürliches (d.h. künstliches) Dentalmaterial eines zuvor behandelten Zahns vom verbliebenen natürlichen Zahnmaterial eindeutig unterscheiden zu können. Eine solche Unterscheidung ist beispielsweise mittels einer Röntgenaufnahme möglich. Anhand einer Röntgenaufnahme können beispielweise in der Füllungstherapie Randspalten erkannt werden. Der Zahnarzt wird insbesondere in die Lage versetzt, bereits kleine Randspalten zwischen einem Füllungskomposit (als Beispiel eines nicht-natürlichen, d.h. künstlichen Dentalmaterials) und dem umgebenden natürlichen Zahnmaterial zu identifizieren und, falls notwendig, genauestens zu excavieren. Hierzu ist es jedoch notwendig, dass das Füllungskomposit (und ganz generell künstliche Dentalmaterialien) eine ausreichend hohe Röntgenopazität (auch als Röntgensichtbarkeit bezeichnet) besitzt, um während der Röntgenaufnahme Röntgenstrahlen ausreichend stark zu absorbieren. Diese Absorption sorgt in einem Röntgenbild für den notwendigen Kontrast, um zwischen natürlichem Zahnmaterial einerseits und Füllungskomposit (bzw. künstlichem Dentalmaterial) andererseits unterscheiden zu können. Das Füllungskomposit (bzw. ganz generell Zahnbestandteile aus künstlichem Dentalmaterial) ist in einem Röntgenbild regelmäßig anhand eines geringeren Schwärzegrades erkennbar. Eine hinreichende Röntgenopazität eines künstlichen Dentalmaterials erlaubt somit sehr häufig eine sichere Unterscheidung zwischen künstlichem Dentalmaterial und natürlichem Zahnmaterial.

Die natürliche Röntgenopazität eines menschlichen Zahns beträgt üblicherweise 2 mm Aluminium (Al) oder weniger (Dentin ca. 1,5 mm Al, Zahnschmelz ca. 2 mm Al). Daher sollte ein röntgenopakes, künstliches Dentalmaterial generell mindestens einen Wert größer 2,5 mm Al besitzen. Beispielsweise bedeutet ein Wert von 10 mm Al, dass ein 1 mm dicker Probekörper aus röntgenopakem, künstlichem, gehärtetem Dentalmaterial zu einer Schwärzung auf einem Röntgenfilm führt, die identisch ist mit der Schwärzung, die durch einen Probekörper aus Aluminium hervorgerufen wird, der eine Dicke von 10 mm besitzt (zur genaueren Bestimmung der Röntgenopazität weiter unten im Text). Besonders bevorzugte Röntgenopazitäten liegen in einem Bereich zwischen 3,0 und 5,0 mm Al. Ein künstliches Dentalmaterial (z.B. ein Füllungskomposit) kann in einem Röntgenbild umso besser vom natürlichen Zahnmaterial unterschieden werden, je höher die Röntgenopazität des künstlichen Dentalmaterials ist. Ein bekanntes, aber auch umstrittenes geeignetes künstliches Dentalmaterial ist Amalgam, dessen Röntgenopazität bis über 10 mm Aluminium (Al) betragen kann.

Auf dem Gebiet der Röntgendiagnostik wurden in den letzten Jahren erhebliche Fortschritte erzielt, die eine immer exaktere Unterscheidung zwischen künstlichem Dentalmaterial und natürlichem Zahnmaterial erlauben. In den letzten Jahren hat sich insbesondere ein Verfahren in den zahnärztlichen Praxen etabliert, das als Digitale Volumentomographie (DVT) bekannt ist. Ein DVT-Gerät besteht im Wesentlichen aus einer Röntgenquelle und einem gegenüberliegenden Detektor (z.B. Flatpanel-Detektor). Bei der DVT wird ein Untersuchungsobjekt von einem konusförmigen oder pyramidalen, meist gepulsten Röntgenstrahl (Röntgenblitz) durchdrungen. Daher hat sich im englischen Sprachraum die Bezeichnung CBCT (cone beam computer tomography) durchgesetzt. Auf der der Röntgenquelle gegenüberliegenden Seite werden die durch das Untersuchungsobjekt abgeschwächten Signale als zweidimensionale Projektion auf dem Detektor detektiert. Während der Untersuchung rotiert die Einheit aus Röntgenquelle und Detektor (Gantry) um das Untersuchungsobjekt, um eine Vielzahl von Einzelaufnahmen zu erstellen. In jeder Einzelaufnahme werden abgeschwächte Grauwerte-Röntgenbilder als 2D-Projektion gewonnen. Aus diesen Einzelaufnahmen wird mittels Rückprojektion eine dreidimensionale Rekonstruktion (Grauwert-Koordinatenbild, Volumengrafik) errechnet, die die anatomischen Strukturen des Untersuchungsobjektes in Form von Voxeln unterschiedlicher Graustufen abbildet. Betrachtet werden kann die dreidimensionale Rekonstruktion entweder in Form einzelner, zweidimensionaler Schnittbilder (Tomogramme) oder in einer 3D-Ansicht.

Als besonders vorteilhaft haben sich DVT-generierte Bilder in der dentalen Implantologie erwiesen. Sie erlauben eine besonders sorgfältige Planung von Implantaten und deren Einsetzung unter Berücksichtigung des zur Verfügung stehenden Knochenangebots. Auch bei der Lokalisation von Weisheitszähnen in Vorbereitung chirurgischer Maßnahmen haben sich DVT-generierte Bilder als sehr zuverlässig erwiesen.

Ähnlich wie in der klassischen Röntgendiagnostik spielt auch in der DVT die Röntgenopazität eine entscheidende Rolle bei der Unterscheidung zwischen künstlichem Dentalmaterial und natürlichem Zahnmaterial. Es ist somit ein ständiges Bedürfnis, die Röntgenopazität härtbarer Dentalmaterialien, die mittels Polymerisation polymerisierbarer Monomere zu gehärteten, künstlichen Dentalmaterialien weiterverarbeitet werden, vorteilhaft einzustellen.

Es stehen hierzu insbesondere zwei Methoden zur Verfügung:

Methode I: Der Einsatz polymerisierbarer Monomere in härtbaren Dentalmaterialien, wobei die Monomere mittels kovalenter Bindungen mit Röntgenstrahlen-absorbierenden Atomen/Verbindungen verknüpft sind.

Methode II: Der Einsatz härtbarer Dentalmaterialien, die röntgenopake, anorganische Füllstoffe (z.B. Oxide und/oder Carbonate ausgewählter Metalle) umfassen.

Gemäß Methode I werden in einem härtbaren Dentalmaterial insbesondere polymerisierbare Monomere eingesetzt, die kovalent mit z.B. Halogenatomen (z.B. Bromatomen) verbunden sind, so dass die gewünschte Röntgenopazität in einem entsprechenden künstlichen Dentalmaterial erzielt wird. Beispiele hierzu werden in der internationalen Patentanmeldung mit der Veröffentlichungsnummer WO 82/01006 A1 offenbart. WO 82/01006 A1 betrifft Homo- oder Copolymere von (Meth)acrylatestern, umfassend kovalent gebundene Röntgenstrahlen-absorbierende Atome ("homo- or copolymer of a (meth)acrylate ester into which atoms capable of absorbing x-radiation are incorporated by covalent bonding", WO 82/01006 A1, Anspruch 1), wobei diese Atome Halogenatome einschließen ("the atoms comprise halogen atoms capable of absorbing x-radiation", WO 82/01006 A1, Anspruch 2) bzw. die Halogenatome schließen Bromatome ein ("the halogen atoms capable of absorbing x-radiation comprise bromine atoms", WO 82/01006 A1, Anspruch 3).

Gegenstand der Veröffentlichung DE 35 02 594 A1 ist ein "Röntgenopaker Dentalwerkstoff" (Titel).

Gegenstand der Veröffentlichung DE 19617 931 A1 ist ein "Gefülltes und polymerisierbares Dentalmaterial" (Titel).

DE 10 2004 017 124 A1 betrifft "härtbare Dentalmaterialien mit einer einstellbaren Transluzenz und hohen Opaleszenz" (Titel).

EP 2 436 363 A2 betrifft eine "Zusammensetzung umfassend ein Monomer mit einem polyalicyclischen Strukturelement zum Füllen und/oder Versiegeln eines Wurzelkanals" (Titel). Die Zusammensetzung ist hierbei eine "härtbare dentale Zusammensetzung bestehend aus oder umfassend: (a) eine Monomerenkomponente [...], (b) ein oder mehrere Initiatoren und/oder Katalysatoren, und (c) eine Füllstoffkomponente".

Die deutsche Patentanmeldung mit der Veröffentlichungsnummer DE 21 21 480 A1 und dem Titel "In Monomerem lösliche röntgenstrahlenundurchlässige Methacrylatteilchen" "befaßt sich mit feinen, körnigen Methacrylatteilchen in Perlenform, wobei aliphatische Halogenide durch die Perlen verteilt sind, um ein röntgenstrahlenundurchlässiges Material herbeizuführen". Es heißt in DE 21 21 480 A1 weiter: "Die aliphatischen Halogenidverbindungen enthalten entweder Brom oder Jod. Um den günstigen körnigen Methacrylatkunststoff zu erhalten, ist es erforderlich, daß die spezielle Halogenidkomponente wenigstens etwa 50 Gew.-% des aliphatischen Halogenidmoleküls umfaßt" (DE 21 21 480 A1, Seite 8, erster vollständiger Absatz).

Die deutsche Patentanmeldung mit der Veröffentlichungsnummer DE 41 11 914 A1 betrifft "einen Werkstoff aus Kunststoff mit röntgenabsorbierendem Comonomer". Gemäß Anspruch 1 in DE 41 11 914 A1 ist der "Werkstoff aus Kunststoff mit covalent gebundenem Röntgenkontrastmittel".

Die europäische Patentanmeldung mit der Veröffentlichungsnummer EP 0 685 454 A1 betrifft "röntgenopake Ester und Amide iodsubstituierter Benzoesäure [...] sowie daraus hergestellte Polymere und Dentalmaterialien" (EP 0 685 454 A1, Seite 2, Zeilen 1 und 2). Die in EP 0 685 454 A1 offenbarten Dentalmaterialien eignen sich als "Füllungskomposites, Befestigungszemente, Haftvermittler (Bondings) sowie zur Herstellung künstlicher Zähne, Inlays, Implantate, Kronen, Brücken und Fertigteilen, vorzugsweise als Zahnfüllmaterial, Befestigungszement oder Bonding" (EP 0 685 454 A1, Seite 4, Zeilen 26 bis 29).

US 2012/082954 A1 betrifft gemäß Anspruch 1 eine härtbare Dentalzusammensetzung umfassend oder bestehend aus (a) einer Monomerkomponente, (b) einem oder einer Vielzahl von Initiatoren und/oder Katalysatoren, und (c) einer Füllstoffkomponente.

Härtbare Dentalmaterialien, die gemäß Methode I hergestellt werden, besitzen jedoch regelmäßig auch Nachteile.

Neben recht aufwändigen Syntheseverfahren zur Darstellung der entsprechenden polymerisierbaren Monomere mit kovalent verknüpften, Röntgenstrahlen-absorbierenden Atomen/Verbindungen sind in der Regel hohe Anteile an Röntgenstrahlen-absorbierenden Atomen/Verbindungen in den polymerisierbaren Monomeren notwendig, um eine ausreichende Röntgenopazität zu erzielen. Dies führt jedoch oft zu Schwierigkeiten bei der radikalischen Polymerisation der polymerisierbaren Monomere, da der chemische Einbau von Elementen mit hohen Atomgewichten (z.B. Iod, Brom, usw.) häufig zu Kettenabbrüchen beim Aufbau der Polymerisate (d.h. der gehärteten Dentalmaterialien) führt. Dies hat zur Folge, dass die Polymerisationsneigung solcher polymerisierbaren Monomere häufig sehr gering ist und daraus hergestellte gehärtete Dentalmaterialien häufig einen niedrigen Polymerisationsgrad besitzen. Dies führt regelmäßig zu schlechten mechanischen Eigenschaften im gehärteten Dentalmaterial.

Wie bereits vorstehend gezeigt, werden als Röntgenstrahlen-absorbierende Atome/Verbindungen unter anderem organische Halogenverbindungen eingesetzt, die häufig die Elemente Iod und/oder Brom umfassen. Diese Verbindungen sind jedoch oft wenig stabil, wenig kompatibel mit den weiteren Bestandteilen eines härtbaren Dentalmaterials und neigen dazu, das gehärtete Dentalmaterial zu verfärben. Es wurde außerdem in eigenen Experimenten beobachtet, dass solche Verbindungen dazu neigen, aus der polymeren Matrix eines gehärteten Dentalmaterials herauszuwandern, was häufig zu einer Schwächung des mechanischen Verbundes führt.

Gemäß Methode II werden röntgenopake, anorganische Füllstoffe (beispielsweise Oxide und/oder Carbonate des Lanthans, Strontiums, Hafniums oder Tantals sowie Halogensalze des Ytterbiums) eingesetzt.

Die deutsche Patentanmeldung mit der Veröffentlichungsnummer DE 24 58 380 A1 und dem Titel "Zahnfüllmasse" "betrifft Zahnfüllmassen für die zahnärztliche Ausbesserungspraxis, wie Füllungen, Kitte, Einlagen und dergleichen". Auf Seite 3, zweiter vollständiger Absatz heißt es in DE 24 58 380 A1: "Die Füllmassen gemäss der Erfindung enthalten nicht-toxische, Röntgenstrahlen absorbierende Atome, in erster Linie Lanthan, Strontium und Tantal, und in zweiter Linie Hafnium, in Form von Oxiden oder Carbonaten."

Die deutsche Patentanmeldung mit der Veröffentlichungsnummer DE 23 47 591 A1 und dem Titel "Glaskeramik als Füllstoff für polymerisierende Zahnfüllmassen" offenbart eine "[f]arblose, transparente Glaskeramik [...] mit hoher Absorption für Röntgenstrahlen, zur Verwendung in Kunststoff-Glaskeramik-Mischkörpern für Zahnfüllmassen [...]" (DE 23 47 591 A1, Anspruch 1). Als Glaskeramikmaterialien werden offenbart: $SiO_2$, $Al_2O_3$, $Li_2O$, $P_2O_5$, MgO, ZnO, $ZrO_2$, $Ta_2O_5$, $La_2O_3$.

Die US-Patentanmeldungen mit den Veröffentlichungsnummern US 3,801,344 A und US 3,808,170 A betreffen ein Zahnfüllmaterial umfassend eine Mischung aus fein verteilten anorganischen Materialien und röntgenopaken Glasfüllstoffen ("tooth filling and facing composition having a mixture of finely divided inorganic material and a radiopaque glass as a filler and an organic polymer as a binder" bzw. ein "epoxy resin derived from bisphenol A and a methacrylic acid compound crosslinked with a diester or triester of methacrylic acid as the matrix, and a bariumcontaining glass filler"). Beide Dokumente offenbaren den Einsatz von Bariumoxid zur Erzielung röntgenopaker Eigenschaften.

Die europäische Patentanmeldung mit der Veröffentlichungsnummer EP 0 011 735 A2 und dem Titel "Röntgenopake Dentalwerkstoffe auf Basis von organischen Kunststoffen in pastöser Form" betrifft "[r]öntgenopake Dentalwerkstoffe auf Basis von organischen Kunststoffen in pastöser Form, [die] [...] aus a) polymerisierbaren Bindern, b) vernetzten Polymerisaten, c) Röntgenkontrastmitteln und gegebenenfalls d) feinteiligen, nicht röntgenopaken anorganischen Füllstoffen bestehen" (EP 0 011 735 A2, Anspruch 1). Bezüglich der Röntgenkontrastmittel heißt auf Seite 8, Zeilen 18 bis 25: "Besonders gut eignen sich Bariumverbindungen, z.B. Bariumsulfat, Bariumfluorid und Bariumsilikat. Aber auch Verbindungen von Wismut, z.B. Wismutoxynitrat, Zirkon, z.B. Zirkondioxid und Lanthan, z.B. Lanthanoxid, sowie Verbindungen von Thorium und den seltenen Erdmetallen sind geeignet. Darüber hinaus können anorganische und organische Jodverbindungen als Röntgenkontrastmittel verwendet werden."

Die europäische Patentanmeldung mit der Veröffentlichungsnummer EP 0 189 540 A2 und dem Titel "Röntgenopaker dentalwerkstoff" betrifft "röntgenopake Dentalwerkstoffe wie z.B. Füllungsmaterialien, Dentalzemente, Kronen- und Brückenmaterialien, Prothesenmaterialien sowie deren Verwendung zur Herstellung von künstlichen Zähnen, Inlays, Implantaten und Fertigteilen" (EP 0 189 540 A2, Seite 1, Zeilen 1 bis 5). Der in EP 0 189 540 A2 offenbarte röntgenopake Dentalwerkstoff ist dadurch gekennzeichnet, dass "er ein Fluorid der Seltenen Erdmetalle (Elemente 57 bis 71) [auch als SE-Fluoride bezeichnet] des Periodensystems der Elemente) oder ein Gemisch dieser Fluoride enthält (EP 0 189 540 A2, Anspruch 1). Besonders bevorzugt ist Ytterbiumtrifluorid (EP 0 189 540 A2, Anspruch 3). Auf Seite 3, Zeilen 30 bis 36 heißt es: "Die mittlere Korngröße der Primärteilchen kann dabei schwanken. Bei einem mikrogefüllten Zahnfüllungsmaterial liegt sie im Bereich von 5 bis 700, insbesondere 20 bis 500, vorzugsweise 50 bis 300 nm. Gegebenenfalls kann die mittlere Primärteilchengröße auch im Bereich von 700 nm bis 5 $\mu$m liegen. Es heißt außerdem: "Der Gehalt an SE-Fluoriden, bezogen auf das Gesamtgewicht, beträgt zwischen 1 und 50 %, insbesondere 5 bis 40 %, vorzugsweise liegt er zwischen 10 und 25 %" (EP 0 189 540 A2, Seite 4, Zeilen 1 bis 3).

Die europäische Patentanmeldung mit der Veröffentlichungsnummer EP 2 548 546 A1 und dem Titel "Röntgenopaker Infiltrant" betrifft einen "Infiltranten für die Dentalapplikation, der vernetzende Monomere enthält sowie dessen Verwendung zur Prävention bzw. Behandlung (Versiegelung) käriöser Schmelzläsionen" (Absatz [0001] in EP 2 548 546 A1). Anspruch 1 der EP 2 548 546 A1 offenbart einen "Infiltrant[en] für die Dentalapplikation, der vernetzende Monomere und Initiator enthält und gemessen bei 23°C eine dynamische Viskosität von 50 mPas oder weniger aufweist, dadurch gekennzeichnet, dass der Infiltrant mindestens einen nanoskaligen röntgenopaken Füllstoff und/oder eine röntgenopake organische Verbindung aufweist". In Absatz [0024] wird offenbart: "Unter den Salzen der Seltenerdmetalle (Elemente 57-71), des Scandiums oder des Yttriums sind die Trifluoride bevorzugt. Zu den bevorzugten Seltenerdmetallen zählen Lanthan, Cer, Samarium, Gadolinium, Dysprosium, Erbium oder Ytterbium. Unter deren Salzen sind die Fluoride bevorzugt, insbesondere Ytterbiumtrifluorid (YbF3). Bevorzugte Barium- und Strontiumsalze sind Fluoride, Phosphate und Sulfate, insbesondere die Sulfate."

Gemäß Absatz [0028] in EP 2 548 546 A1 weisen die nanoskaligen röntgenopaken Füllstoffe "vorzugsweise folgende mittlere Primärpartikelgrößen $d_{50}$ bzw. Bereiche dieser Partikelgrößen auf:

- kleiner 1000 nm, kleiner 700 nm, kleiner 500 nm, kleiner 200 nm, kleiner 100 nm, kleiner 25 nm,

- zwischen 1 nm und 80 nm, zwischen 4 nm und 60 nm, zwischen 6 nm und 50 nm, zwischen 0,5 nm und 22 nm, zwischen 1 nm und 20 nm, zwischen 1 nm und 10 nm oder zwischen 1 nm und 5 nm."

Gemäß Absatz [0029] heißt es in EP 2 548 546 A1: "Besonders bevorzugt sind vereinzelt vorliegende, nicht aggregierte und nicht agglomerierte nanoskalige Füllstoffe. Weiterhin bevorzugt sind Füllstoffe mit unimodaler Teilchengrößenverteilung."

Gemäß Anspruch 8 der EP 2 548 546 A1 beträgt "der Gehalt des nanoskaligen röntgenopaken Füllstoffs im Infiltranten bezogen auf die Gesamtmasse des Infiltranten mit allen Inhaltsstoffen 1 Gew.-% bis 30 Gew.-%, vorzugsweise 5 Gew.-% bis 25 Gew.-%, weiter vorzugsweise 10 Gew.-% bis 20 Gew.-% oder 1 Gew.-% bis 5 Gew.-%, 5 Gew.-% bis 10 Gew.-%, 10 Gew.-% bis 15 Gew.-% oder 15 Gew.-% bis 20 Gew.% [...]".

Die internationale Patentanmeldung mit der Veröffentlichungsnummer WO 2005/011621 A1 und dem Titel "Röntgenopakes Dentalmaterial mit oberflächenmodifizierten Nanopartikeln" "betrifft ein röntgenopakes Dentalmaterial, [...] das oberflächenmodifizierte Salze der Seltenerdmetalle, des Scandiums, Yttriums, Bariums oder Strontiums oder ein Wolframat enthält, und seine Verwendung in der Dentaltechnik" (WO 2005/011621 A1, Seite 1, erster vollständiger Absatz). Gemäß Anspruch 1 der WO 2005/011621 A1 enthält das Dentalmaterial "mindestens einen Füllstoff [...], der unter Salzen des Bariums, Strontiums, der Seltenerdmetalle, des Scandiums oder Yttriums oder unter Wolframaten ausgewählt wird, [wobei] [...] der Füllstoff in Form oberflächenmodifizierter Nanopartikel mit einer mittleren Teilchengröße von weniger als 25 nm vorliegt, deren Oberfläche mit einer organischen Verbindung modifiziert ist, die mit einer N-, P-, S- und/oder O-haltigen funktionellen Gruppe an die Nanopartikel bindet". Gemäß Seite 4, vorletzter vollständiger Absatz heißt es in WO 2005/011621 A1: "Zu den bevorzugten Seltenerdmetallen zählen Lanthan, Cer, Samarium, Gadolinium, Dysprosium, Erbium oder Ytterbium. Unter deren Salzen sind die Fluoride bevorzugt, insbesondere Ytterbiumtrifluorid ($YbF_3$)."

Gemäß Seite 10 in WO 2005/011621 A1, letzter Absatz handelt es sich bei den oberflächenmodifizierenden Verbindungen vorzugsweise "um eine phosphororganische Verbindung oder ein mono- oder disubstituiertes Amin". Im vorletzten Absatz auf Seite 10 wird offenbart, dass sich diese oberflächenmodifizierende organische Verbindung "nicht nur positiv auf die spätere Dispergierung in einem Dentalmaterial [auswirkt]. Sie zeigt zusätzlich eine das Kristallwachstum begrenzende Wirkung und führt zu den zuvor angegebenen geringen Teilchengrößen von weniger als 25nm in enger Teilchengrößenverteilung".

Die deutsche Patentanmeldung mit der Veröffentlichungsnummer DE 36 09 038 A1 und dem Titel "Röntgenopake polymerisierbare Dentalmassen" "betrifft neue röntgenopake polymerisierbare Dentalmassen, insbesondere Zahnfüllmassen" (DE 36 09 038 A1, Seite 2, Zeile 35). Diese enthalten gemäß Anspruch 1 in DE 36 09 038 A1 "ein schwerlösliches Schwermetallfluorid aus der Gruppe $YF_3$ und komplexe Schwermetallfluoride der allgemeinen Formel $M^{II}M^{IV}F_6$ [...], wobei M" ein Calcium-, Strontium- oder Bariumion und $M^{IV}$ ein Titan-, Zirkon- oder Hafniumion bedeutet". Gemäß Anspruch 5 in DE 36 09 038 A1 ist ein besonders bevorzugtes schwerlösliches Schwermetallfluorid $YF_3$.

Die deutsche Patentanmeldung mit der Veröffentlichungsnummer DE 198 46 556 A1 "richtet sich auf einen Dentalwerkstoff sowie ein Verfahren zu dessen Herstellung. Ebenso betrifft die Erfindung poröse Glaskeramiken, ein Verfahren zu deren Herstellung und Verwendung" (DE 198 46 556 A1, Seite 2, Zeilen 3 und 4). Gemäß Anspruch 1 in DE 198 46 556 A1 heißt es: "Dentalwerkstoff auf Basis [...] eines anorganischen Füllstoffs (A) [...] dadurch gekennzeichnet, daß der anorganische Füllstoff (A) eine poröse Glaskeramik ist, deren Mikro- und/oder Mesoporen mit polymerisierbaren, ethylenisch ungesättigten Monomeren, Epoxiden, Ormoceren, flüssigkristallinen Monomeren, Oxethanen, Spiroorthoester bzw. -carbonaten oder mit deren polymerisierter Form beladen sind". Gemäß Anspruch 5 der DE 198 46 556 A1 "[enthält] der Füllstoff (A) Oxide der Metalle der 1. bis 4. Hauptgruppe sowie Oxide der Metalle der Nebengruppen [...]". Bevorzugt einzusetzende Oxide sind $TiO_2$, $ZrO_2$, $BaO$ und $WO_3$, wobei $ZrO_2$ ganz besonders bevorzugt ist (DE 198 46 556 A1, Seite 5, Zeilen 32 und 33).

Das US-Patent mit der Veröffentlichungsnummer US 8,476,338 B2 betrifft ein Dentalkomposit ("a dental composite resin, that can be used as a substitute for a part of a natural tooth or an entire natural tooth in the field of dental treatment" (US 8,476,338 B2, Spalte 1, Zeilen 10 bis 14). Gemäß Anspruch 7 der US 8,476,338 B2 umfasst das Komposit neben einem polymerisierbaren Monomer (A) anorganische Partikel (B), anorganische Partikel (C) und ultra-feine anorganische Partikel (D) ("a polymerizable monomer (A)", "inorganic particles (B)", "inorganic particles (C)" and "inorganic ultrafine particles (D)").

Auch gemäß Methode II hergestellte härtbare Dentalmaterialien besitzen regelmäßig Nachteile.

In der Regel wird zwar mit zunehmendem Anteil an röntgenopaken, partikulären anorganischen Füllstoffen eine zunehmende Röntgenopazität beobachtet. Eine zufriedenstellende Röntgenopazität wird jedoch häufig erst bei einem vergleichsweise hohen Anteil röntgenopaker anorganischer Füllstoffe erzielt. Ein vergleichsweise hoher Anteil solcher partikulärer Füllstoffe führt jedoch häufig in gehärteten Dentalmaterialen zu einer erhöhten visuellen Opazität (d.h. zu einer vergleichsweise geringen Transluzenz). Diese vergleichsweise geringe Transluzenz führt dazu, dass das gehärtete Dentalmaterial ästhetisch nicht mehr optimal dem Erscheinungsbild des umgebenden natürlichen Zahnmaterials angepasst ist. Außerdem führt eine nicht ausreichende Transluzenz bei der lichtinitiierten Polymerisation häufig zu einer begrenzten (unzureichenden) Aushärtungstiefe / Durchdringungstiefe des Lichtes. Das für die Polymerisation benötigte Licht dringt nicht mehr genügend tief in das härtbare Dentalmaterial ein, so dass das Material nur unvollständig polymerisiert. Dies kann wiederum zu fehlerhaften Restaurationen führen. In gut eingestellten Dentalmaterialien führt der Anteil röntgenopaker anorganischer Füllstoffe in einem gehärteten Dentalmaterial mit einer als akzeptabel geltenden Röntgenopazität häufig zu einer noch akzeptablen Verminderung der Transluzenz. Die Transluzenz des gehärteten Dentalmaterials hängt in entscheidender Weise zusätzlich von den Brechungsindizes der organischen und der anorganischen Bestandteile ab. Allgemein gilt: Je enger diese Brechungsindizes beieinander liegen, desto transluzenter ist das ausgehärte Dentalmaterial. Optimal wäre somit ein Gemisch, in dem die anorganischen Stoffe und die organischen Stoffe identische Brechungsindices aufweisen. Üblicherweise liegen die Brechungsindizes der organischen Bestandteile (z.B. die polymerisierten Monomere) jedoch im Bereich von 1,45 bis 1,55, während die Brechungsindizes der anorganischen Bestandteile (z.B. die Röntgenstrahlen-absorbierenden anorganischen Füllstoffe) in einem Bereich größer 1,55 liegen. Dies hat zur Folge, dass zur Herstellung ausreichend transluzenter, röntgenopaker Dentalmaterialien komplexe Anpassungen hinsichtlich der Brechungsindizes der Bestandteile vorgenommen werden müssen.

In einer Vielzahl röntgenopaker Dentalmaterialien werden (häufig zusätzlich zu den vorstehend genannten röntgenopaken, anorganischen, partikulären Füllstoffen) röntgenopake Dentalgläser verarbeitet (umfassend Schweratome), die häufig den Hauptanteil der Füllstoffmenge ausmachen. Elemente höherer Ordnungszahlen (Schweratome) in den dentalen Gläsern bewirken eine höhere Röntgenopazität, verglichen mit dentalen Gläsern, die keine Elemente höherer Ordnungszahlen enthalten. Die röntgenopaken Dentalgläser ermöglichen in der Regel eine ausreichende Transluzenz bei akzeptabler Röntgenopazität. Wünschenswert wäre jedoch auch hier eine äußerst hohe Transluzenz bei sehr guter Röntgenopazität. Röntgenopake Dentalmaterialien auf Basis röntgenopaker Dentalgläser weisen allerdings ebenfalls einige Nachteile auf.

Zum einen sind Dentalgläser hydrolyseanfällig, so dass regelmäßig eine Gefahr besteht, dass Ionen aus den Dentalgläsern (im gehärteten Dentalmaterial) in den Mundraum übertreten. Dies kann neben dem Abbau des gehärteten Dentalmaterials auch zu toxikologischen Schäden beim Menschen führen.

Zum anderen hat sich in einigen Fällen gezeigt, dass mit Dentalgläsern gefüllte, gehärtete Dentalmaterialien vergleichsweise weniger abriebfest ein können, z.B. verglichen mit einem gehärteten Dentalmaterial auf Basis von Quarz. Dies liegt an der geringeren Härte üblicher Dentalgläser im Vergleich mit Quarz.

Des Weiteren wurde in Einzelfällen beobachtet, dass sich mit Dentalglas gefüllte Dentalmaterialien gar nicht oder nur unzureichend hochglanzpolieren lassen, bedingt durch die Partikelgrößen üblicher Dentalgläser. So wurde in diesen Fällen beobachtet, dass während des Polierens insbesondere voluminöse Füllstoffpartikel aus einer Füllung ausbrechen. Dies führt dazu, dass kleine Löcher zurückbleiben. Zusätzlich üben die ausgebrochenen Füllstoffpartikel einen Schmirgeleffekt auf die umliegende Oberfläche aus, so dass sich in diesen Fällen mit Dentalgläsern gefüllte Dentalmaterialien kaum hochglanzpolieren lassen und ästhetische Defizite aufweisen. Hochglanzpolierte, gehärtete Dentalmaterialien werden üblicherweise insbesondere dann erreicht, wenn partikuläre Füllstoffe eingesetzt werden, deren mittlere Teilchengröße ca. 0,04 $\mu$m beträgt.

Auch in der bereits oben beschriebenen DVT zeigen bestimmte gehärtete Dentalmaterialien bessere Ergebnisse als andere. Wie bei jedem bildgebenden Verfahren kommt es auch bei der DVT zu Bildstörungen und insbesondere zu sogenannten Artefakten. Ein Bildartefakt ist eine im erzeugten Bild sichtbare Struktur, die keine Entsprechung bzw. keine entsprechende Struktur im Untersuchungsobjekt hat. Die Ursachen der Störungen und Artefakte sind vielfältig und betreffen sowohl die Datenaufnahme der Primärdaten (Artefakte werden z.B. hervorgerufen durch Detektorrauschen, Streuung der Röntgenstrahlen, Bewegung des Patienten) als auch die mathematischen Bildrekonstruktionen nach der Datenaufnahme. Artefakte werden in DVT-Bildern hauptsächlich in Form von Streifen, linearen Strukturen und Schatten beobachtet, besonders häufig in Nachbarschaft zu künstlichen Dentalmaterialien bzw. zahnärztlichen Rekonstruktionen. Die betroffenen künstlichen Dentalmaterialien bzw. zahnärztlichen Rekonstruktionen bestehen in der Regel aus Metall (Titan, Gold, Amalgam, usw.) oder enthalten Verbindungen umfassend Elemente höherer Ordnungszahlen (z.B. Barium, Ytterbium, usw.), so dass sie eine gewisse Röntgenopazität besitzen. Gerade diese für die klassische Röntgendiagnostik wichtige Röntgenopazität, d.h. die Fähigkeit, Röntgenstrahlen abzuschwächen, begünstigt auch die Entstehung von Auslöschungs- und Strahlaufhärtungsartefakten in der DVT (Strahlaufhärtungsartefakten entstehen durch Detektion kurzwelliger, energiereicher Röntgenstrahlung, die neben langwelliger, energiearmer Röntgenstrahlung im Röntgenstrahlspektrum enthalten ist und nur wenig an röntgenopaken Strukturen absorbiert wird und somit ungehinderter den Detektor erreicht, verglichen mit der energiearmen Röntgenstrahlung). In der Praxis zeigen sich diese Artefakte in DVT-Bildern insbesondere in Form von sogenannten hyper- oder hypodensen Bereichen, die leicht als Sekundärkaries fehlinterpretiert werden können (für genaue Erläuterungen der Artefakte siehe: R. Schulze et al., "Artefacts in CBCT: a review", Dentomaxillofacial Radiology, 40, 265-273 (2011)). Es wird vermutet, dass härtbare Dentalmaterialien, die speziell auf die Bedürfnisse der DVT gerichtet sind, zu vergleichsweise weniger Artefakten in einem DVT-Bild führen.

Es besteht somit ein dringendes Bedürfnis, härtbare bzw. gehärtete Dentalmaterialien zur Verfügung zu stellen, die

- auf einfache Art und Weise herstellbar sind,

- eine hohe Transluzenz,

- eine hohe Röntgenopazität,

- eine ansprechende Ästhetik

- und gute physikalische Eigenschaften besitzen,

- sowie insbesondere bei Anwendung in der DVT zu vergleichsweise artefaktarmen DVT-Bildern führen.

Es war daher eine Aufgabe der vorliegenden Erfindung, ein härtbares Dentalmaterial zur Verfügung zu stellen, das nach Polymerisation polymerisierbarer Monomere (d.h. im gehärteten Zustand) und während einer DVT-Messung zu vergleichsweise weniger Artefakten in den resultierenden DVT-Bildern führt (verglichen mit sonst üblichen, wie vorstehend aus dem Stand der Technik bekannten härtbaren Dentalmaterialien) und zusätzlich mehrere oder sämtliche der nachfolgenden Eigenschaften besitzt:

- auf einfache Art und Weise herstellbar,

- hohe Transluzenz,

- hohe Röntgenopazität,

- ansprechende Ästhetik, insbesondere eine gute Polierbarkeit,

- gute physikalische Eigenschaften, wie beispielsweise (und vorzugsweise) eine gute Biegefestigkeit und/oder eine gute Abrasionsbeständigkeit.

Diese Aufgabe wird gelöst durch ein härtbares Dentalmaterial gemäß Patentanspruch 1. Dieses Dentalmaterial ist herstellbar durch Mischen von Startmaterialien, wobei als zu mischende Startmaterialien ausschließlich eingesetzt werden:

- ein, zwei, drei oder mehr als drei polymerisierbare Monomere, welche die Gesamtmenge (A) der polymerisierbaren Monomere im härtbaren Dentalmaterial bilden,

- ein, zwei, drei oder mehr als drei partikuläre Füllstoffe, welche die Gesamtmenge (B) der partikulären Füllstoffe im härtbaren Dentalmaterial bilden,
sowie

- ein, zwei, drei oder mehr als drei Hilfsstoffe, welche die Gesamtmenge (C) der Hilfsstoffe im härtbaren Dentalmaterial bilden,

wobei
das härtbare Dentalmaterial eine der Gesamtmenge (B) zugehörige Teilmenge (B1) umfasst, bestehend aus der im härtbaren Dentalmaterial enthaltenen Gesamtmenge an

(a) nicht-aggregierten und nicht-agglomerierten Füllstoffpartikeln mit einer Partikelgröße im Bereich von 25 bis 120 nm aus Ytterbiumfluorid
und

(b) nicht-aggregierten und nicht-agglomerierten Füllstoffpartikeln mit einer Partikelgröße im Bereich von 25 bis 120 nm aus Bariumsulfat,

wobei
der Anteil der Teilmenge (B1)

- 2,5 Gew.-% oder größer als 2,5 Gew.-% ist, bezogen auf die Gesamtmasse des härtbaren Dentalmaterials

und

- 90 Gew.-% oder größer als 90 Gew.-% ist, bezogen auf die Gesamtmasse an Füllstoffpartikeln aus Ytterbiumfluorid und Bariumsulfat im härtbaren Dentalmaterial.

Es ist dabei nicht erforderlich, dass in einem erfindungsgemäßen härtbaren Dentalmaterial beide Spezies (a) und (b) enthalten sind. In der Praxis ist der Einsatz nur einer Spezies (a) oder (b) häufig vorteilhaft, siehe dazu die Ausführungen und Beispiele weiter unten. Sofern nur eine der Spezies (d.h. (a) oder (b)) in dem erfindungsgemäßen härtbaren Dentalmaterial enthalten ist, entspricht die Gesamtmenge somit der Menge dieser einzelnen Spezies (a) oder (b), die im erfindungsgemäßen härtbaren Dentalmaterial enthalten ist.

Die vorstehend und nachfolgend in Verbindung mit der erfindungsgemäßen Lehre genannten Angaben zu Partikelgrößen schließen die Bereichsgrenzen ein. Beispielsweise bedeutet eine Partikelgröße im Bereich von 25 nm bis 120 nm: im Bereich von (einschließlich) 25 nm bis (einschließlich) 120 nm.

Bei der Diskussion der erfindungsgemäßen härtbaren Dentalmaterialien (wie vorstehend beschrieben) bzw. von daraus durch Polymerisation polymerisierbarer Monomere hergestellten erfindungsgemäßen gehärteten Dentalmaterialen umfasst der Ausdruck "Ytterbiumfluorid" begrifflich "Ytterbium(III)-fluorid" und "Ytterbiumtrifluorid" (zu den erfindungsgemäßen gehärteten Dentalmaterialien siehe weiter unten im Text).

Die

(a) nicht-aggregierten und nicht-agglomerierten Füllstoffpartikel mit einer Partikelgröße im Bereich von 25 bis 120 nm aus Ytterbiumfluorid
und die

(b) nicht-aggregierten und nicht-agglomerierten Füllstoffpartikel mit einer Partikelgröße im Bereich von 25 bis 120 nm aus Bariumsulfat

sind üblicherweise kristallin; Anteile amorpher Partikel und Anteile an Partikeln mit sowohl kristallinen als auch amorphen Bereichen sind jedoch nicht ausgeschlossen. Bevorzugt (und üblicherweise) umfasst die Teilmenge (B1) somit kristalline Partikel.

Die

(a) nicht-aggregierten und nicht-agglomerierten Füllstoffpartikel mit einer Partikelgröße im Bereich von 25 bis 120 nm aus Ytterbiumfluorid
und die

(b) nicht-aggregierten und nicht-agglomerierten Füllstoffpartikel mit einer Partikelgröße im Bereich von 25 bis 120 nm aus Bariumsulfat

haben üblicherweise eine weitestgehend sphärische (kugelförmige) Gestalt. Bei der Bestimmung der zuvor genannten Partikelgröße wird kalkulatorisch davon ausgegangen, dass alle der Partikel eine sphärische (kugelförmige) Gestalt besitzen (zu Details der Bestimmung und Kalkulation siehe unten unter Beispiele, Punkte I.d bis I.f).

Bevorzugt gilt für die Gesamtmenge der nicht-aggregierten und nicht-agglomerierten Füllstoffpartikel aus Ytterbiumfluorid und der nicht-aggregierten und nicht-agglomerierten Füllstoffpartikel aus Bariumsulfat in der Gesamtmenge (B), dass die mittlere Partikelgröße dieser Füllstoffpartikel im Bereich von 25 bis 120 nm liegt.

Besonders bevorzugt gilt für die Gesamtmenge der Füllstoffpartikel aus Ytterbiumfluorid und der Füllstoffpartikel aus Bariumsulfat in der Gesamtmenge (B), dass die mittlere Partikelgröße im Bereich von 25 bis 120 nm liegt.

Sofern vorstehend oder nachfolgend in erfindungsgemäßen Ausgestaltungen von einer "mittleren Partikelgröße" die Rede ist, handelt es sich um die volumenbezogene mittlere Partikelgröße.

Bevorzugt ist somit ein erfindungsgemäßes härtbares Dentalmaterial (wie vorstehend beschrieben, vorzugsweise wie vorstehend als bevorzugt definiert), herstellbar durch Mischen von Startmaterialien, wobei als zu mischende Startmaterialien ausschließlich eingesetzt werden:

- ein, zwei, drei oder mehr als drei polymerisierbare Monomere, welche die Gesamtmenge (A) der polymerisierbaren Monomere im härtbaren Dentalmaterial bilden,

- ein, zwei, drei oder mehr als drei Füllstoffe, welche die Gesamtmenge (B) der Füllstoffe im härtbaren Dentalmaterial bilden,
sowie

- ein, zwei, drei oder mehr als drei Hilfsstoffe, welche die Gesamtmenge (C) der Hilfsstoffe im härtbaren Dentalmaterial bilden,

wobei
das härtbare Dentalmaterial eine der Gesamtmenge (B) zugehörige Teilmenge (B1) umfasst, bestehend aus der im härtbaren Dentalmaterial enthaltenen Gesamtmenge an

(a) nicht-aggregierten und nicht-agglomerierten Füllstoffpartikeln mit einer Partikelgröße im Bereich von 25 bis 120 nm aus Ytterbiumfluorid
und

(b) nicht-aggregierten und nicht-agglomerierten Füllstoffpartikeln mit einer Partikelgröße im Bereich von 25 bis 120 nm aus Bariumsulfat,

wobei
der Anteil der Teilmenge (B1)

- 2,5 Gew.-% oder größer als 2,5 Gew.-% ist, bezogen auf die Gesamtmasse des härtbaren Dentalmaterials und
- 90 Gew.-% oder größer als 90 Gew.-% ist, bezogen auf die Gesamtmasse an Füllstoffpartikeln aus Ytterbiumfluorid und Bariumsulfat im härtbaren Dentalmaterial

und

- die Füllstoffpartikel der Gesamtmenge an Füllstoffpartikel aus Ytterbiumfluorid und Füllstoffpartikel aus Bariumsulfat in der Gesamtmenge (B) eine mittlere Partikelgröße im Bereich von 25 bis 120 nm besitzen, vorzugsweise die Füllstoffpartikel der Gesamtmenge an Füllstoffpartikel aus Ytterbiumfluorid und der Füllstoffpartikel aus Bariumsulfat in der Gesamtmenge (B) eine mittlere Partikelgröße im Bereich von 25 bis 80 nm besitzen.

Bestimmte (bevorzugte) Eigenschaften der erfindungsgemäßen härtbaren Dentalmaterialien (z.B. die Brechungsindices der Füllstoffpartikel der Teilmenge (B1)) sind am erfindungsgemäßen härtbaren Dentalmaterial selbst nur mit recht hohem Aufwand zu bestimmen, weshalb in der vorstehenden Definition unter anderem auf die eingesetzten Startmaterialien abgestellt wird. Durch (vorzugsweise sanftes) Mischen der oben genannten Startmaterialien ändern sich die Anteile und Zusammensetzungen der Gesamtmengen (A), (B) und (C) üblicherweise jedoch nicht. Ebenso wenig findet üblicherweise eine durch das (sanfte) Mischen ausgelöste Stoffumwandlung statt. Die vorliegende Erfindung betrifft deshalb auch ein härtbares Dentalmaterial, bestehend aus

- ein, zwei, drei oder mehr als drei polymerisierbaren Monomeren, welche die Gesamtmenge (A) der polymerisierbaren Monomere im härtbaren Dentalmaterial sind,

- ein, zwei, drei oder mehr als drei Füllstoffen, welche die Gesamtmenge (B) der Füllstoffe im härtbaren Dentalmaterial sind,
sowie

- ein, zwei, drei oder mehr als drei Hilfsstoffen, welche die Gesamtmenge (C) der Hilfsstoffe im härtbaren Dentalmaterial sind,

wobei
das härtbare Dentalmaterial eine der Gesamtmenge (B) zugehörige Teilmenge (B1) umfasst, bestehend aus der im härtbaren Dentalmaterial enthaltenen Gesamtmenge an

(a) nicht-aggregierten und nicht-agglomerierten Füllstoffpartikeln mit einer Partikelgröße im Bereich von 25 bis 120 nm aus Ytterbiumfluorid
und

(b) nicht-aggregierten und nicht-agglomerierten Füllstoffpartikeln mit einer Partikelgröße im Bereich von 25 bis 120 nm aus Bariumsulfat,

wobei
der Anteil der Teilmenge (B1)

- 2,5 Gew.-% oder größer als 2,5 Gew.-% ist, bezogen auf die Gesamtmasse des härtbaren Dentalmaterials und

- 90 Gew.-% oder größer als 90 Gew.-% ist, bezogen auf die Gesamtmasse an Füllstoffpartikeln aus Ytterbiumfluorid und Bariumsulfat im härtbaren Dentalmaterial.

Das vorstehend und nachfolgend zu dem erfindungsgemäßen, durch Mischen bestimmter Startmaterialien herstellbaren, härtbaren Dentalmaterial Gesagte gilt entsprechend für ein erfindungsgemäßes härtbares Dentalmaterial, welches aus den vorstehend genannten Gesamtmengen (A), (B) und (C) besteht.

In dem erfindungsgemäßen härtbaren Dentalmaterial (wie vorstehend beschrieben, vorzugsweise wie vorstehend als bevorzugt definiert), herstellbar durch Mischen von Startmaterialien, umfassen die Füllstoffe, welche die Gesamtmenge (B) der Füllstoffe im härtbaren Dentalmaterial bilden, eine Teilmenge (B1). Diese Teilmenge (B1) besteht aus der im härtbaren Dentalmaterial enthaltenen Gesamtmenge an

(a) nicht-aggregierten und nicht-agglomerierten Füllstoffpartikeln mit einer Partikelgröße im Bereich von 25 bis 120 nm aus Ytterbiumfluorid und

(b) nicht-aggregierten und nicht-agglomerierten Füllstoffpartikeln mit einer Partikelgröße im Bereich von 25 bis 120 nm aus Bariumsulfat.

Besonders bevorzugt bilden 95 Gew.-% oder mehr, vorzugsweise 98 Gew.-% oder mehr, besonders bevorzugt 99 Gew.-% oder mehr, insbesondere bevorzugt 100 Gew.-% der Gesamtmenge der Füllstoffpartikel aus Ytterbiumfluorid und der Füllstoffpartikel aus Bariumsulfat die Gesamtmenge der im härtbaren Dentalmaterial enthaltenen Füllstoffpartikel der Teilmenge (B1). Es gilt vorzugsweise, dass der Anteil der Teilmenge (B1) 95 Gew.-% oder mehr, vorzugsweise 98 Gew.-% oder mehr, besonders bevorzugt 99 Gew.-% oder mehr, insbesondere bevorzugt 100 Gew.-% ist, bezogen auf die Gesamtmasse an Füllstoffpartikeln aus Ytterbiumfluorid und Bariumsulfat im härtbaren Dentalmaterial.

Sämtliche Füllstoffpartikel, die nicht der Teilmenge (B1) zuzuordnen sind, werden einer weiteren Teilmenge (B2) zugeordnet, beispielsweise Füllstoffpartikel, die nicht aus Ytterbiumfluorid oder Bariumsulfat bestehen oder eine andere Partikelgröße besitzen.

Sofern das härtbare Dentalmaterial beispielsweise lediglich Füllstoffpartikel aus Ytterbiumfluorid und/oder Bariumsulfat umfasst, eine Fraktion dieser Füllstoffpartikel aus Ytterbiumfluorid und/oder Bariumsulfat aber beispielsweise aggregiert und/oder agglomeriert ist, wird diese Fraktion nicht der Teilmenge (B1), sondern der Teilmenge (B2) zugeordnet, wobei gilt: Die Teilmenge (B2) (Differenz) ist gleich der Gesamtmenge (B) (Minuend) minus Teilmenge (B1) (Subtrahend). Demzufolge gilt: Die Gesamtmenge (B) ist die Summe aus Teilmenge (B1) (Summand) und Teilmenge (B2) (Summand).

Vorzugsweise sind 95 Gew.-% oder mehr, besonders bevorzugt 98 Gew.-% oder mehr, insbesondere bevorzugt 99 Gew.-% oder mehr und am bevorzugtesten 100 Gew.-% der Füllstoffpartikel aus (i) Ytterbiumfluorid mit einer Partikelgröße im Bereich von 25 bis 120 nm und (ii) aus Bariumsulfat mit einer Partikelgröße im Bereich von 25 bis 120 nm nicht-aggregiert und nicht-agglomeriert (d.h. Primärpartikel), bezogen auf die Gesamtmasse aus Füllstoffpartikeln aus Bariumsulfat und aus Ytterbiumfluorid im erfindungsgemäßen härtbaren Dentalmaterial, die jeweils alle eine Partikelgröße im Bereich von 25 bis 120 nm besitzen.

Sofern die Startmaterialien beispielsweise Füllstoffpartikel aus Ytterbiumfluorid und/oder Bariumsulfat umfassen, welche beispielsweise eine Partikelgröße kleiner 25 nm oder größer 120 nm besitzen, werden diese ebenfalls nicht der Teilmenge (B1), sondern der Teilmenge (B2) zugeordnet.

Vorzugsweise besitzen 95 Gew.-% oder mehr, besonders bevorzugt 98 Gew.-% oder mehr, insbesondere bevorzugt 99 Gew.-% oder mehr und am bevorzugtesten 100 Gew.-% der nicht-aggregierten und nicht-agglomerierten Füllstoffpartikel aus (i) Ytterbiumfluorid und (ii) Bariumsulfat eine Partikelgröße im Bereich von 25 bis 120 nm, bezogen auf die Gesamtmasse der Füllstoffpartikel aus Bariumsulfat und aus Ytterbiumfluorid im erfindungsgemäßen härtbaren Dentalmaterial, die jeweils alle nicht-aggregiert und nicht-agglomeriert sind.

Der Anteil der Teilmenge (B1) beträgt 2,5 Gew.-% oder ist größer als 2,5 Gew.-%, bezogen auf die Gesamtmasse des härtbaren Dentalmaterials. In eigenen Untersuchungen hat sich gezeigt, dass ein erfindungsgemäßes härtbares Dentalmaterial (wie vorstehend beschrieben, vorzugsweise wie vorstehend als bevorzugt definiert) eine gute Transluzenz, eine gute Röntgenopazität und gute physikalische Eigenschaften besitzt, wobei insbesondere in DVT-Bildern vergleichsweise wenig Artefakte (bzw. weniger ausgeprägte Artefakte) auftreten. Härtbare Dentalmaterialien, herstellbar durch Mischen der oben genannten Startmaterialien, wobei der Anteil der Teilmenge (B1) (deutlich) kleiner 2,5 Gew.-% ist, bezogen auf die Gesamtmasse des härtbaren Dentalmaterials, zeigen gemäß eigenen Untersuchungen in vielen Fällen keine signifikante Reduzierung von Artefakten in DVT-Bildern. Weiterhin wurde in einer Vielzahl von Fällen beobachtet, dass ein härtbares Dentalmaterial mit einem Anteil kleiner 2,5 Gew.-% keine zufriedenstellende Röntgenopazität besitzt, insbesondere bei Abwesenheit ausreichender Mengen sonstiger röntgenopaker partikulärer Füllstoffe.

Insbesondere die Füllstoffpartikel der Teilmenge (B1) tragen zu der vergleichsweise geringen Anzahl an Artefakten in DVT-Bildern bei, die von einem gehärteten Dentalmaterial auf Basis des erfindungsgemäßen härtbaren Dentalmaterials gemacht wurden.

Füllstoffpartikel der Teilmenge (B1) sind vorzugsweise organisch oberflächenmodifiziert. Für eine organische Oberflächenmodifizierung sind vorzugsweise Verbindungen des allgemeinen Typs X-Sp-Y geeignet, wobei "X" und "Y" funktionelle Gruppen bedeuten, die durch einen Linker (Spacer, "Sp") miteinander verbunden sind.

Die funktionelle Gruppe "X" ist vorzugsweise so ausgewählt, dass sie unter Komplexbildung eine entsprechende Bindung mit der Oberfläche des Füllstoffpartikels eingehen kann. Geeignet sind beispielsweise Gruppen des Typs Phosphate, Phosphonate, Carboxylate, Dithiophosphate, Dithiophosphonate, Amine und Amide. Die Oberflächenanbindung der Verbindung für eine organische Oberflächenmodifizierung an die Füllstoffpartikel kann durch mehrfache Ausbildung einer funktionellen Gruppe (Polyphosphate, Polycarboxylate) verbessert werden.

Als Linker (Spacer, "Sp") eignen sich (und sind somit bevorzugt) lineare oder verzweigte Alkylketten, Aromaten oder Kombinationen dieser Gruppen, die jeweils durch Heteroatome wie O, N, S oder P unterbrochen sein können.

Die funktionelle Gruppe "Y" vermittelt die Kompatibilität der Füllstoffpartikel mit der Gesamtmenge der polymerisierbaren Monomere (A), beispielsweise durch Hydrophobierung. Bevorzugt sind lineare oder verzweigte Alkyl-, Arenyl- oder Alkenylgruppen, wobei letztere den Vorteil bieten, in die Polymerisation der polymerisierbaren Monomere miteinbezogen zu werden, was zu einer besonders guten Einbindung der Partikel in das gehärtete Dentalmaterial führt. Besonders bevorzugt sind in diesem Zusammenhang Methacrylgruppen.

Bevorzugt ist in einigen Fällen ein erfindungsgemäßes härtbares Dentalmaterial (wie vorstehend beschrieben, vorzugsweise wie vorstehend als bevorzugt definiert), wobei 70 Gew.-% oder mehr, vorzugsweise 80 Gew.-% oder mehr, der partikulären Füllstoffe, welche die Gesamtmenge (B) der partikulären Füllstoffe im härtbaren Dentalmaterial bilden, organisch oberflächenmodifiziert sind, vorzugsweise umfasst das härtbare Dentalmaterial ausschließlich Füllstoffpartikel, die organisch oberflächenmodifiziert sind. Vorzugsweise handelt es sich dabei um organische Oberflächenmodifizierungen wie vorstehend bzw. nachfolgend beschrieben.

In anderen Fällen ist ein erfindungsgemäßes härtbares Dentalmaterial bevorzugt, wobei die Teilmenge (B1) ausschließlich organisch oberflächenmodifizierte Füllstoffpartikel umfasst.

Besonders bevorzugt ist ein erfindungsgemäßes härtbares Dentalmaterial (wie vorstehend beschrieben, vorzugsweise wie vorstehend als bevorzugt definiert), wobei die Gesamtmenge (B) der partikulären Füllstoffe im härtbaren Dentalmaterial ausschließlich organisch oberflächenmodifizierte partikuläre Füllstoffe umfasst. Das härtbare Dentalmaterial umfasst in diesem Fall keine partikulären Füllstoffe ohne organische Oberflächenmodifikation. Das nachfolgend und vorstehend zu erfindungsgemäßen härtbaren Dentalmaterialien Gesagte gilt insbesondere für erfindungsgemäße härtbare Dentalmaterialien, wobei die Teilmenge (B1) ausschließlich organisch oberflächenmodifizierte Füllstoffpartikel umfasst (vorzugsweise wie vorstehend beschrieben), vorzugsweise das erfindungsgemäße härtbare Dentalmaterial ausschließlich organisch oberflächenmodifizierte Füllstoffpartikel umfasst (vorzugsweise organische Oberflächenmodifizierungen wie vorstehend bzw. nachfolgend im Text beschrieben).

Besonders bevorzugt ist ein erfindungsgemäßes härtbares Dentalmaterial (wie vorstehend beschrieben, vorzugsweise wie vorstehend als bevorzugt definiert), wobei die Gesamtmenge (A) aus dem polymerisierbaren Monomer bzw. aus den polymerisierbaren Monomeren einen Brechungsindex $n_A$ im Bereich von 1,45 bis 1,55 besitzt, vorzugsweise einen Brechungsindex $n_A$ im Bereich von 1,48 bis 1,55. Der Brechungsindex $n_A$ wird vorzugsweise am entsprechenden Startmaterial bestimmt.

Eines, mehrere oder sämtliche der ein, zwei, drei oder mehr als drei polymerisierbaren Monomere, welche die Gesamtmenge (A) der polymerisierbaren Monomere im härtbaren Dentalmaterial bilden, sind vorzugsweise lichthärtbare (d.h. photopolymerisierbare) Monomere. Diese lichthärtbaren (d.h. photopolymerisierbaren) Monomere sind vorzugsweise ausgewählt aus der Gruppe bestehend aus 2-Hydroxyethylmethacrylat (HEMA), 2-Hydroxypropylmethacrylat und 3-Hydroxypropylmethacrylat (HPMA), 1,2-Dihydroxypropylmethacrylat, 1,3-Dihydroxypropylmethacrylat, 2,3-Dihydroxypropylmethacrylat, Ethylenglykoldimethacrylat (EGDMA), Diethylenglykoldimethacrylat, Triethylenglykoldimethacrylat (TEDMA), Tetraethylenglykoldimethacrylat (TEGDMA), Polyethylenglykoldimethacrylat (PEGDMA), Propylenglykoldimethacrylat (PGDMA), Dipropylenglykoldimethacrylat, Tripropylenglykoldimethacrylat (TPGDMA), Tetrapropylenglykoldimethacrylat, Polypropylenglykoldimethacrylat (PEGDMA), 1,4-Butandioldimethacrylat, 1,3-Butandioldimethacrylat, 1,6-Hexandioldimethacrylat (HEDMA), 1,12-Dodecandioldimethacrylat (DODMA), 2-Hydroxypropyl-1,3-dimethacrylat und 3-Hydroxypropyl-1,2-dimethacrylat (GDMA), Bisphenol-A-glycerolat-dimethacrylat (Bis-GMA), ethoxyliertes Bisphenol-A-dimethacrylat (Bis-EMA), 7,7,9-Trimethyl-4,13-dioxo-5,12-diazahexadecan-1,16-dioxydimethacrylat (UDMA), Neopentylglykoldimethacrylat, Trimethylolpropantrimethacrylat (TMPTMA), Pentaerythritoldimethacrylat und Dimethacrylate des Dihydro-xymethyltricyclo[5.2.1.0$^{2,6}$]decans wie Tricyclodecandimethanoldimethacrylat (TCD-DMA).

In einigen Fällen besonders bevorzugt sind die Dimethacrylate bzw. Diacrylate des Dihydroxymethyltricyclo[5.2.1.0$^{2,6}$]decans, wie in den Druckschriften DE 2419887 A1, DE 2406557 A1, DE 2931926 A1, DE 3522005 A1, DE 3522006 A1, DE 3703120 A1, DE 102005021332 A1, DE 102005053775 A1, DE 102006060983 A1, DE 69935794 T2 und DE 102007034457 A1 beschrieben.

Zudem können auch lichthärtbare Monomere mit ethylenischen Doppelbindungen auf Basis von Polysiloxanen verwendet werden, wie sie beispielsweise in der DE 19903177 A1 oder in der DE 4416857 C1 beschrieben sind.

Weitere bevorzugte polymerisierbare Monomere besitzen eine oder mehrere Säurefunktionen.

Geeignete, eine Phosphorsäuregruppe enthaltende Monomere sind beispielsweise 2-(Meth)acryloyloxyethyldihydrogenphosphat, Bis[2-(meth)acryloyloxyethyl]hydrogen-phosphat, 2-(Meth)acryloyloxyethylphenylhydrogenphosphat, 6-(Meth)acryloyloxyhexyldihydrogenphosphat, 10-(Meth)acryloyloxydecyldihydrogenphosphat (MDP), 6-(Meth)-acryloyloxyhexylphenylhydrogenphosphat, 10-(Meth)acryloyloxydecyldihydrogenphosphat, 1,3-Di(meth)acryloyloxypropan-2-dihydrogenphosphat, 1,3-Di(meth)-acryloyloxypropan-2-phenylhydrogenphosphat und Bis[5-(2-(meth)acryloyloxyethoxycarbonyl)heptyl]hydrogenphosphat.

Geeignete, eine Carbonsäuregruppe enthaltende Monomere sind beispielsweise 4-(Meth)acryloxyethyltrimellithsäure (4-MET), 4-(Meth)acryloxyethyltrimellithsäureanhydrid (4-META), 4-(Meth)acryloxydecyltrimellithsäure, 4-(Meth)acryloxydecyltrimellithsäureanhydrid, 11-(Meth)acryloyloxy-1,1-undecandicarbonsäure, 1,4-Di(meth)-acryloyloxypyromellithsäure, 2-(Meth)acryloyloxyethylmaleinsäure, 2-(Meth)acryloyloxyethylphthalsäure und 2-(Meth)acryloyloxyethylhexahydrophthalsäure.

Weitere geeignete, säuregruppentragende Monomere sind beispielsweise in EP 0980682 B1 und EP 0948955 A1 genannt.

In ausgewählten Fällen ist ein erfindungsgemäßes härtbares Dentalmaterial (wie vorstehend beschrieben, vorzugsweise wie vorstehend als bevorzugt definiert) bevorzugt, wobei die Gesamtmenge (A) der polymerisierbaren Monomere im härtbaren Dentalmaterial das polymerisierbare Monomer TCD-DMA umfasst oder daraus besteht, vorzugsweise umfasst.

Die in Verbindung mit dem erfindungsgemäßen härtbaren Dentalmaterial (wie vorstehend und nachfolgend beschrieben, insbesondere nachfolgend unter der Überschrift "Beispiele") und dem erfindungsgemäßen gehärteten Dentalmaterial (wie nachfolgend beschrieben und insbesondere wie nachfolgend unter der Überschrift "Beispiele") angegebenen Brechungsindices gelten für eine Temperatur von 20°C und für Licht einer Wellenlänge von 589 nm (Natrium-D-Linie) ($n_D^{20}$).

Es hat sich in eigenen Untersuchungen gezeigt, dass ein Brechungsindex $n_A$ der Gesamtmenge (A) aus dem polymerisierbaren Monomer bzw. aus den polymerisierbaren Monomeren im Bereich von 1,45 bis 1,55 sehr häufig zu sehr guten Transluzenzwerten führt, da die Brechungsindices der ein, zwei, drei oder mehr als drei Füllstoffe, welche die Gesamtmenge (B) der Füllstoffe im härtbaren Dentalmaterial bilden, vergleichsweise einfach auf einen solchen Brechungsindex $n_A$ abgestimmt werden können (in einer entsprechenden Mischung aus polymerisierbaren Monomeren und Füllstoffen; weitere Details weiter unten im Text). Diese gute Abstimmung hat zur Folge, dass bei lichtinduzierter Polymerisation von polymerisierbaren Monomeren im härtbaren Dentalmaterial das eingesetzte Licht eine hohe Durchdringungstiefe erreicht und somit eine gleichmäßige Polymerisation bewirkt. Dies führt zu einem qualitativ hochwertigen, gehärteten Dentalmaterial (wie unten im Text beschrieben).

Sofern der Brechungsindex $n_A$ kleiner 1,45 oder größer 1,55 ist, wird die vorstehend genannte Abstimmung in vielen Fällen komplexer (insbesondere bei einem Brechungsindex kleiner 1,45) und die Durchdringungstiefe des Lichtes ist nur noch in wenigen Fällen akzeptabel bzw. kaum noch akzeptabel (da in den meisten Fällen eine besonders intensive Lichtstreuung zu erwarten ist). Dies erhöht das Risiko, dass aus einem (nur teil-)gehärteten Dentalmaterial nicht-polymerisierte polymerisierbare Monomere austreten und in den Mundraum wandern. Zusätzlich besitzen derartige (teil-)gehärtete Dentalmaterialien in vielen Fällen eine reduzierte Festigkeit. Solche härtbaren Dentalmaterialien müssen dann aufwendig in sehr dünnen Schichten aufgetragen und jede Schicht einzeln gehärtet werden, um die vorstehend genannten Nachteile zu minimieren. Sofern der Brechungsindex größer 1,45 aber kleiner 1,48 ist, wird in den meisten Fällen eine ausreichend gute Abstimmung erreicht, so dass eine ausreichend gute Durchdringungstiefe des Lichtes resultiert. Eine Applikation eines solchen härtbaren Dentalmaterials (und die anschließende Härtung) kann dann entsprechend ohne großen Aufwand erfolgen. Liegt der Brechungsindex in einem Bereich von 1,48 bis 1,55 werden regelmäßig sehr gute Ergebnisse erreicht.

Besonders bevorzugt ist ein erfindungsgemäßes härtbares Dentalmaterial (wie vorstehend beschrieben, vorzugsweise wie vorstehend als bevorzugt definiert), wobei die Gesamtmenge der partikulären Füllstoffe in der Teilmenge (B1) einen Brechungsindex $n_{B1}$ im Bereich von 1,55 bis 1,64 besitzt.

Eigene Untersuchungen haben gezeigt, dass ein erfindungsgemäßes härtbares Dentalmaterial (wie vorstehend beschrieben, vorzugsweise wie vorstehend als bevorzugt definiert) zu sehr guten Ergebnissen in DVT-Untersuchungen führt, die mit einem gehärteten Dentalmaterial auf Basis des erfindungsgemäßen härtbaren Dentalmaterials durchgeführt wurden. Dies ist - wie der Vergleich mit nicht-erfindungsgemäßen härtbaren Dentalmaterialien in eigenen Untersuchungen gezeigt hat - insbesondere auf die Anwesenheit der oben beschriebenen partikulären Füllstoffpartikel aus Ytterbiumfluorid und/oder Bariumsulfat zurückzuführen.

Bariumsulfat besitzt einen Brechungsindex von 1,64, Ytterbiumfluorid von 1,55. Somit besitzt die oben beschriebene Teilmenge (B1) einen Brechungsindex $n_{B1}$ im Bereich von 1,55 bis 1,64. Sofern die Teilmenge (B1) aus Bariumsulfat besteht, besitzt die Teilmenge (B1) einen Brechungsindex $n_{B1}$ von 1,64 (der Brechungsindex der Teilmenge (B1) entspricht in diesem Fall dem Brechungsindex von Bariumsulfat). Sofern die Teilmenge (B1) aus Ytterbiumfluorid besteht, besitzt die Teilmenge (B1) einen Brechungsindex $n_{B1}$ von 1,55 (der Brechungsindex der Teilmenge (B1) entspricht in diesem Fall dem Brechungsindex von Ytterbiumfluorid) (zur Bestimmung des Brechungsindexes siehe unten im Text unter "Beispiele", Punkt I.a).

In Abhängigkeit von der gewünschten Verwendung ist in manchen Fällen ein erfindungsgemäßes härtbares Dentalmaterial bevorzugt (wie vorstehend beschrieben, vorzugsweise wie vorstehend als bevorzugt definiert), in dem eine Mischung aus Bariumsulfat und Ytterbiumfluorid eingesetzt wird, wobei der Brechungsindex dieser Mischung vorzugsweise im Bereich von 1,55 bis 1,64 liegt, bevorzugt im Bereich von 1,55 bis 1,60 liegt. Der Brechungsindex solch einer Mischung kann, wie unten unter "Beispiele", Punkt I.a beschrieben, bestimmt werden.

Der Brechungsindex der Teilmenge (B1) ist von Bedeutung, da dieser Brechungsindex Einfluss auf den Brechungsindex des härtbaren Dentalmaterials hat (siehe hierzu weiter unten im Text). Um eine hohe Transluzenz im härtbaren Dentalmaterial zu erreichen (und damit einhergehend eine gute Durchdringungstiefe des Lichtes zur Polymerisation polymerisierbarer Monomere im härtbaren Dentalmaterial) ist die genaue Bestimmung bzw. die genaue Kenntnis der einzelnen Brechungsindices wichtig.

Besonders bevorzugt ist ein erfindungsgemäßes härtbares Dentalmaterial (wie vorstehend beschrieben, vorzugsweise wie vorstehend als bevorzugt definiert), wobei der Anteil der Teilmenge (B1) in einem Bereich von 2,5 Gew.-% bis 20 Gew.-% liegt, vorzugsweise in einem Bereich von 5 Gew.-% bis 18 Gew.-%, weiter bevorzugt in einem Bereich von 10,1 Gew.-% bis 18 Gew.-%, besonders bevorzugt in einem Bereich von 10,1 Gew.-% bis 16 Gew.-%, bezogen auf die Gesamtmasse des härtbaren Dentalmaterials.

Es hat sich in eigenen Untersuchungen gezeigt, dass röntgenopake Füllstoffpartikel, wie vorstehend für die Teilmenge (B1) definiert, in einem erfindungsgemäßen härtbaren Dentalmaterial (wie vorstehend beschrieben, vorzugsweise wie vorstehend als bevorzugt definiert) zu guten Ergebnissen in DVT-Bildern führen. Insbesondere wurde eine reduzierte Anzahl an Artefakten in entsprechenden DVT-Bildern beobachtet, im Vergleich mit DVT-Bildern, die bei ansonsten identischen Aufnahmebedingungen Prüfkörper zeigen, deren Zusammensetzung nicht mit der Zusammensetzung erfindungsgemäßer härtbarer Dentalmaterialien übereinstimmt, insbesondere solche Prüfkörper, die keine Füllstoffpartikel umfassen, wie sie oben im Text für die Teilmenge (B1) definiert sind.

Als typische Artefakte in DVT-Bildern gelten (eine genaue Erläuterung der Artefakte kann dem folgenden Artikel entnommen werden: R. Schulze et al., "Artefacts in CBCT: a review", Dentomaxillofacial Radiology, 40, 265-273 (2011)):

- Aufhärtungsartefakte,

- Auslöschungsartefakte,

- Ringartefakte,

- Bewegungsartefakte,

- Alias-Artefakte,

- Partielle Volumeneffekte/"Exponential Edge Gradient" Effekte,

- Generelle Artefakte wie

  - Streuung,

  - Rauschen,

  - lokale Tomographie

Sofern der Anteil der Teilmenge (B1) in einem Bereich von 2,5 Gew.-% bis 20 Gew.-% liegt, bezogen auf die Gesamtmasse des härtbaren Dentalmaterials, werden regelmäßig vergleichsweise gute Ergebnisse erlangt, d.h. die Anzahl der Artefakte und die Störung durch Artefakte in DVT-Bildern ist regelmäßig reduziert. Liegt der Anteil der Teilmenge (B1) in einem Bereich von 5 Gew.-% bis 18 Gew.-% werden regelmäßig besonders gute Ergebnisse erzielt, d.h. eine besonders gute Reduzierung von Artefakten. Liegt der Anteil der Teilmenge (B1) in einem Bereich von 10,1 Gew.-% bis 18 Gew.-%, bezogen auf die Gesamtmasse des härtbaren Dentalmaterials, werden regelmäßig sehr gute Ergebnisse erzielt, d.h. eine regelmäßige, sehr gute Reduzierung von Artefakten. In manchen Fällen ist es bevorzugt, dass der Anteil der Teilmenge (B1) in einem Bereich von 10,1 Gew.-% bis 16 Gew.-% liegt, bezogen auf die Gesamtmasse des härtbaren Dentalmaterials. Auch in diesen Fällen werden regelmäßig sehr gute Ergebnisse erzielt, d.h. eine sehr gute Reduzierung von Artefakten.

Unterschreitet der Anteil der Teilmenge (B1) einen Wert von 2,5 Gew.-%, wird regelmäßig (i) keine gute/zufriedenstellende Reduzierung von Artefakten und (ii) keine zufriedenstellende Röntgenopazität erreicht (insbesondere bei Abwesenheit ausreichender Mengen sonstiger röntgenopaker partikulärer Füllstoffe). Diese Effekte verstärken sich, je weiter der Anteil der Teilmenge (B1) unter dem Wert von 2,5 Gew.-% liegt.

Überschreitet der Anteil der Teilmenge (B1) einen Wert von 20 Gew.-%, wird regelmäßig eine nicht gut zu verarbeitende Konsistenz des erfindungsgemäßen härtbaren Dentalmaterials beobachtet sowie eine Beeinträchtigung der Eigenschaften Festigkeit, Härte und Abrasionsbeständigkeit eines draus hergestellten erfindungsgemäßen gehärteten Dentalmaterials.

Besonders bevorzugt ist ein erfindungsgemäßes härtbares Dentalmaterial (wie vorstehend beschrieben, vorzugsweise wie vorstehend als bevorzugt definiert), wobei die Gesamtmenge (B) der partikulären Füllstoffe (wie vorstehend definiert) aus der Teilmenge (B1) und einer Teilmenge (B2) (wie vorstehend definiert) besteht, wobei die Gesamtmenge der partikulären Füllstoffe in der Teilmenge (B2) vorzugsweise einen Brechungsindex $n_{B2}$ im Bereich von 1,49 bis 1,62, besonders bevorzugt im Bereich von 1,50 bis 1,56 besitzt.

Der Teilmenge (B2) werden - wie ausgeführt - sämtliche Füllstoffpartikel zugeordnet, die nicht der Teilmenge (B1) zuzuordnen sind. Bevorzugte Füllstoffe der Teilmenge (B2) sind ein, zwei oder mehr als zwei Füllstoffe ausgewählt aus der Gruppe bestehend aus anorganischen Füllstoffen und organischen Füllstoffen, wobei jeweils gilt: Füllstoffe der Teilmenge (B2) sind keine Füllstoffe der Teilmenge (B1) bzw. umfassen keine Füllstoffe der Teilmenge (B1).

Bevorzugte anorganische Füllstoffe zur Verwendung als Bestandteil der Teilmenge (B2) sind ausgewählt aus der Gruppe bestehend aus amorphen Materialien auf Basis von $SiO_2$, $ZrO_2$ und/oder $TiO_2$, Mischoxide, pyrogene Kieselsäure, Fällungskieselsäure, Bariumsilikatgläser, Bariumfluorsilikatgläser, Strontiumsilikatgläser, Strontiumfluorsilikatgläser und sonstige Dentalgläser, Quarzglas, Li/Al-Silikatgläser, Calciumsilikate, Natriumaluminiumsilikate, Fluoraluminiumsilikatgläser, Feldspat, Oxide von Aluminium oder Silicium, Zeolithe, Apatit, Zirkonsilikate und schwerlösliche Metallsalze wie Calciumfluorid. Besonders bevorzugte anorganische Füllstoffpartikel zur Verwendung als Bestandteil der Teilmenge (B2) sind dentale Gläser z.B. der Firma Schott, deren Brechungsindices im Bereich von 1,49 bis 1,62, besonders bevorzugt im Bereich von 1,50 bis 1,56 liegen. Ganz besonders bevorzugte dentale Gläser der Firma Schott tragen die Bezeichnung GM27884 und G018-186.

Bevorzugt ist der Einsatz oberflächenmodifizierter anorganischer Füllstoffpartikel (anorganische Füllstoffpartikel wie vorstehend beschrieben, vorzugsweise wie vorstehend als bevorzugt definiert). Bevorzugte oberflächenmodifizierte anorganische Füllstoffpartikel werden durch Oberflächenmodifizierung mit einem Silan erhalten, vorzugsweise durch Oberflächenmodifizierung mit gamma-Methacryloxypropyltrimethoxysilan.

In einigen Fällen können z.B. verstärkend wirkende Füllstoffmaterialien, wie Glasfasern, Polyamid- oder Kohlenstofffasern eingesetzt werden. Ein erfindungsgemäßes härtbares Dentalmaterial kann auch feinteilige Splitter oder Perlpolymerisate enthalten, wobei die Perlpolymerisate Homo- oder Copolymere organischer härtbarer Monomere sein können.

Bevorzugte organische Füllstoffe zur Verwendung als Bestandteil der Teilmenge (B2) umfassen oder bestehen aus beispielsweise ein oder mehr Verbindungen ausgewählt aus der Gruppe bestehend aus Polyvinylacetat und Copolymere des Polyvinylacetats mit einer oder mehreren polymerisierbaren Verbindungen, Polystyrol, Polyethylen, Polypropylen, Wachse wie Polyethylenwachs, Polybutylen, Polybutadien, Copolymere des Butadiens und des Styrols, Polyacrylnitril, Harze wie Kolophoniumharz oder Kohlenwasserstoffharze, Poly(meth)acrylatester, d.h. Umsetzungsprodukte von Poly(meth)acrylsäure mit linearen oder verzweigten aliphatischen, aromatischen oder cycloaliphatischen Alkoholen wie Methanol, Ethanol, Propanol, Isopropanol, den isomeren Butanolen und höheren Homologen der genannten Alkohole mit bis zu 22 Kohlenstoffatomen, Cyclohexanol, Benzylalkohol und dergleichen, Polydialkylmaleinate wie Dibutylmaleinat und deren Copolymere und Silylgruppen-haltige Polymere wie Polyvinylsilane oder Copolymere von Vinylsilan mit einem oder mehreren der genannten Monomeren.

Besonders bevorzugt ist ein erfindungsgemäßes härtbares Dentalmaterial (wie vorstehend beschrieben, vorzugsweise wie vorstehend als bevorzugt definiert), wobei der Anteil der Gesamtmenge (A) im Bereich von 7,95 bis 27,95 Gew.-%, vorzugsweise im Bereich von 9 bis 24,9 Gew.-% liegt, bezogen auf die Gesamtmasse der zu mischenden Startmaterialien.

Liegt der Anteil der Gesamtmenge (A) im Bereich von 7,95 bis 27,95 Gew.-%, bezogen auf die Gesamtmasse der zu mischenden Startmaterialien, besitzt das erfindungsgemäße härtbare Dentalmaterial (wie vorstehend beschrieben, vorzugsweise wie vorstehend als bevorzugt definiert) regelmäßig eine gute Verarbeitbarkeit bei der Herstellung des erfindungsgemäßen gehärteten Dentalmaterials (wie vorstehend beschrieben, vorzugsweise wie vorstehend als bevorzugt definiert). Ein daraus hergestelltes gehärtetes Dentalmaterial besitzt außerdem regelmäßig gute mechanische Eigenschaften, wie beispielsweise sehr gute Festigkeiten, Härten und Abrasionsbeständigkeiten. Wird der Wert von 7,95 Gew.-% (deutlich) unterschritten, gelangt man nicht zu einem pastösen Dentalmaterial, das angenehm bzw. leicht verarbeitet werden kann. In manchen Fällen wurde sogar beobachtet, dass ein solches Dentalmaterial gar nicht verarbeitet werden konnte.

Ist der Anteil der Gesamtmenge (A) (deutlich) größer 27,95 Gew.-% führt dies in vielen Fällen zu nicht ausreichend festen, harten und abrasionsbeständigen gehärteten Dentalmaterialien.

Das erfindungsgemäße härtbare Dentalmaterial ist vorzugsweise ein hochgefülltes Dentalmaterial. Besonders bevorzugt ist ein erfindungsgemäßes härtbares Dentalmaterial (wie vorstehend beschrieben, vorzugsweise wie vorstehend als bevorzugt definiert), wobei der Anteil der Gesamtmenge (B) im Bereich von 72 bis 92 Gew-% liegt, vorzugsweise im Bereich von 75 bis 89,5 Gew.-% liegt, bezogen auf die Gesamtmasse der zu mischenden Startmaterialien.

Liegt der Anteil der Gesamtmenge (B) im Bereich von 72 bis 92 Gew-%, bezogen auf die Gesamtmasse der zu mischenden Startmaterialien, besitzt das durch Polymerisation polymerisierbarer Monomere im härtbaren Dentalmaterial resultierende erfindungsgemäße gehärtete Dentalmaterial in den meisten Fällen eine sehr gute Abrasionsbeständigkeit, Festigkeit und Härte. Eine besonders gute Abrasionsbeständigkeit, Festigkeit und Härte ergibt sich regelmäßig immer dann, wenn der Anteil der Gesamtmenge (B) 75 Gew.-% oder größer ist, bezogen auf die Gesamtmasse der zu mischenden Startmaterialien.

Ist der Anteil der Gesamtmenge (B) (deutlich) kleiner als 72 Gew.-%, wurde in einzelnen Fällen eine mangelhafte Abrasionsbeständigkeit beobachtet.

Überschreitet der Anteil der Gesamtmenge (B) (deutlich) die 92 Gew.-%, sinkt entsprechend der Anteil polymerisierbarer Monomere im erfindungsgemäßen härtbaren Dentalmaterial und ist regelmäßig so gering, dass man nicht zu einem pastösen Dentalmaterial gelangt, das angenehm bzw. leicht verarbeitet werden kann. In einigen Fällen wurde sogar beobachtet, dass ein solches Dentalmaterial gar nicht verarbeitet werden konnte.

Besonders bevorzugt ist ein erfindungsgemäßes härtbares Dentalmaterial (wie vorstehend beschrieben, vorzugsweise wie vorstehend als bevorzugt definiert), wobei der Anteil der Gesamtmenge (C) im Bereich von 0,05 bis 2 Gew.-% liegt, vorzugsweise im Bereich von 0,1 bis 1,5 Gew.-% liegt, bezogen auf die Gesamtmasse der zu mischenden Startmaterialien.

Die Gesamtmenge (C) besteht vorzugsweise aus einer, mehreren oder sämtlichen Verbindungen ausgewählt aus der Gruppe bestehend aus

- rheologische Hilfsmittel,

- Polymerisationsinitiatoren, vorzugsweise Photoinitiatoren,

- chemische Verbindungen als Katalysatoren bzw. Bestandteile von Katalysatorsystemen,

- Farbmittel, - Stabilisatoren, insbesondere UV- und Tageslichtstabilisatoren,

- Inhibitoren,

- Aktivatoren,

- Molekulargewichtsregler,

- Konservierungsmittel,

- grenzflächenaktive Substanzen,

- Biozide, vorzugsweise Bakterizide,

- organische Polymere und Oligomere und Verbindungen mit hohen Molekulargewichten, vorzugsweise Weichmacher,

- Verdickungsmittel und

- dentale Arzneimittel,

Besonders bevorzugt als Hilfsstoffe sind Polymerisationsinitiatoren, vorzugsweise Photoinitiatoren, Farbmittel und Inhibitoren. Es gilt hierbei: Stoffe sind dann als Bestandteil der Gesamtmenge (C) aufzufassen, wenn sie nicht den Definitionen zu (A) und (B) entsprechen, es sich also weder um ein polymerisierbares Monomer, noch um einen partikulären Füllstoff handelt. Farbpigmente beispielsweise sind zwar selbstverständlich Farbmittel, werden jedoch der Komponente (B) zugeordnet, weil sie auch als partikulärer Füllstoff aufgefasst werden können.

Bevorzugte erfindungsgemäße härtbare Dentalmaterialien (wie vorstehend definiert, vorzugsweise wie vorstehend als bevorzugt definiert) sind lichthärtbar (photohärtbar). Die Polymerisation polymerisierbarer Monomere erfolgt dann regelmäßig durch Einwirkung von Licht bestimmter Wellenlängen und in Gegenwart von Photoinitiatoren. Beispiele für einen Photoinitiator schließen Verbindungen ein, die nur photosensibilisierend wirken, sowie Kombinationen aus Photosensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Beispiele der unterschiedlichen Klassen finden sich beispielsweise in DE 10 2006 019 092 A1 und DE 39 41 629 C2.

Beispiele für Beschleuniger, die zusammen mit Photosensibilisatoren eingesetzt werden, sind tertiäre Amine, sekundäre Amine, Barbitursäuren, Zinnverbindungen, Aldehyde und Schwefelverbindungen. Konkrete Beispiele der unterschiedlichen Klassen finden sich in DE 10 2006 019 092 A1 und DE 39 41 629 C2.

Weitere geeignete Initiatoren sowie Initiatorkombinationen sind in DE 601 16 142 T2 beschrieben.

Im Rahmen der vorliegenden Erfindung bevorzugte Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung (Polymerisation) eines erfindungsgemäßen bzw. erfindungsgemäß anzuwendenden bzw. einzusetzenden härtbaren Dentalmaterials bewirken können.

In einzelnen Fällen ist ein erfindungsgemäßes härtbares Dentalmaterialien (wie vorstehend definiert, vorzugsweise wie vorstehend als bevorzugt definiert) bevorzugt, das durch chemische Aushärtung härtbar ist. Hierzu sind dem Fachmann diverse Initiatoren für eine chemische Aushärtung bekannt. Es sei insoweit exemplarisch auf die Offenbarung in EP 1 720 506 A1 verwiesen.

In vielen Fällen ist ein erfindungsgemäßes härtbares Dentalmaterial bevorzugt, welches sowohl lichthärtbar ist als auch chemisch härtbar ist. Diese bevorzugten dualhärtenden Dentalmaterialien umfassen eine Kombination aus Photoinitiatoren und Initiatoren zur chemischen Aushärtung. Die vorstehenden Ausführungen zu bevorzugten Initiatoren gelten entsprechend.

Bevorzugte erfindungsgemäße lichthärtbare Dentalmaterialien (wie vorstehend definiert, vorzugsweise wie vorstehend als bevorzugt definiert, einschließlich der dualhärtenden erfindungsgemäßen Dentalmaterialien), enthalten vorzugsweise einen oder mehrere Inhibitoren (auch Stabilisatoren genannt). Diese werden üblicherweise zugesetzt, um eine Spontanpolymerisation zu vermeiden. Sie reagieren mit vorzeitig entstehenden Radikalen, die abgefangen werden, so dass eine vorzeitige Polymerisation verhindert wird. Das erhöht die Lagerstabilität der bevorzugten lichthärtbaren Dentalmaterialen (bzw. der dualhärtenden Dentalmaterialien). Bevorzugt einzusetzende Inhibitoren sind Phenolderivate wie Hydrochinonmonomethylether (MeHQ) oder 2,6-Di-*tert*.butyl-4-methylphenol (BHT). Weitere bevorzugt einzusetzende Inhibitoren wie *tert*.-Butylhydroxyanisol (BHA), 2,2-Diphenyl-1-picrylhydrazyl-, Galvinoxyl-, Triphenylmethyl-Radikale, 2,2,6,6,-Tetra-methylpiperidinyl-1-oxylradikale (TEMPO) sowie Derivate von TEMPO oder Phenothiazin sowie Derivate dieser Verbindung werden in EP 0 783 880 B1 beschrieben. Alternative bevorzugte Inhibitoren sind in DE 101 19 831 A1 und in EP 1 563 821 A1 angegeben.

Bevorzugte erfindungsgemäße härtbare Dentalmaterialien (wie vorstehend definiert, vorzugsweise wie vorstehend als bevorzugt definiert) besitzen charakteristische Farben, vorzugsweise eine Zahnfarbe, die von der "VITA classical A1 --D4 Farbskala" umfasst ist; solche Farben werden bezeichnet mit A1 - A4 (Rötlichbräunlich), B1 - B4 (Rötlichgelblich), C1 - C4 (Grautöne), D2 - D4 (Rötlich-grau). Bevorzugte Farben können mittels Farbmitteln, vorzugsweise Farbpigmenten, eingestellt werden.

Übliche Molekulargewichtsregler sind beispielsweise Aldehyde und Ketone, wie Formaldehyd, Acetaldehyd, Propionaldehyd, n-Butyraldehyd, Isobutyraldehyd, Methylethylketon, Aceton, Methylisobutylketon, Ameisensäure, Ammoniumformiat, Hydroxylammoniumsulfat und Hydroxylammoniumphosphat, Verbindungen, die Schwefel in organisch gebundener Form enthalten, wie Di-*n*-butylsulfid, Di-*n*-octylsulfid, Diphenylsulfid, Diisopropyldisulfid, Di-*n*-butyldisulfid, Di-*n*-hexyldisulfid, Diacetyldisulfid und Di-*tert*.-butyl-trisulfid, Verbindungen, die Schwefel in Form von SH-Gruppen aufweisen, wie *n*-Butylmercaptan, *n*-Hexylmercaptan und *n*-Dodecylmercaptan, Octadecylmercaptan, weitere Schwefelverbindungen wie Hydrogensulfite, Disulfite, Verbindungen wie Mercaptoethanol, Mercaptobutanol, Mercaptoessigsäure, 3-Mercaptopropionsäure, Mercaptobernsteinsäure, Thioglycerin, Thioglykolsäure, Diethanolsulfid, Thiodiglykol, Ethylthioethanol, 2,2,4,6,6-Pentamethylheptan-4-thiol, 2,2,4,6,6,8,8-Heptamethylnonan-4-thiol, Thioharnstoff, Dimethylsulfoxid, Ethylhexylthioglycolat, Pentaerythrittetrathioglycolat, Mercaptopropyltrimethoxysilan, dann Allylverbindungen wie Allylalkohol, Allylbromid, oder Benzylverbindungen wie Benzylchlorid oder Alkylhalogenide wie Chloroform, Bromtrichlormethan oder Tetrachlormethan, Tetrabrommethan, Methylenchlorid, weiterhin niedere und höhermolekulare einwertige oder mehrwertige Alkohole wie Methanol, Ethanol, *n*-Propanol, Isopropanol, *tert.*-Butanol, *sec*-Butanol, *n*-Butanol, Amylalkohol, Cyclohexanol, Octanol, Dodecanol, 1-Ethylhexanol, Glycerin, Stearylalkohol, Oleylalkohol, Hydroxyethylmethacrylat oder Amine wie Triethylamin sowie Toluol oder Ethylbenzol.

Weitere, und in vielen Fällen bevorzugte, Molekulargewichtsregler sind beispielsweise diverse Terpene, insbesondere Terpinene ($\alpha$-Terpinen, $\beta$-Terpinen, $\gamma$-Terpinen), Phellandrene ($\alpha$-Phellandren, $\beta$-Phellandren) und Terpinolen (auch $\delta$-Terpinen genannt), 1,4-Cyclohexadien (gegebenenfalls substituiert), 1,3-Cyclohexadien (gegebenenfalls substituiert), 1,4-Dihydronaphtalin, 1,4,5,8-Tetrahydronaphthalin, 2,5-Dihydrofuran oder dimeres $\alpha$-Styrol (2,4-Diphenyl-4-methyl-1-penten) sowie Linolsäure und $\alpha$-Linolensäure.

Wie bereits oben beschrieben, ist für eine kontrastreiche Darstellung in klassischen Röntgenbildern und DVT-Bildern eine gute Röntgenopazität des erfindungsgemäßen härtbaren Dentalmaterials bzw. des erfindungsgemäßen gehärteten Dentalmaterials notwendig (zur Bestimmung der Röntgenopazität siehe unten unter "Beispiele", Punkt I.b). Besonders bevorzugt ist ein erfindungsgemäßes härtbares Dentalmaterial (wie vorstehend beschrieben, vorzugsweise wie vorstehend als bevorzugt definiert), wobei gilt:

- das härtbare Dentalmaterial besitzt eine Röntgenopazität von 3,5 mm Al (gleichbedeutend mit 350 % Al) oder größer

und/oder

- durch Polymerisation polymerisierbarer Monomere in dem härtbaren Dentalmaterial ist ein gehärtetes Dentalmaterial erhältlich, welches eine Röntgenopazität von 3,5 mm Al (gleichbedeutend mit 350 % Al) oder größer besitzt.

Eine Röntgenopazität in mm Al errechnet sich wie folgt:

$$\text{Röntgenopazität [mm Al]} = (d_{Al} / d_P),$$

wobei

$d_{Al}$ die Dicke in mm eines Aluminiumkeils ist, bei der eine identische Schwärzung auftritt wie bei dem Prüfkörper,

$d_P$ die Prüfkörperdicke des erfindungsgemäßen härtbaren Dentalmaterials bzw. des erfindungsgemäßen gehärteten Dentalmaterials in mm ist

Eine Röntgenopazität von 3,5 mm Al bedeutet, dass der Quotient aus $d_{Al}$ und $d_P$ 3,5 beträgt, d.h. bei identischer Schwärzung beträgt die Dicke des Aluminiumkeils 3,5 Mal die Dicke des Prüfkörpers. Beträgt beispielsweise die Dicke $d_P$ eines Prüfkörpers 1 mm und die Dicke $d_{Al}$ eines entsprechenden Aluminiumkeils 3,5 mm, beträgt die Röntgenopazität 3,5 mm (bzw. 350 %). Ein hoher Wert für mm Al (bzw. Prozent Al) bedeutet somit eine hohe Röntgenopazität.

Vorzugsweise wird die Röntgenopazität am erfindungsgemäßen gehärteten Dentalmaterial (wie vorstehend und insbesondere wie nachfolgend beschrieben, vorzugsweise wie vorstehend und insbesondere wie nachfolgend als bevorzugt beschrieben) bestimmt. Somit ist ein erfindungsgemäßes gehärtetes Dentalmaterial mit einer Röntgenopazität von 3,5 mm Al oder größer bevorzugt, vorzugsweise 4,0 mm Al oder größer.

Eigene Untersuchungen haben gezeigt, dass ein erfindungsgemäßes härtbares Dentalmaterial, vorzugsweise ein daraus durch Polymerisation polymerisierbarer Monomere erhältliches erfindungsgemäßes gehärtetes Dentalmaterial mit einer Röntgenopazität von 3,5 mm Al oder größer in sehr vielen Fällen zu sehr guten Ergebnissen in klassischen Röntgenbildern geführt hat. Insbesondere wurde aufgrund der hohen Röntgenopazität ein sehr guter Kontrast zwischen künstlichem Dentalmaterial und natürlichem Zahnmaterial in diesen Bildern beobachtet. Diese Untersuchungen haben außerdem gezeigt, dass ein solches erfindungsgemäßes härtbares (bzw. gehärtetes) Dentalmaterial in DVT-Bildern ebenfalls regelmäßig zu sehr guten Ergebnissen führt. Aufgrund der hohen Röntgenopazität und der Zusammensetzung des erfindungsgemäßen härtbaren Dentalmaterials wurden in diesen DVT-Bildern einerseits sehr gute Kontraste zwischen künstlichen Dentalmaterialen und natürlichen Zahnmaterialen und andererseits kaum Artefakte beobachtet.

Liegt die Röntgenopazität (deutlich) unter der Grenze von 3,5 mm Al wurde in einigen wenigen Fällen in DVT-Bildern zwar noch ein ausreichender Kontrast zwischen künstlichem Dentalmaterial und natürlichem Zahnmaterial beobachtet, jedoch traten vereinzelt zunehmend Artefakte auf (insbesondere je geringer die Röntgenopazität ist).

Wie bereits oben erwähnt, ist es wünschenswert, dass das erfindungsgemäße härtbare Dentalmaterial (wie vorstehend beschrieben, vorzugsweise wie vorstehend als bevorzugt definiert) eine hohe Transluzenz besitzt. Eine hohe Transluzenz führt dazu, dass das für eine Polymerisation polymerisierbarer Monomere erforderliche Licht auch in tiefere Schichten einer Präparation aus einem erfindungsgemäßen härtbaren Dentalmaterial vordringen kann (gute Durchdringungstiefe). Nur so ist gewährleistet, dass das erforderliche Licht auch in tiefer liegenden Schichten polymerisierbare Monomere erreicht und zu einer gleichmäßigen Aushärtung führt.

Bei der Bewertung der Transluzenz orientiert sich der Praktiker bei der Konzeption härtbarer Dentalmaterialien häufig an einer Teilmischung, die dem finalen Produkt (beispielsweise einem erfindungsgemäßen härtbaren Dentalmaterial) bereits sehr ähnlich ist und eine sehr hohe Transluzenz besitzt. Eine solche Teilmischung enthält üblicherweise keine dem Fachmann geläufigen und in der Praxis üblicherweise eingesetzten partikulären Farbstoffe und auch keine sonstigen Farbmittel (d.h. Farbstoffe und Farbmittel, die für die Veränderung der Farbe oder Tönung eingesetzt werden). Typischerweise besitzen solche Teilmischungen (bzw. durch Polymerisation polymerisierbarer Monomere in der Teilmischung resultierende gehärtete Teilmischungen) eine Transluzenz von 45 % oder größer, vorzugsweise von 50 % oder größer (zur Bestimmung einer Transluzenz siehe Punkt I.c unter "Beispiele" weiter unten im Text).

Ausgehend von der Teilmischung mit sehr hoher Transluzenz besitzt, nach Beimischung ausgewählter/spezifischer partikulärer Farbstoffe und/oder Farbmittel, ein resultierendes erfindungsgemäßes härtbares Dentalmaterial (bzw. ein erfindungsgemäßes gehärtetes Dentalmaterial) definierte Schattierungen bzw. Farben. Solche bevorzugten erfindungsgemäßen Dentalmaterialien (härtbar bzw. gehärtet) besitzen zwar regelmäßig eine etwas verringerte Transluzenz, verglichen mit der Teilmischung, d.h. verglichen mit einem ansonsten identisch zusammengesetzten Dentalmaterial, dem jedoch keine partikulären Farbstoffe sowie Farbmittel beigemischt wurden. Eine gezielt herbeiführbare Verringerung der Transluzenz erlaubt es, ein erfindungsgemäßes Dentalmaterial für spezifische Anwendungen bereitzustellen (z.B. als Dentinersatz: 10 % Transluzenz, Universalfarbton: 20 % Transluzenz, Schmelzersatz: 30 % Transluzenz, Schneidkante: 40 % Transluzenz).

Vorzugsweise wird die Transluzenz an der gehärteten Teilmischung (wie vorstehend beschrieben) bestimmt.

In sehr vielen Fällen gilt (sowohl für die vorstehend genannte Teilmischung als auch für ein erfindungsgemäßes härtbares Dentalmaterial): Liegt die Transluzenz (deutlich) unter 45 % wird regelmäßig eine (deutliche) Verlängerung der Belichtungszeit erforderlich sein, um eine vollständige Aushärtung zu erreichen. Dem Fachmann ist klar, dass der Polymerisationsgrad von der Belichtungsdauer (Licht mit geeigneter Wellenlänge) abhängt. Alternativ kann es in solchen Fällen erforderlich sein, ein Dentalmaterial schichtweise zu applizieren, wobei jede dieser vergleichsweise dünnen Einzelschichten separat ausgehärtet wird (wobei die Aushärtung dieser dünnen Einzelschichten dann wiederum sehr schnell erfolgen kann). Die jeweiligen Einzelschichten verbinden sich beim Aushärten miteinander, so dass abschließend eine Einheit eines gehärteten Dentalmaterials vorliegt.

Ist die Transluzenz vergleichsweise hoch (z.B. deutlich größer 50 %) verkürzt sich die notwendige Belichtungszeit entsprechend.

Es hat sich in eigenen Untersuchungen gezeigt, dass eine gute Durchdringungstiefe des Lichtes im erfindungsgemäßen härtbaren Dentalmaterial (bzw. im erfindungsgemäßen (teil-)gehärteten Dentalmaterial) regelmäßig immer dann erreicht wird, wenn der Brechungsindex $n_A$ und der Brechungsindex $n_{B2}$ dicht beieinander liegen (weitere Details dazu weiter unten im Text).

Allgemein gilt für die Gesamtmenge der Füllstoffpartikel in der Teilmenge (B1) aus

(a) nicht-aggregierten und nicht-agglomerierten Füllstoffpartikeln mit einer Partikelgröße im Bereich von 25 bis 120 nm aus Ytterbiumfluorid
und

(b) nicht-aggregierten und nicht-agglomerierten Füllstoffpartikeln mit einer Partikelgröße im Bereich von 25 bis 120 nm aus Bariumsulfat,

dass diese eine Partikelgröße besitzen, die kleiner ist als die halbe Wellenlänge des sichtbaren Lichtes (Wellenlänge des sichtbaren Lichts: ca. 380 nm bis 780 nm). Diese Eigenschaft erscheint sehr vorteilhaft. Eigene Untersuchungen weisen darauf hin, dass die Füllstoffpartikel der Teilmenge (B1) mit den vorstehend genannten Partikelgrößen (und mit den zu diesen Füllstoffpartikeln gehörenden Brechungsindizes) auf vorteilhafte Weise mit den polymerisierbaren Monomeren der Gesamtmenge (A) zusammenwirken. Eine Mischung aus den polymerisierbaren Monomeren der Gesamtmenge (A) und den Füllstoffpartikeln der Teilmenge (B1) zeigt regelmäßig eine zu vernachlässigende bis gar keine Lichtstreuung, d.h. die Transluzenz einer solchen Mischung verschlechtert sich regelmäßig nicht durch Anwesenheit der Füllstoffpartikel der Teilmenge (B1). Trotzdem wird eine deutliche Verbesserung der Röntgenopazität erreicht. Die zuvor erwähnte Mischung aus polymerisierbaren Monomeren der Gesamtmenge (A) und den Füllstoffpartikeln der Teilmenge (B1) besitzt regelmäßig einen höheren Brechungsindex (nachfolgend vereinfacht als "modifizierter Brechungsindex" bezeichnet) als die polymerisierbaren Monomere der Gesamtmenge (A) alleine (insbesondere wenn die Füllstoffpartikel der Teilmenge (B1) Ytterbiumfluorid umfassen). Dieser modifizierte Brechungsindex einer solchen Mischung erlaubt es in der Praxis, Dentalgläser auszuwählen (und beizumischen), deren Brechungsindizes vergleichsweise hoch sind aber dennoch sehr dicht bei dem modifizierten Brechungsindex liegen. Erfindungsgemäße härtbare Dentalmaterialien, die dieses Prinzip ausnutzen, besitzen einerseits regelmäßig eine sehr gute Transluzenz (aufgrund der sehr dicht beieinanderliegenden Brechungsindizes) und eine sehr gute Röntgenopazität (aufgrund der Füllstoffpartikel der Teilmenge (B1) und ggf. zusätzlicher, röntgenopaker Dentalgläser). Bevorzugt ist ein erfindungsgemäßes härtbares Dentalmaterial (wie vorstehend beschrieben, vorzugsweise wie vorstehend als bevorzugt definiert) mit einem Brechungsindex $n_{härtbar}$ im Bereich von 1,50 bis 1,559.

Neben den bereits oben erwähnten dentalen Gläsern kann die Gesamtmenge (B) eine oder mehrere Verbindungen einer Vielzahl weiterer anorganischer partikulärer Füllstoffe umfassen. In vereinzelten Fällen ist ein erfindungsgemäßes härtbares Dentalmaterial (wie vorstehend beschrieben, vorzugsweise wie vorstehend als bevorzugt definiert) bevorzugt, wobei die zu mischenden Startmaterialien Siliziumdioxid in einer Gesamtmenge von kleiner 0,1 Gew.-%, bezogen auf die Gesamtmasse der zu mischenden Startmaterialien, vorzugsweise gar kein Siliziumdioxid umfassen.

Die röntgenopaken Eigenschaften eines erfindungsgemäßen härtbaren Dentalmaterials (wie vorstehend beschrieben, vorzugsweise wie vorstehend als bevorzugt definiert) werden vorzugsweise durch die Füllstoffpartikel der Teilmenge (B1) erreicht. Besonders bevorzugt ist ein erfindungsgemäßes härtbares Dentalmaterial (wie vorstehend beschrieben, vorzugsweise wie vorstehend als bevorzugt definiert), wobei die partikulären Füllstoffe, welche die Gesamtmenge (B) der partikulären Füllstoffe im härtbaren Dentalmaterial bilden, keine

(a) nicht-aggregierten und nicht-agglomerierten Füllstoffpartikel mit einer Partikelgröße im Bereich von 25 bis 120 nm aus organisch oberflächenmodifiziertem Ytterbiumfluorid enthalten
oder keine

(b) nicht-aggregierten und nicht-agglomerierten Füllstoffpartikel mit einer Partikelgröße im Bereich von 25 bis 120 nm aus organisch oberflächenmodifiziertem Bariumsulfat, enthalten,

vorzugsweise keine

(a) nicht-aggregierten und nicht-agglomerierten Füllstoffpartikel mit einer Partikelgröße im Bereich von 25 bis 120 nm aus Ytterbiumfluorid enthalten
oder keine

(b) nicht-aggregierten und nicht-agglomerierten Füllstoffpartikel mit einer Partikelgröße im Bereich von 25 bis 120 nm aus Bariumsulfat, enthalten.

In diesen Fällen besitzt die Teilmenge (B1) entweder einen Brechungsindex $n_{B1}$ von 1,55 (Ytterbiumfluorid) oder 1,64 (Bariumsulfat). Das erfindungsgemäße härtbare Dentalmaterial umfasst aber mindestens immer einen der zwei zuvor genannten partikulären Füllstoffe, deren Füllstoffpartikel nicht-aggregiert und nicht-agglomeriert sind und eine Partikelgröße im Bereich von 25 bis 120 nm besitzen.

In manchen Fällen ist ein erfindungsgemäßes härtbares Dentalmaterial (wie vorstehend beschrieben, vorzugsweise wie vorstehend als bevorzugt definiert) bevorzugt, wobei die Teilmenge (B1) nur aus nicht-aggregierten und nicht-agglomerierten Füllstoffpartikeln mit einer Partikelgröße im Bereich von 25 bis 120 nm aus (vorzugsweise organisch oberflächenmodifiziertem) Ytterbiumfluorid besteht. In diesem Fall besitzt die Teilmenge (B1) einen Brechungsindex $n_{B1}$ von 1,55. Wie bereits vorstehend ausgeführt, liegt der Brechungsindex $n_A$ der polymerisierbaren Monomere vorzugsweise im Bereich von 1,45 bis 1,55; der Brechungsindex $n_{B2}$ der Teilmenge (B2) (z.B. Dentalgläser) vorzugsweise im Bereich von 1,49 bis 1,62. In einem erfindungsgemäßen härtbaren Dentalmaterial ist es grundsätzlich besonders wünschenswert, dass der Brechungsindex $n_A$ und der Brechungsindex $n_{B2}$ besonders eng beieinander liegen. Wie ebenfalls bereits oben beschrieben, ist der Brechungsindex einer Mischung aus polymerisierbaren Monomeren der Gesamtmenge (A) und den Füllstoffpartikeln der Teilmenge (B1) durch die Anwesenheit der Füllstoffpartikeln der Teilmenge (B1) beeinflusst (verändert), verglichen mit dem Brechungsindex $n_A$ der polymerisierbaren Monomere der Gesamtmenge (A). Beträgt beispielsweise der Brechungsindex $n_A$ der polymerisierbaren Monomere 1,52 und der Brechungsindex $n_{B2}$ der Dentalgläser 1,55, ist es beispielsweise von Vorteil, durch Einsatz von Ytterbiumfluorid mit einem Brechungsindex von 1,55 die Brechungsindices anzugleichen. Beträgt hingegen der Brechungsindex $n_A$ 1,48 und der Brechungsindex $n_{B2}$ 1,61, ist es beispielsweise von Vorteil, durch Einsatz von Bariumsulfat mit einem Brechungsindex von 1,64 die Brechungsindices anzugleichen.

Besonders bevorzugt ist ein erfindungsgemäßes härtbares Dentalmaterial (wie vorstehend beschrieben, vorzugsweise wie vorstehend als bevorzugt definiert), wobei in der Teilmenge (B1), bestehend aus der im härtbaren Dentalmaterial enthaltenen Gesamtmenge an

(a) nicht-aggregierten und nicht-agglomerierten Füllstoffpartikeln mit einer Partikelgröße im Bereich von 25 bis 120 nm aus Ytterbiumfluorid
und

(b) nicht-aggregierten und nicht-agglomerierten Füllstoffpartikeln mit einer Partikelgröße im Bereich von 25 bis 120 nm aus Bariumsulfat,

nicht-aggregierte und nicht-agglomerierte Füllstoffpartikel aus Ytterbiumfluorid mit einer Partikelgröße im Bereich von 30 bis 100 nm, vorzugsweise im Bereich von 30 bis 80 nm, besonders bevorzugt im Bereich von 30 bis 60 nm, insbesondere vorzugsweise im Bereich von 35 bis 55 nm, enthalten sind
und/oder
nicht-aggregierte und nicht-agglomerierte Füllstoffpartikel aus Bariumsulfat mit einer Partikelgröße im Bereich von 30 bis 100 nm, vorzugsweise im Bereich von 30 bis 80 nm, besonders bevorzugt im Bereich von 30 bis 60 nm, insbesondere vorzugsweise im Bereich von 35 bis 55 nm, enthalten sind.

Vorzugsweise gilt das zuvor zu erfindungsgemäßen härtbaren Dentalmaterialien Gesagte bzw. zu erfindungsgemäßen gehärteten Dentalmaterialien Gesagte in Verbindung mit den zuvor genannten bevorzugten bzw. besonders bevorzugten Partikelgrößen entsprechend.

Es hat sich in eigenen Untersuchungen gezeigt, dass die Füllstoffpartikel der Teilmenge (B1) (wie vorstehend beschrieben, vorzugsweise wie vorstehend als bevorzugt definiert) regelmäßig zu einerseits einer sehr guten Röntgenopazität und andererseits zu einer sehr guten Transluzenz beitragen. Gemäß eigenen Untersuchungen wurden regelmäßig besonders dann gute Ergebnisse erzielt, wenn die Teilmenge (B1) Füllstoffpartikel mit einer Partikelgröße im Bereich von 30 bis 80 nm (insbesondere im Bereich von 30 bis 60 nm) enthalten (dies gilt in den meisten Fällen sowohl für Füllstoffpartikel aus Ytterbiumfluorid als auch für Füllstoffpartikel aus Bariumsulfat). Ein erfindungsgemäßes härtbares Dentalmaterial umfassend solche Füllstoffpartikel bzw. ein daraus durch Polymerisation polymerisierbarer Monomere hergestelltes erfindungsgemäßes gehärtetes Dentalmaterial führt in DVT-Bildern in sehr vielen Fällen zu einer deutlichen Reduzierung von Artefakten. Ebenfalls sehr gute Ergebnisse werden regelmäßig erzielt, wenn die Teilmenge (B1) Füllstoffpartikel mit einer Partikelgröße im Bereich von 35 bis 55 nm enthält. Insbesondere führen Füllstoffpartikel der Teilmenge (B1) mit den zuvor genannten bevorzugten und besonders bevorzugten Partikelgrößen regelmäßig zu erfindungsgemäßen härtbaren Dentalmaterialien mit sehr guten Konsistenzen, so dass eine sehr gute Verarbeitbarkeit gegeben ist.

Eigene Untersuchungen haben gezeigt, dass in einigen Fällen die Anzahl der Artefakte in DVT-Bildern zunimmt, wenn die Partikelgröße in einem Bereich (deutlich) kleiner 25 nm liegt (vgl. Beispiele unten im Text). Zusätzlich wurde in einigen Fällen beobachtet, dass das resultierende Dentalmaterial dann keine zufriedenstellende Konsistenz mehr besitzt. Eigene Untersuchungen haben ebenfalls ergeben, dass die Transluzenz in einigen Fällen nicht mehr zufriedenstellend ist, wenn die Partikelgröße 120 nm (deutlich) überschreitet.

Besonders bevorzugt ist ein erfindungsgemäßes härtbares Dentalmaterial (vorzugsweise wie vorstehend beschrieben, besonders bevorzugt wie vorstehend als bevorzugt definiert), herstellbar durch Mischen von Startmaterialien, wobei als zu mischende Startmaterialien ausschließlich eingesetzt werden:

- in einer Gesamtmenge im Bereich von 7,95 bis 27,95 Gew.-%, bezogen auf die Gesamtmasse der zu mischenden Startmaterialien, ein, zwei, drei oder mehr als drei polymerisierbare Monomere, welche die Gesamtmenge (A) der polymerisierbaren Monomere im härtbaren Dentalmaterial bilden und wobei die Gesamtmenge (A) aus dem polymerisierbaren Monomer bzw. aus den polymerisierbaren Monomeren einen Brechungsindex $n_A$ in einem Bereich von 1,45 bis 1,55 besitzt,

- in einer Gesamtmenge im Bereich von 72 bis 92 Gew-%, bezogen auf die Gesamtmasse der zu mischenden Startmaterialien, ein, zwei, drei oder mehr als drei partikuläre Füllstoffe, welche die Gesamtmenge (B) der partikulären Füllstoffe im härtbaren Dentalmaterial bilden,

- in einer Gesamtmenge im Bereich von 0,05 bis 2 Gew.-%, bezogen auf die Gesamtmasse der zu mischenden Startmaterialien, ein, zwei, drei oder mehr als drei Hilfsstoffe, welche die Gesamtmenge (C) der Hilfsstoffe im härtbaren Dentalmaterial bilden,

wobei
das härtbare Dentalmaterial eine der Gesamtmenge (B) zugehörige Teilmenge (B1) umfasst, bestehend aus der im härtbaren Dentalmaterial enthaltenen Gesamtmenge an

(a) nicht-aggregierten und nicht-agglomerierten Füllstoffpartikeln mit einer Partikelgröße im Bereich von 25 bis 120 nm aus Ytterbiumfluorid,

- wobei vorzugsweise die Teilmenge (B1), vorzugsweise zu mehr als 50 Gew.-%, nicht-aggregierte und nicht-agglomerierte Füllstoffpartikel aus Ytterbiumfluorid mit einer Partikelgröße im Bereich von 30 bis 100 nm enthält, besonders bevorzugt im Bereich von 30 bis 80 nm, insbesondere bevorzugt im Bereich von 30 bis 60 nm, ganz besonders bevorzugt im Bereich von 35 bis 55 nm

und

(b) nicht-aggregierten und nicht-agglomerierten Füllstoffpartikeln mit einer Partikelgröße im Bereich von 25 bis 120 nm aus Bariumsulfat,

- wobei vorzugsweise die Teilmenge (B1), vorzugsweise zu mehr als 50 Gew.-%, nicht-aggregierte und nicht-agglomerierte Füllstoffpartikel aus Bariumsulfat mit einer Partikelgröße im Bereich von 30 bis 100 nm enthält, besonders bevorzugt im Bereich von 30 bis 80 nm, insbesondere bevorzugt im Bereich von 30 bis 60 nm, ganz besonders bevorzugt im Bereich von 35 bis 55 nm,

wobei

- der Anteil der Teilmenge (B1)

- 2,5 Gew.-% oder größer als 2,5 Gew.-% ist, vorzugsweise in einem Bereich von 2,5 Gew.-% bis 20 Gew.-% liegt, besonders vorzugsweise in einem Bereich von 5 Gew.-% bis 18 Gew.-%, weiter bevorzugt in einem Bereich von 10,1 Gew.-% bis 18 Gew.-% liegt, besonders bevorzugt in einem Bereich von 10,1 Gew.-% bis 16 Gew.-% liegt, bezogen auf die Gesamtmasse des härtbaren Dentalmaterials

und

- 90 Gew.-% oder größer als 90 Gew.-% ist, bezogen auf die Gesamtmasse an Füllstoffpartikeln aus Ytterbiumfluorid und Bariumsulfat im härtbaren Dentalmaterial

und

- vorzugsweise die Gesamtmenge der partikulären Füllstoffe in der Teilmenge (B1) einen Brechungsindex $n_{B1}$ im Bereich von 1,55 bis 1,64 besitzt.

Die vorliegende Erfindung betrifft auch ein erfindungsgemäßes härtbares Dentalmaterial (wie vorstehend beschrieben, vorzugsweise wie vorstehend als bevorzugt definiert) zur Anwendung (Verwendung) in einem Verfahren zur chirurgischen oder therapeutischen Behandlung des menschlichen oder tierischen Körpers und/oder zur Anwendung (Verwendung) in einem Diagnostizierverfahren, das am menschlichen oder tierischen Körper vorgenommen wird (auch bezeichnet als härtbares Dentalmaterial zur Anwendung in einem Verfahren zur therapeutischen Behandlung des menschlichen Körpers und/oder in einem Diagnostizierverfahren),
vorzugsweise zur spezifischen Anwendung

- in einem therapeutischen Verfahren zum temporären oder permanenten Füllen einer dentalen Kavität,

wobei die dentale Kavität vorzugsweise aus der Gruppe ausgesucht ist bestehend aus Kavitäten im Zahnschmelz, Kavitäten im Zahnzement, Kavitäten im Dentin einer Zahnkrone und Kavitäten im Dentin einer Zahnwurzel in Form eines Sackloches (Anmerkung: der Begriff "dentale Kavität" wird vom Fachmann regelmäßig nicht für Zahnwurzelkanäle benutzt; die erfindungsgemäße spezifische Anwendung betrifft entsprechend nicht das Füllen von Wurzelkanälen),oder

- in einem therapeutischen Verfahren als

- Zahnfüllungsmaterial,

- Dentalzement,

- dentales Unterfüllungsmaterial,

- als fliessfähiges Kompositmaterial (Flow-Material),

- als Kronenmaterial,

- als Inlay und/oder Onlay,

- als Brückenmaterial

- und/oder als Stumpfaufbaumaterial.

Wie bereits oben beschrieben, kann durch Polymerisation (vorzugsweise wie oben beschrieben) polymerisierbarer Monomere (vorzugsweise wie oben beschrieben) in einem erfindungsgemäßen härtbaren Dentalmaterial (wie vorstehend beschrieben, vorzugsweise wie vorstehend als bevorzugt definiert) ein erfindungsgemäßes gehärtetes Dentalmaterial erhalten werden. Somit betrifft die vorliegende Erfindung auch ein gehärtetes Dentalmaterial, erhältlich durch Polymerisation polymerisierbarer Monomere in einem erfindungsgemäßen härtbaren Dentalmaterial (wie vorstehend beschrieben, vorzugsweise wie vorstehend als bevorzugt definiert).

Das oben zu dem erfindungsgemäßen härtbaren Dentalmaterial Gesagte gilt für das erfindungsgemäße gehärtete Dentalmaterial entsprechend.

Die vorliegende Erfindung betrifft auch die Verwendung eines erfindungsgemäßen härtbaren Dentalmaterials (wie vorstehend beschrieben, vorzugsweise ein härtbares Dentalmaterial wie vorstehend als bevorzugt definiert), zur Herstellung eines dentalen Erzeugnisses, wobei die Herstellung nicht am menschlichen oder tierischen Körper erfolgt, vorzugsweise zur Herstellung eines dentalen Erzeugnisses ausgewählt aus der Gruppe bestehend aus künstlichen Zähnen, Inlays, Onlays, Kronen, Brücken, Fräsrohlingen, Implantaten und Zahnfertigteilen.

Zusätzlich lassen sich die erfindungsgemäßen härtbaren Dentalmaterialien hervorragend für generative Fertigungsverfahren aus der Kategorie des "Rapid Prototyping" / "Rapid Manufacturing" (sogenanntes "Rapid Product Development") verwenden. Diese Verfahren beginnen in der Regel mit einer digitalen dreidimensionalen Erfassung der Mundsituation durch einen 3D-Scan (chairside: intraoral oder labside: extraoral), um anschließend auf Basis der generierten CAD-Daten ein dentales Erzeugnis (auch dentales Formteil genannt) zu fertigen. Hierbei werden dentale Erzeugnisse durch sukzessiven Schichtaufbau aus flüssigen bis pastösen härtbaren Dentalmaterialien hergestellt, vorzugsweise aus erfindungsgemäßen härtbaren Dentalmaterialien, beispielsweise durch 3D-Druck, Stereolithografie, Digital Light Processing etc. Dentale Erzeugnisse wie Inlays, Onlays, Kronen, Brücken, Provisorien sowie KFO-Produkte (Kieferorthopädie-Produkte) können (i) direkt vor Ort in der Zahnarztpraxis, (ii) ohne hohen zeitlichen und präparativen Aufwand sowie (iii) ohne hohen Materialverlust angefertigt werden. Zur Herstellung solcher dentalen Erzeugnisse mittels stereolithografischen Verfahren werden flüssige bzw. pastöse strahlungshärtbare Dentalmaterialien eingesetzt, vorzugsweise entsprechende erfindungsgemäße härtbare Dentalmaterialien, wie vorstehend beschrieben. Die schichtweise Aushärtung wird durch punktuelle oder mittels Maskensystem selektive Belichtung mit aktinischer Strahlung erreicht.

Besonders bevorzugt ist die Verwendung eines erfindungsgemäßen härtbaren Dentalmaterials (wie vorstehend beschrieben, vorzugsweise ein härtbares Dentalmaterial wie vorstehend als bevorzugt definiert) als Baumaterial in einem generativen, ein digitales Datenmodell verwendenden Fertigungsverfahren, vorzugsweise im 3D-Druck, vorzugsweise zur Herstellung eines dentalen Erzeugnisses, vorzugsweise zur Herstellung eines dentalen Erzeugnisses ausgewählt aus der Gruppe bestehend aus künstlichen Zähnen, Inlays, Onlays, Kronen, Brücken, Fräsrohlingen, Implantaten und Zahnfertigteilen. Eine solche Herstellung dentaler Erzeugnisse erfolgt selbstverständlich nicht am menschlichen oder tierischen Körper,

Die vorliegende Erfindung betrifft auch ein Verfahren zur Herstellung eines dentalen Erzeugnisses mittels eines generativen, ein digitales Datenmodell verwendenden Fertigungsverfahrens umfassend die Schritte

- Herstellen oder Bereitstellen eines erfindungsgemäßen härtbaren Dentalmaterials (wie vorstehend beschrieben, vorzugsweise ein härtbares Dentalmaterial wie vorstehend als bevorzugt definiert), vorzugsweise Herstellen nach einem erfindungsgemäßen Verfahren wie nachfolgend beschrieben (vorzugsweise wie nachfolgend als bevorzugt definiert),

- Verarbeiten des hergestellten bzw. bereitgestellten härtbaren Dentalmaterials in dem generativen, ein digitales Datenmodell verwendenden Fertigungsverfahren, so dass das dentale Erzeugnis oder eine Vorstufe des dentalen Erzeugnisses resultiert,

wobei das dentale Erzeugnis vorzugsweise ausgewählt ist aus der Gruppe bestehend aus künstlichen Zähnen, Inlays, Onlays, Kronen, Brücken, Fräsrohlingen, Implantaten und Zahnfertigteilen.

Das erfindungsgemäße Herstellverfahren erfolgt üblicherweise nicht am menschlichen oder tierischen Körper; das Verarbeiten des hergestellten bzw. bereitgestellten härtbaren Dentalmaterials in dem generativen, ein digitales Datenmodell verwendenden Fertigungsverfahren erfolgt vorzugsweise in einem Fertigungsraum einer Apparatur zur Durchführung dieses generativen Fertigungsverfahrens.

Die vorliegende Erfindung betrifft auch ein Kit, umfassend

- eine oder mehr als eine dentale Spritze
  und

  (i) ein, zwei oder mehr als zwei erfindungsgemäße härtbare Dentalmaterialien (wie vorstehend beschrieben, vorzugsweise wie vorstehend als bevorzugt definiert)
  oder
  (ii) eine, zwei oder mehr als zwei Basispasten und eine, zwei oder mehr als zwei Katalysatorpasten, wobei durch Mischen einer Basispaste und der dazugehörigen Katalysatorpaste ein erfindungsgemäßes härtbares Dentalmaterial (wie vorstehend beschrieben, vorzugsweise wie vorstehend als bevorzugt definiert) erhältlich ist, welches einen Polymerisationsinitiator umfasst.

Der Einsatz von Basis- und Katalysatorpasten ist üblicherweise notwendig, wenn das Kit ein erfindungsgemäßes härtbares Dentalmaterial zur Verfügung stellen soll, das (i) chemisch härtbar oder (ii) dualhärtend ist.

Dem Fachmann sind diverse Initiatoren für eine chemische Aushärtung bekannt (es sei insoweit auf EP 1 720 506 A1 verwiesen).

Bevorzugte Initiatoren zur chemischen Härtung sind Benzoylperoxid, Lauroylperoxid insbesondere Dibenzoylperoxid in Kombination mit Aminen wie N,N-Dimethyl-p-toluidin, N,N-Dihydroxyethyl-p-toluidin sowie strukturverwandten Aminen.

Die für eine chemische Härtung notwendigen Initiatoren (z.B. Peroxide, Amine bzw. entsprechende Kombinationen von Peroxiden und Aminen) werden üblicherweise auf entsprechende Basis- und Katalysatorpasten aufgeteilt. Beim Mischen der aminhaltigen Komponente (Basispaste) mit der peroxidhaltigen Komponente (Initiator- oder Katalysatorpaste) wird durch die Reaktion von Amin und Peroxid (Redoxreaktion) die radikalische Reaktion initiiert.

Dualhärtende Systeme umfassen eine Kombination aus Photoinitiatoren und Initiatoren zur chemischen Aushärtung (zu Photoinitiatoren siehe weiter oben im Text).

Beispielsweise kann die Basispaste zusätzlich einen Photoinitiator enthalten, so dass die Basispaste entweder allein als lichthärtender oder zusammen mit der Initiatorpaste als licht- und selbsthärtender Dentalwerkstoff eingesetzt werden kann.

Neben den oxidativ wirksamen organischen Peroxidverbindungen können als Redoxsysteme auch Barbitursäuren bzw. Barbitursäurederivate sowie Malonylsulfamide verwendet werden.

Von den Barbitursäuresystemen sind die sogenannten "Bredereck-Systeme" von hoher Bedeutung. Beispiele geeigneter "Bredereck-Systeme" sowie Verweise auf die entsprechende Patentliteratur findet man in EP 1 839 640 A2 sowie in DE 1495520 A1, WO 02/092021 A1 oder in WO 02/092023 A1.

Geeignete Malonylsulfamide sind in EP 0 059 451 A1 beschrieben. Bevorzugte Verbindungen sind 2,6-Dimethyl-4-isobutylmalonylsulfamid, 2,6-Diisobutyl-4-propylmalonyl-sulfamid, 2,6-Dibutyl-4-propylmalonylsulfamid, 2,6-Dimethyl-4-ethylmalonylsulfamid sowie 2,6-Diocytyl-4-isobutylmalonylsulfamid.

Ferner können Schwefelverbindungen in der Oxidationsstufe +2 oder +4 wie Natriumbenzolsulfinat oder Natriumparatoluolsulfinat eingesetzt werden.

Zur Beschleunigung der Aushärtung kann die Polymerisation in Gegenwart von Schwermetallverbindungen wie Ce, Fe, Cu, Mn, Co, Sn oder Zn durchgeführt werden, wobei Kupferverbindungen besonders bevorzugt sind. Die Schwermetallverbindungen werden bevorzugt in Form löslicher organischer Verbindungen eingesetzt. Bevorzugte Kupferverbindungen sind dabei Kupferbenzoat, Kupferacetat, Kupferethylhexanoat, Kupferdi(methacrylat), Kupferacetylacetonat und Kupfernaphthenat.

Die dentale(n) Spritze(n) ist (sind) für dentale Zwecke geeignet, d.h. geeignet für Applikationen im Mundraum.

Sofern ein erfindungsgemäßes Kit (wie vorstehend beschrieben) eine oder mehrere Basispasten und eine oder mehrere Katalysatorpasten umfasst, bildet die Basispaste (bzw. die Basispasten) und die dazugehörige Katalysatorpaste (bzw. die dazugehörigen Katalysatorpasten) jeweils eine Teilmischung aus den oben beschriebenen ausschließlich eingesetzten Startmaterialien. In diesem Fall bedeutet das, dass das oben beschriebene Mischen der Startmaterialien erst durch Mischen einer Basispaste und der entsprechenden Katalysatorpaste abgeschlossen ist. Es resultiert dann ein erfindungsgemäßes härtbares Dentalmaterial (wie vorstehend definiert, vorzugsweise ein härtbares Dentalmaterial wie vorstehend als bevorzugt definiert).

Vorzugsweise umfasst das erfindungsgemäße Kit (wie vorstehend definiert, vorzugsweise wie vorsehend als bevorzugt beschrieben) zusätzlich einen, mehr als einen oder sämtliche der Gegenstände ausgewählt aus der Gruppe bestehend aus:

- ein, zwei oder mehr als zwei Bondings,
- ein, zwei oder mehr als zwei Ätzgele,
- eine oder mehr als eine Farbskala,
- einen oder mehr als einen Pinsel,

- ein oder mehr als ein Dentalmaterial mit einer Viskosität, die von der/den Viskosität(en) des/der erfindungsgemäßen härtbaren Dentalmaterials bzw. Dentalmaterialien und/oder von der/den Viskosität(en) der Basispaste(n) bzw. der Katalysatorpaste(n) verschieden ist, vorzugsweise ein oder mehr als ein Flow-Material.

Das oben zu dem erfindungsgemäßen härtbaren Dentalmaterial bzw. zu dem erfindungsgemäßen gehärteten Dentalmaterial Gesagte gilt für die Verwendung des erfindungsgemäßen härtbaren Dentalmaterials bzw. für die Verwendung des erfindungsgemäßen gehärteten Dentalmaterials entsprechend, insbesondere für die Herstellung von dentalen Erzeugnissen ausgewählt aus der Gruppe bestehend aus künstlichen Zähnen, Inlays, Onlays, Kronen, Brücken, Fräsrohlingen, Implantaten und Zahnfertigteilen und/oder für die dentalen Erzeugnissen ausgewählt aus der Gruppe bestehend aus künstlichen Zähnen, Inlays, Onlays, Kronen, Brücken, Fräsrohlingen, Implantaten und Zahnfertigteilen selbst.

Die vorliegende Erfindung betrifft außerdem ein Verfahren zur Herstellung eines erfindungsgemäßen härtbaren Dentalmaterials (wie vorstehend beschrieben, vorzugsweise ein härtbares Dentalmaterial wie vorstehend als bevorzugt definiert) umfassend die folgenden Schritte:

(i) Herstellen oder Bereitstellen der Startmaterialien wie oben definiert (vorzugsweise Startmaterialien, wie vorzugsweise vorstehend als bevorzugt definiert),
oder
Herstellen oder Bereitstellen von Teilmischungen aus den Startmaterialien wie oben definiert (vorzugsweise Startmaterialien, wie vorzugsweise vorstehend als bevorzugt definiert),

(ii) Mischen der gemäß (i) hergestellten bzw. bereitgestellten Startmaterialien bzw. der gemäß (i) hergestellten bzw. bereitgestellten Teilmischungen, so dass jeweils das härtbare Dentalmaterial resultiert.

Das erfindungsgemäße Herstellverfahren erfolgt vorzugsweise nicht am menschlichen oder tierischen Körper; dies gilt sowohl für den Schritt (i) als auch für den Schritt (ii). Vorzugsweise erfolgt in Schritt (ii) das Mischen in einem Mischraum einer Mischvorrichtung.

Die vorliegende Erfindung betrifft auch ein Verfahren zur Herstellung eines erfindungsgemäßen gehärteten Dentalmaterials (wie vorstehend beschrieben, vorzugsweise ein gehärtetes Dentalmaterial wie vorstehend als bevorzugt definiert) oder eines dentalen Erzeugnisses wie vorstehend definiert (vorzugsweise eines dentalen Erzeugnissen, wie vorstehend als bevorzugt definiert), umfassend die Schritte:

(I) Herstellen oder Bereitstellen eines erfindungsgemäßen härtbaren Dentalmaterials (wie vorstehend beschrieben, vorzugsweise ein härtbares Dentalmaterial wie vorstehend als bevorzugt definiert), vorzugsweise Herstellen nach einem erfindungsgemäßen Verfahren zur Herstellung eines erfindungsgemäßen härtbaren Dentalmaterials (ein Verfahren, wie vorstehend beschrieben, vorzugsweise wie vorstehend als bevorzugt beschrieben),

(II) Polymerisieren polymerisierbarer Monomere in dem härtbaren Dentalmaterial, so dass das gehärtete Dentalmaterial resultiert.

Zusätzlich wird beschrieben ein Verfahren zum dentalen Behandeln eines Patienten umfassend die folgenden Schritte:

(1) Herstellen oder Bereitstellen eines erfindungsgemäßen härtbaren Dentalmaterials (wie vorstehend beschrieben, vorzugsweise ein härtbares Dentalmaterial wie vorstehend als bevorzugt definiert) oder eines erfindungsgemäßen gehärteten Dentalmaterials (wie vorstehend beschrieben, vorzugsweise ein gehärtetes Dentalmaterial wie vorstehend als bevorzugt definiert),

(2) Einbringen und Positionieren des hergestellten bzw. bereitgestellten härtbaren Dentalmaterials in der Mundhöhle des Patienten und Härten des härtbaren Dentalmaterials bzw.

Einbringen und Positionieren des hergestellten bzw. bereitgestellten gehärteten Dentalmaterials in der Mundhöhle des Patienten.

Beschrieben wird auch die Verwendung einer Menge an

(a) nicht-aggregierten und nicht-agglomerierten Füllstoffpartikeln mit einer Partikelgröße im Bereich von 25 bis 120 nm aus Ytterbiumfluorid
und/oder

(b) nicht-aggregierten und nicht-agglomerierten Füllstoffpartikeln mit einer Partikelgröße im Bereich von 25 bis 120 nm aus Bariumsulfat,

als röntgenopaker Füllstoff in einem härtbaren Dentalmaterial.

Das vorstehend zu einem erfindungsgemäßen härtbaren/gehärteten Dentalmaterial Gesagte gilt für die erfindungsgemäße Verwendung der vorstehend definierten Menge entsprechend.

Nachfolgend wird die Erfindung anhand von Beispielen näher erläutert.

Beispiele:

I. Bestimmungsmethoden:

I.a Bestimmung der Brechungsindices:

Brechungsindices wurden bei 20°C und für Licht einer Wellenlänge von 589 nm (Natrium-D-Linie) bestimmt. Für fließfähige und pastöse Proben wurde ein analoges Abbe-Refraktometer AR4 der Firma A. Krüss Optronic bzw. ein digitales Gerät RE 40 der Firma Mettler-Toledo verwendet.

Sofern ein Brechungsindex pulverförmiger, partikulärer Füllstoffe nicht vom Hersteller angegeben wurde, wurde der Brechungsindex mittels der Immersionsmethode bestimmt. Hierzu wurden vergleichend Immersionsflüssigkeiten mit bekannten Brechungsindizes verwendet. Die Bestimmung wurde wie folgt durchgeführt:

In einem ersten Schritt wurden von dem zu untersuchenden Füllstoffpulver mehrere Einzelproben auf Mikroskop-Objektträgern bereitgestellt. Jede dieser Einzelproben wurde in einem zweiten Schritt mit einer spezifischen Immersionsflüssigkeit benetzt. Die spezifischen Immersionsflüssigkeiten unterschieden sich dabei in Ihren Brechungsindices. In einem dritten Schritt wurden die benetzten Einzelproben unter einem Lichtmikroskop des Typs BA 210 LED der Firma Motic untersucht und der Brechungsindex durch Vergleich ermittelt. Der Brechungsindex einer Einzelprobe stimmt mit dem Brechungsindex einer Immersionsflüssigkeit überein, wenn unter dem Mikroskop keine Phasengrenze erkennbar war.

I.b Bestimmung der Röntgenopazität:

Die Röntgenopazität gehärteter Dentalmaterialien wurde gemäß DIN ISO 4049:2010-03 (dort als Röntgensichtbarkeit bezeichnet) mittels eines digitalen Gerätes bestimmt. Die Proben wurden bei 60 kV und 7 mA für jeweils 0,04 s belichtet.

I.c Bestimmung der Transluzenz:

Eine Transluzenz wurde an Prüfkörpern gehärteter Dentalmaterialien bestimmt.

Die angefertigten Prüfkörper wurden mit einem Zweistrahl-Spektralphotometer ColorFlex EZ 45/0 der Firma Hunterlab untersucht. Die Untersuchung erfolgte unter D65 Normlicht und einem 10° Beobachtungswinkel im Reflektionsmodus gegen eine schwarze und eine weiße Kachel. Es wurden die Tristimulus-Werte $Y_{schwarz}$ und $Y_{weiß}$ bestimmt und anschließend die Opazität in Prozent gemäß der Formel Opazität = 100 * $Y_{schwarz}$ / $Y_{weiß}$ berechnet.

In einem dritten Schritt wurde die berechnete Opazität in eine Transluzenz gemäß der Formel Transluzenz = 100 minus Opazität umgerechnet.

Zur Herstellung der Prüfkörper wurden die gemäß Tabelle 1 definierten härtbaren Dentalmaterialien eingesetzt (vgl. zusätzlich unten im Text Punkt III.a).

I.d Bestimmung der Partikelgrößen:

Partikelgrößen wurden mittels Transmissionselektronenmikroskopie (TEM) bestimmt, sofern keine Herstellerangaben verfügbar waren. Weitere Details zur Bestimmung mittels TEM siehe Riwotzki et al., "Liquid-Phase Synthesis of Doped Nanoparticles: Colloids of Luminescing LaPO4:Eu and CePO4:Tb Particles with a Narrow Particle Size Distribution", J. Phys. Chem. B 2000, 104, 2824-2828.

I.e Qualitative und quantitative Charakterisierung der Füllstoffpartikel:

Die nachfolgend beschriebenen Schritte in der qualitativen und quantitativen Charakterisierung der Füllstoffpartikel (insbesondere von Füllstoffpartikel mit einer Partikelgröße in einem Bereich von 25 bis 120 nm) sind dem Fachmann gut bekannt und in der Literatur beschrieben.

I.e.i Harz-/Füllstofftrennung:

In einem ersten Schritt werden 1 g eines härtbaren Dentalmaterials (nachfolgend auch als dentales Kompositmaterial bzw. dentales Komposit bezeichnet) in 10 ml Aceton resuspendiert und die erhaltene Suspension anschließend mit einer Zentrifuge für 10 min bei 5000 U/min zentrifugiert. Der Überstand (nachfolgend Harzphase genannt) wird in ein Sammelglas abdekantiert und der Rückstand in 5 ml Aceton aufgeschlämmt. Es wird erneut für 10 min bei 5000 U/min zentrifugiert, dekantiert und der Rückstand in 5 ml Aceton erneut aufgeschlämmt. Die Schritte Zentrifugieren, Dekantieren und Aufschlämmen werden noch zweimal unter identischen Bedingungen wiederholt. Die von den Harzphasen getrennte Gesamtmenge an Rückständen wird getrocknet und die Gesamtmenge an Harzphasen am Rotationsverdampfer von Aceton befreit.

Nach Durchführung des ersten Schrittes umfasst die getrocknete Gesamtmenge an Rückständen regelmäßig solche Füllstoffpartikel, die eine Partikelgröße von ca. 400 nm oder größer 400 nm besitzen (nachfolgend makroskopische Füllstoffpartikel genannt). Die von Aceton befreite Gesamtmenge an Harzphasen (nachfolgend Harzanteil genannt) umfasst neben polymerisierbaren Monomeren außerdem regelmäßig Füllstoffpartikel mit einer Partikelgröße von ca. 400 nm oder insbesondere kleiner 400 nm (nachfolgend nanoskalige Teilchen genannt). Dieses Verfahren stellt somit sicher, dass das dentale Kompositmaterial durch die Zentrifugation in (i) einen Anteil makroskopischer Füllstoffpartikel, wobei speziell die dentalen Gläser im Größenordnungsbereich von > 400 nm bis in den hohen Mikrometerbereich betroffen sind, und (ii) einen Harzanteil umfassend nanoskaligen Teilchen vollständig aufgetrennt wird.

Die nanoskaligen Teilchen, die sich in dem Harzanteil befinden, können sowohl nicht-aggregierte und nicht agglomerierte röntgenopake Partikel sein (z.B. Füllstoffpartikel der Teilmenge (B1)) als auch nicht-röntgenopake Aerosile, die als pyrogene Kieselsäuren in Form von Aggregaten und/oder Agglomeraten mit einer Partikelgröße z.B. in einem Bereich von ca. 150 nm bis ca. 300 nm vorliegen. Füllstoffpartikel der Teilmenge (B1) befinden sich regelmäßig im Harzanteil.

Der Gesamtmassenanteil anorganischer Partikel in dem Harzanteil wird durch Differenzwiegung nach Veraschung eines entsprechenden Harzanteils gravimetrisch bestimmt.

I.e.ii TEM in Kombination mit EELS:

In einem zweiten Schritt werden die Füllstoffpartikel in dem Harzanteil einer qualitativen und quantitativen Charakterisierung unterzogen. Hierzu wird TEM (Transmissionselektronenmikroskopie) in Verbindung mit EELS (Elektronenenergieverlustspektroskopie) eingesetzt.

Mittels TEM werden die Partikelgrößen der einzelnen Partikel sowie deren Anzahl ermittelt; eine Elementarbestimmung einzelner Partikel erfolgt mittels EELS.

Zur Durchführung der kombinierten TEM/EELS-Charakterisierung wird in einem ersten Schritt durch Verdünnung mit härtbarem Harz (Verdünnerharz) die Konzentration der nanoskaligen Teilchen im Harzanteil zunächst reduziert (Verdünnung: 1 Volumenanteil Harzanteil und 99 Volumenanteile Verdünnerharz). Dadurch wird weitestgehend ausgeschlossen, dass eine "Überlagerung" von nanoskaligen Teilchen in den späteren Bildern beobachtet wird. Eine solche "Überlagerung" würde die Partikelcharakterisierung verfälschen.

Aus den durch Verdünnung mit härtbarem Harz gewonnenen verdünnten Harzanteilen werden in einem zweiten Schritt durch Aushärtung Stäbchen hergestellt. Aus diesen Stäbchen werden anschließend mit einem Ultradiamantmesser (beispielsweise Ultramikrotom ULTRCAT UCT, LEICA, Wetzlar) mehrere 300 nm dünne Ultradünnschnitte angefertigt. Die Ultradünnschnitte werden zur Stabilisierung auf Kupfer-TEM-Grids transferiert. Es resultieren Dünnschnittpräparate. Diese Dünnschnittpräparate werden dann mit 120 kV Beschleunigungsspannung in einem TEM mittels Hellfeld-Abbildungen untersucht.

Eine TEM-Untersuchung an den vorstehend beschriebenen Dünnschichtpräparaten erlaubt es, nicht aggregierte und nicht agglomerierte nanoskalige Partikel von aggregierten und/oder agglomerierten Partikeln (z.B. Kieselsäuren, wie beispielsweise Aerosilen), zu unterscheiden (zur Identifizierung der chemischen Zusammensetzung siehe die nachfolgenden Ausführungen).

Sofern hochauflösende Abbildungen untersucht werden sollen, können Ultradünnschnitte mit Schichtdicken kleiner 100 nm hergestellt und untersucht werden.

In einem dritten Schritt werden die Füllstoffpartikel in den Ultradünnschnitten bzw. Dünnschnittpräparaten mittels EELS-Punktanalysen chemisch charakterisiert, so dass die chemische Zusammensetzung einzelner Partikel bekannt wird (zur Bestimmung der Oberflächenmodifikation von Partikeln siehe nachfolgend Punkt I.f).

Die volumen- oder gewichtsbezogenen Anteile von (ggf. auch mehrerer) Partikelfraktionen werden in einem vierten Schritt wie folgt aus einer TEM-Aufnahme ermittelt: Der im Mikroskop betrachtete Bildausschnitt einer TEM-Aufnahme stellt eine Fläche dar, deren Kantenlängen a und b mittels der Legende bestimmt werden. Multipliziert mit der Dicke c des Ultradünnschnittes ergibt ein Gesamtvolumen $V_{Gesamt}$ für den in der TEM betrachteten Bereich. Dieses Gesamtvolumen $V_{Gesamt}$ ist die Summe aus dem Harzvolumen $V_{Harz}$ und dem Volumen aller Partikel $V_{Partikel}$ innerhalb dieses Volumens (das Volumen aller Partikel umfasst ggf. mehrere Gruppen von Partikeln, z.B. sortiert nach verschiedenen Kriterien wie z.B. der Größe). Es gilt VGesamt = a * b * c = $V_{Harz}$ + $V_{Partikel}$.

Das Volumen einzelner Partikel (und damit das Volumen aller Partikel in dem betrachteten Volumen) ist kalkulatorisch zugänglich über das Kugelvolumen der einzelnen Partikel. Hierzu wird in der TEM-Aufnahme der Durchmesser bzw. Radius eines entsprechenden Partikels bestimmt. Das daraus errechnete Kugelvolumen, multipliziert mit der Dichte des entsprechenden Materials, aus dem das Partikel besteht (Material identifizierbar mittels EELS), ergibt die Masse des Partikels. Das Harzvolumen, zugänglich aus dem Gesamtvolumen minus dem Partikelvolumen, multipliziert mit der Harzdichte, ergibt die Harzmasse. Die Harzdichte ergibt sich aus der Dichte des zur Verdünnung eingesetzten Harzes und ggf. der Dichte des verdünnten Harzanteils (letztere kann ggf. bei der Berechnung der Harzdichte vernachlässigt werden, wenn der Anteil des verdünnten Harzes vernachlässigbar ist). Der Anteil der Partikel (oder einer Gruppe von Partikeln) in Gewichtsprozent errechnet sich aus $m_p$*100/($m_{partikel}$+$m_{Harz}$), wobei $m_P$ die Masse der betrachteten Partikelfraktion in dem betrachteten Volumen, $m_{Partikel}$ die Masse aller Partikel im betrachteten Volumen und $m_{Harz}$ die Masse des Harzes in dem betrachteten Volumen bedeutet. Bei der abschließenden Berechnung des Gewichtsanteils der zu betrachtenden Partikelfraktion wird der Verdünnungsfaktor entsprechend berücksichtigt.

I.f Bestimmung organischer Oberflächenmodifikationen:

I.f.i Vorabbetrachtung:

Viele bekannte röntgenopake Füllstoffmaterialien (wie z.B. Ytterbiumfluorid oder Bariumsulfat) weisen den Nachteil auf, dass sie nur schwer in die Matrix (Harzmatrix) aus polymerisierbaren Monomeren (die sogenannte organische Harzphase) einzuarbeiten sind, weil sie keine hinreichenden chemischen Bindungen (Anbindungsmöglichkeiten) mit den hydrophoben Gruppen des Mediums eingehen. Glasartige Füllstoffe lassen sich beispielsweise mit Hilfe der Silanisierung über Si-OH Gruppen hervorragend in die Harzmatrix dentaler Kompositmaterialien einarbeiten. Bei Ytterbiumfluorid und Bariumsulfat sind solche Gruppen auf den Oberflächen nicht vorhanden; sie sind somit nicht silanisierbar und führen in einem gehärteten Dentalmaterial zu einer unzureichenden physikalischen und chemischen Resistenz (siehe hierzu WO 2005/011621 A1, Seite 2 unten).

Die in einem erfindungsgemäßen härtbaren Dentalmaterial eingesetzten röntgenopaken nanoskaligen Teilchen werden somit auf ihren Oberflächen keine Silane aufweisen. Vielmehr erfolgt die Verknüpfung über Stickstoff-, Sauerstoff-, Schwefel- und/oder Phosphoratomen (siehe hierzu wiederum WO 2005/011621 A1 sowie unsere Ausführungen weiter oben im Text).

I.f.ii Abtrennung polymerisierbarer Monomere von nanoskaligen Teilchen:

I.f.ii.a "Cross-Flow"-Verfahren:

Die Abtrennung polymerisierbarer Monomere von nanoskaligen Teilchen erfolgt beispielsweise in einem dem Fachmann bekannten "Cross-Flow"-Verfahren mittels Ultrafiltrationsmembranen.

Hierbei wird ein Harzanteil (wie vorstehend bereits beschrieben) enthaltend nanoskalige Teilchen, polymerisierbare Monomere und ggf. ein geeignetes Verdünnungsmittel aus einem Behältnis mittels einer Pumpe in einen Kreislauf aus bestimmten Membranen gepumpt, wobei die polymerisierbaren Monomere die Poren der Membranen passieren und als Filtrat separiert werden, während die nanoskaligen Teilchen innerhalb des Kreislaufs (und damit im Behältnis) verbleiben.

Für diesen Trennungsgang ist beispielsweise das System "Vivaflow 50" von "Sartorius Stedim Biotech GmbH, Göttingen" geeignet. Pumpenantrieb (7554-95) und Pumpenkopf entstammen der Reihe "Masterflex L/S" von "Cole-Palmer Instrument Co.", Illinois, USA. Der Betrieb der Pumpe wird während der Filtration auf 2,5 bar eingestellt. Zwei Trennmembranen des Typs "50,000 MWCO (PES)" werden hintereinander geschaltet. Der MWCO (Molecular Weight Cut Off) gibt hierbei die Trenngrenze an, d.h. die Größe der Moleküle, die noch effizient die Membran passieren können. Dieser Wert wird in Dalton angegeben. Die erhaltenen Fraktionen werden anschließend wie unter Punkt I.f.iii beschrieben, untersucht.

I.f.ii.b Sedimentations-Feld-Fluss-Fraktionierung (SF3):

Alternativ zu einer gemäß Schritt I.f.ii.a beschriebenen Vorgehensweise kann eine Sedimentations-Feld-Fluss-Fraktionierung (SF3) durchgeführt werden. Dabei lassen sich insbesondere unterschiedliche Partikelfraktionen voneinander und zusätzlich vom Harzanteil trennen. Voraussetzung hierbei ist, dass sich die unterschiedlichen Partikelfraktionen ausreichend in Größe und/oder Dichte voneinander unterscheiden.

Entsprechende Geräte, die eine hierfür notwendige Trennsäule enthalten, sind bei der Firma Postnova Analytics

EP 3 011 949 B1

GmbH, Landsberg, erhältlich. Das die Trennsäule enthaltende Modul trägt die Bezeichnung CF2000 Centrifugal FFF und wird durch die weiteren Module PN7140 (Eluent Organizer), PN1130 (Isocratic Pump), PN5300 (Autosampler), PN3621 MALS (21-Multi-Angle Light Scattering Detector) und PN8050 (Fraction Collector) ergänzt. In dieser Kombination erlaubt die Centrifugal FFF-Anlage nicht nur die analytische, sondern auch die präparative Trennung von Partikelfraktionen. Die erhaltenen Fraktionen werden anschließend wie unter Punkt I.f.iii beschrieben, untersucht.

I.f.iii Charakterisierung der Oberflächenmodifikation:

Eine gemäß I.f.ii.a hergestellte und anschließend von Lösungsmitteln befreite Probe, die nanoskalige Teilchen in Form eines Pulvers enthält oder eine gemäß I.f.ii.b hergestellte Probe wird anschließend mittels spektroskopischer Verfahren untersucht (z.B. mittels $^1$H-NMR, $^{13}$C-NMR, $^{15}$N-NMR, $^{29}$Si-NMR und $^{31}$P-NMR sowie IR).

Signale, die sich nicht einem Silan, beispielsweise dem gamma-Methacryloxypropylsilylrest, zuordnen lassen, werden organischen Oberflächenmodifikationen zugeordnet, die nicht auf Silanen basieren, z.B. Oberflächenmodifikationen mittels organischer Verbindungen auf Oberflächen von Ytterbiumfluorid- bzw. Bariumsulfatpartikeln.

Die Anteile organisch oberflächenmodifizierter Partikel bzw. nicht organisch oberflächenmodifizierter Partikel können regelmäßig auch durch Auswertung der Intensitäten entsprechender Schwingungsbanden im IR-Spektrum bestimmt werden. Hierzu werden üblicherweise Referenz-Schwingungsbanden (Referenzkurven) organisch oberflächenmodifizierter bzw. nicht organisch oberflächenmodifizierter Partikel mit den entsprechenden chemischen Zusammensetzungen herangezogen.

I.g Alternative Charakterisierung mittels Bildanalyse und Ramanspektroskopie:

Dem Fachmann sind zusätzliche Verfahren bzw. Kopplungen von Verfahren bekannt, die eine qualitative und quantitative Charakterisierung der Füllstoffpartikel erlauben. Insoweit sei beispielsweise verwiesen auf den Artikel "Chemische Identität einzelner Partikel" von Deborah Huck-Jones und Renate Hessemann in "Nachrichten aus der Chemie", Volume 62, September 2014, Seiten 886 und 887. Die darin offenbarte Kombination von Bildanalyse und Ramanspektroskopie eignet sich regelmäßig auch zur Charakterisierung der Füllstoffpartikel im Rahmen der vorliegenden Erfindung. Dies gilt insbesondere für Proben, die nach der oben beschriebenen Harzfüllstofftrennung erhalten werden. Eine geeignete Bildanalyse ist z.B. auch die oben im Text beschriebene TEM-Analyse.

II. Härtbare Dentalmaterialien:

II.a Zusammensetzung erfindungsgemäßer und nicht erfindungsgemäßer härtbarer Dentalmaterialien:

Alle Angaben in Tabelle 1 sind Gew.-%-Angaben. Die Bezeichnung "Ref" bezieht sich auf nicht-erfindungsgemäße Zusammensetzungen härtbarer Dentalmaterialien.

Tabelle 1

| | Startmaterial | A (Ref) | B | C | D | E (Ref) | F | G | H |
|---|---|---|---|---|---|---|---|---|---|
| (A) | UDMA | 10,74 | 10,74 | 10,74 | 10,74 | 10,74 | 10,74 | 10,74 | 10,74 |
| | TCD-DMA | 10,74 | 10,74 | 10,74 | 10,74 | 10,74 | 10,74 | 10,74 | 10,74 |
| (C) | TinuvinP | 0,09 | 0,09 | 0,09 | 0,09 | 0,09 | 0,09 | 0,09 | 0,09 |
| | MeHQ | 0,02 | 0,02 | 0,02 | 0,02 | 0,02 | 0,02 | 0,02 | 0,02 |
| | CQ | 0,06 | 0,06 | 0,06 | 0,06 | 0,06 | 0,06 | 0,06 | 0,06 |
| | DABE | 0,10 | 0,10 | 0,10 | 0,10 | 0,10 | 0,10 | 0,10 | 0,10 |
| (B) | Dentalglas 1 (1,5 $\mu$m) | 50,00 | 50,00 | 50,00 | 50,00 | -- | -- | -- | -- |
| | Dentalglas 1 (0,7 $\mu$m) | 16,66 | 16,66 | 16,66 | 16,66 | -- | -- | -- | -- |
| | Dentalglas 2 (1,5 $\mu$m) | -- | -- | -- | -- | 50,00 | 50,00 | 50,00 | 50,00 |
| | Dentalglas 2 (0,7 $\mu$m) | -- | -- | -- | -- | 16,66 | 16,66 | 16,66 | 16,66 |
| | Ytterbiumfluorid | 11,59 | 11,59 | 11,59 | 11,59 | 0 | 0 | 0 | 0 |
| | Bariumsulfat | 0 | 0 | 0 | 0 | 11,59 | 11,59 | 11,59 | 11,59 |

Die Abkürzung "UDMA" ist wie oben bereits erläutert die chemische Verbindung 7,7,9-Trimethyl-4,13-dioxo-5,12-diazahexadecan-1,16-dioxydimethacrylat.

Die Abkürzung "TCD-DMA" bezeichnet die chemische Verbindung Bis(methacryloyloxymethyl)tricyclo[5.2.10^{2,6}]decan.

Die Abkürzung "MeHQ" ist wie oben bereits erläutert die chemische Verbindung Hydrochinonmonomethylether.

Die Abkürzung "CQ" bezeichnet die chemische Verbindung Campherchinon.

Die Abkürzung "DABE" bezeichnet die chemische Verbindung Ethyl-*p-N,N*-dimethylaminobenzoat.

Die härtbaren Dentalmaterialien A bis H unterscheiden sich wie folgt:

Die härtbaren Dentalmaterialien A bis D umfassen nicht-aggregierte, nicht-agglomerierte, organisch oberflächenmodifizierte Füllstoffpartikel aus Ytterbiumfluorid. Diese Füllstoffpartikel unterscheiden sich hinsichtlich ihrer Partikelgrößen wie folgt (bei den Angaben zu D10, D50 und D90 handelt es sich um volumenbezogene Partikelgrößen in nm):

- nicht-erfindungsgemäßes härtbares Dentalmaterial A:
  Partikelgrößenverteilung: D10: 12 nm, D50: 15 nm, D90: 20 nm

- erfindungsgemäßes härtbares Dentalmaterial B:
  Partikelgrößenverteilung: D10: 26 nm, D50: 30 nm, D90: 39 nm

- erfindungsgemäßes härtbares Dentalmaterial C:
  Partikelgrößenverteilung: D10: 38 nm, D50: 50 nm, D90: 65 nm

- erfindungsgemäßes härtbares Dentalmaterial D:
  Partikelgrößenverteilung: D10: 69 nm, D50: 90 nm, D90: 117 nm

Die härtbaren Dentalmaterialien E bis H umfassen nicht-aggregierte, nicht-agglomerierte, organisch oberflächenmodifizierte Füllstoffpartikel aus Bariumsulfat. Diese Füllstoffpartikel unterscheiden sich hinsichtlich ihrer Partikelgrößen wie folgt:

- nicht-erfindungsgemäßes härtbares Dentalmaterial E:
  Partikelgrößenverteilung: D10: 11 nm, D50: 15 nm, D90: 20 nm

- erfindungsgemäßes härtbares Dentalmaterial F:
  Partikelgrößenverteilung: D10: 25 nm, D50: 30 nm, D90: 40 nm

- erfindungsgemäßes härtbares Dentalmaterial G:
  Partikelgrößenverteilung: D10: 38 nm, D50: 50 nm, D90: 67 nm

- erfindungsgemäßes härtbares Dentalmaterial H:
  Partikelgrößenverteilung: D10: 68 nm, D50: 90 nm, D90: 120 nm

TinuvinP ist ein UV-Stabilisator, MeHQ ein Inhibitor, CQ ein Initiator und DABE ein Coinitiator. Der Füllstoffgehalt der härtbaren Dentalmaterialien A bis H beträgt jeweils 78,26 %.

II.b Herstellung der härtbaren Dentalmaterialien A bis H:

In einem ersten Schritt wurden für die Herstellung der einzelnen härtbaren Dentalmaterialien A bis H die polymerisierbaren Monomere der Gesamtmenge (A) unter Rühren ineinander gelöst. Es resultierte eine Harzmischung, von der anschließend der Brechungsindex "$n_A$ (Harz)" bestimmt wurde (vgl. Tabelle 2 unten).

In einem zweiten Schritt wurde die Teilmenge (B1) der partikulären Füllstoffe (das als kolloidale Lösung in Ethanol vorliegende Ytterbiumfluorid bzw. Bariumsulfat) zusammen mit der Harzmischung in einen Kolben eingewogen, die resultierende Mischung durch Rühren homogenisiert und am Rotationsverdampfer vom Lösungsmittel befreit. Anschließend wurde der Brechungsindex "Harz und YbF$_3$" bzw. "Harz und BaSO$_4$" bestimmt (vgl. Tabelle 2 unten)

In einem dritten Schritt wurden die Hilfsstoffe (C) in die homogenisierte und vom Lösungsmittel befreite Mischung, die nach Schritt 2 resultierte, unter Rühren eingetragen.

In einem vierten Schritt wurde die Teilmenge (B2) der partikulären Füllstoffe (die Dentalgläser) mittels des Mischgerätes AM 501 der Firma Hauschild in die nach dem dritten Schritt resultierende Mischung (aus den Komponenten (A), (B1) und (C)) eingearbeitet und anschließend zu einer homogenen pastösen Masse verarbeitet (4*12 s), die für 10 min bei 50 U/min und -0,85 bar in einem Laborkneter mit Balkenrührern (PC Laborsystem, Magden, CH) entlüftet wurde.

II.c Brechungsindices ausgewählter Teilmengen der härtbaren Dentalmaterialien A bis H:

Tabelle 2

|  | A (Ref) | B | C | D | E (Ref) | F | G | H |
|---|---|---|---|---|---|---|---|---|
| Brechungsindex: $n_A$ (Harz) | 1,4941 | 1,4941 | 1,4941 | 1,4941 | 1,4941 | 1,4941 | 1,4941 | 1,4941 |
| Brechungsindex: Harz und $YbF_3$ | 1,5153 | 1,5153 | 1,5153 | 1,5153 | -- | -- | -- | -- |
| Brechungsindex: Harz und $BaSO_4$ | -- | -- | -- | -- | 1,5448 | 1,5448 | 1,5448 | 1,5448 |
| Brechungsindex: $n_{B2}$ (Gläser) | 1,5300 | 1,5300 | 1,5300 | 1,5300 | 1,5500 | 1,5500 | 1,5500 | 1,5500 |

Die Brechungsindices "Harz und $YbF_3$" bzw. "Harz und $BaSO_4$" beziehen sich auf den ungehärteten Zustand. Vergleichsuntersuchungen haben gezeigt, dass sich durch Zugabe entsprechender Katalysatoren und Aushärten solcher Mischungen der Brechungsindex dieser gehärteten Mischungen noch dichter an die Brechungsindizes der dentalen Gläser heranrückt.

III. Gehärtete Dentalmaterialien:

III.a Herstellung der gehärteten Dentalmaterialien A bis H:

Bei den gehärteten Dentalmaterialien A bis H handelt es sich um die gehärteten härtbaren Dentalmaterialien A bis H.

Die gemäß Punkt II.b hergestellten härtbaren Dentalmaterialien A bis H wurden durch Bestrahlung mit blauem Licht der Wellenlänge 470 nm mit der LED-Polymerisationslampe Celalux II der Firma VOCO in die gehärteten Dentalmaterialien A bis H überführt.

Zur Herstellung der Prüfkörper zur Bestimmung der Transluzenz (Durchmesser 15 mm, Höhe 2 mm) sowie der Röntgenopazität (Durchmesser 15 mm, Höhe 1 mm) wurden Teflonformen verwendet, in die die härtbaren Dentalmaterialien eingebracht, zur Vermeidung einer Inhibitionsschicht mit PET-Folie abgedeckt und zwischen zwei Glasplatten verpresst wurden. Auf die obere Glasplatte wurde das Lichtaustrittsfenster der Polymerisationslampe aufgesetzt und ein Bereich des Materials für 10 s bestrahlt. Anschließend wurde das Lichtaustrittsfenster weiter bewegt und ein weiterer Bereich, der mit dem ersten überlappte, bestrahlt. Dieses Vorgehen wurde so lange fortgesetzt, bis der jeweilige Prüfkörper vollflächig durch Bestrahlung ausgehärtet war.

III.b Eigenschaften der hergestellten gehärteten Dentalmaterialien A bis H:

Tabelle 3

|  | A (Ref) | B | C | D | E (Ref) | F | G | H |
|---|---|---|---|---|---|---|---|---|
| Transluzenz [%] | 58,7 | 63,2 | 66,8 | 47,4 | 61,3 | 67,5 | 64,4 | 59,6 |
| Röntgenopazität [mm Al] | 4,1 | 4,1 | 4,1 | 4,1 | 3,9 | 3,9 | 3,9 | 3,9 |

IV. DVT-Messungen:

IV.a Herstellung der Prüfkörper A bis H für DVT-Messungen:

Die Prüfkörper A bis H entsprechen gehärteten Dentalmaterialien A bis H (siehe oben Punkt III.a.)

Die Prüfkörper A bis H wurden aus den entsprechenden härtbaren Dentalmaterialien hergestellt (vgl. obige Tabelle 1 und obigen Punkt II.b). In einem ersten Schritt wurde das entsprechende härtbare Dentalmaterial in eine Teflonform von 2 mm x 2 mm x 2 mm eingebracht. Die Härtung des härtbaren Dentalmaterials erfolgte gemäß obigem Punkt III.a. Nach Entnahme aus den Formen resultierten gehärtete Dentalmaterialien (jeweils ein entsprechender Prüfkörper) jeweils mit den Maßen 2 mm x 2 mm x 2 mm.

In einem nächsten Schritt wurde jeder Prüfkörper an Epoxidharz (Einbettmasse) fixiert. Jeder Prüfkörper wurde dabei jeweils einseitig am Epoxidharz fixiert.

IV.b Durchführung der DVT-Messungen:

Die DVT-Messungen wurden mit dem DVT-Gerät CS 9300 der Firma Carestream Health Inc. im Aufnahmemodus Teeth_5x5_HR_IB durchgeführt. Die Größe der 567x567x561 Voxel betrug in diesem Modus 90 μm. Die Röhrenspannung betrug 65 kV bei einem Röhrenstrom von 2 mA.

IV.c Auswertung der mittels DVT-Messungen erhaltenen DVT-Bilder:

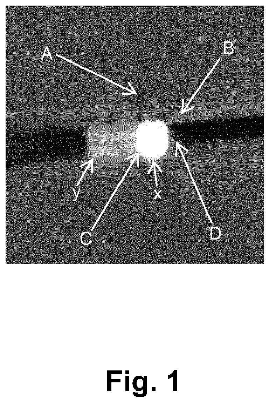

Die Auswertung der DVT-Bilder bezieht sich auf 11 bis 12 zweidimensionale Schnittbilder (Einzeltomogramme), die aus einer DVT-Messung pro Prüfkörper gewonnen wurden. Diese 11 bis 12 zweidimensionalen Schnittbilder (Einzeltomogramme) wurden hinsichtlich der nachfolgend genannten Artefakte ausgewertet. Fig. 1 zeigt beispielhaft die nachfolgend genannten Artefakte an einem zweidimensionalen Schnittbild eines Beispielprüfkörpers (in Fig. 1: x).

A: schwarze Streifen, die von den Ecken des Prüfkörpers ausgehen und in den zweidimensionalen Schnittbildern senkrecht nach oben und unten verlaufen

B: weiße Streifen, die von mindestens einer Ecke des Prüfkörpers ausgehen und in den zweidimensionalen Schnittbildern diagonal verlaufen

C: die Prüfkörper erscheinen in den zweidimensionalen Schnittbildern nicht rechteckig, sondern mit nach außen gewölbten Kanten

D: weiße Streifen rechts und/oder links der Prüfkörper (ähnlich einem Kometenschweif)

In Fig. 1 bedeutet y: zur Fixierung des Prüfkörpers x eingesetztes Harz (Fixierharz).

Bei der Auswertung der zweidimensionalen Schnittbilder wurden in jedem Schnittbild geprüft, (i) ob eines der oben genannten Artefakte identifiziert werden konnte und (ii) mit welcher Intensität es auftrat. Nachfolgend haben wir die Auswertung der zweidimensionalen Schnittbilder für die Prüfkörper der gehärteten Dentalmaterialien A bis H in Tabellen zusammengefasst. Die Symbole in den nachfolgenden Tabellen haben die folgende Bedeutung:

- nicht sichtbar

o kaum wahrnehmbar
+ sichtbar
++ deutlich sichtbar
n.a. keine Daten gemessen

Im Tabellenkopf jeder der nachfolgenden Tabellen befindet sich der Hinweis, welches gehärtete Dentalmaterial untersucht wurde. In der ersten Spalte (links) sind die oben genannten Artefakte angegeben. Die Bezeichnung "A.01" (vgl. z.B. Tabelle 4) bedeutet, dass es sich um das erste der 11 bzw. 12 zweidimensionalen Schnittbilder handelt. Entsprechendes gilt für die Tabellen 5 bis 11.

Tabelle 4

| | A (Ref) (nicht-erfindungsgemäß) | | | | | | | | | | | |
|---|---|---|---|---|---|---|---|---|---|---|---|---|
| | A.01 | A.02 | A.03 | A.04 | A.05 | A.06 | A.07 | A.08 | A.09 | A.10 | A.11 | A.12 |
| A | o | ++ | + | ++ | ++ | + | ++ | ++ | + | ++ | + | ++ |
| B | ++ | o | + | ++ | o | o | + | - | - | - | o | - |
| C | ++ | ++ | ++ | ++ | ++ | ++ | ++ | ++ | ++ | ++ | ++ | ++ |
| D | o | + | + | o | + | + | o | + | + | o | + | ++ |

Tabelle 5

| | B (erfindungsgemäß) | | | | | | | | | | | |
|---|---|---|---|---|---|---|---|---|---|---|---|---|
| | B.01 | B.02 | B.03 | B.04 | B.05 | B.06 | B.07 | B.08 | B.09 | B.10 | B.11 | B.12 |
| A | o | o | + | o | o | - | + | o | - | o | o | o |
| B | - | + | + | o | o | o | o | + | o | o | o | o |
| C | + | + | + | + | + | + | + | + | + | + | + | + |
| D | - | o | - | - | - | - | - | - | o | o | - | + |

Tabelle 6

| | C (erfindungsgemäß) | | | | | | | | | | | |
|---|---|---|---|---|---|---|---|---|---|---|---|---|
| | C.01 | C.02 | C.03 | C.04 | C.05 | C.06 | C.07 | C.08 | C.09 | C.10 | C.11 | n.a. |
| A | - | o | o | o | - | o | o | - | o | o | - | n.a. |
| B | - | o | o | o | o | o | o | o | o | + | o | n.a. |
| C | + | + | + | + | + | + | + | + | + | + | + | n.a. |
| D | - | o | o | - | - | o | - | - | - | - | o | n.a. |

Tabelle 7

| | D (erfindungsgemäß) | | | | | | | | | | | |
|---|---|---|---|---|---|---|---|---|---|---|---|---|
| | D.01 | D.02 | D.03 | D.04 | D.05 | D.06 | D.07 | D.08 | D.09 | D.10 | D.11 | D.12 |
| A | - | o | o | o | o | o | o | o | - | o | o | - |
| B | + | o | o | + | o | o | + | + | - | - | o | o |
| C | + | + | + | + | + | + | + | + | + | + | + | + |
| D | - | - | o | - | - | o | - | o | o | o | o | o |

Tabelle 8

| | E (Ref) (nicht-erfindungsgemäß) | | | | | | | | | | | |
|---|---|---|---|---|---|---|---|---|---|---|---|---|
| | E.01 | E.02 | E.03 | E.04 | E.05 | E.06 | E.07 | E.08 | E.09 | E.10 | E.11 | E.12 |
| A | + | + | o | + | + | + | + | + | + | ++ | + | o |
| B | o | + | + | o | o | o | o | o | o | + | + | o |
| C | + | ++ | ++ | ++ | ++ | ++ | ++ | + | ++ | ++ | ++ | + |
| D | - | - | o | - | o | o | - | o | + | o | o | + |

Tabelle 9

| | F (erfindungsgemäß) | | | | | | | | | | | |
|---|---|---|---|---|---|---|---|---|---|---|---|---|
| | F.01 | F.02 | F.03 | F.04 | F.05 | F.06 | F.07 | F.08 | F.09 | F.10 | F.11 | F.12 |
| A | - | o | - | o | o | o | + | o | - | o | - | - |
| B | + | + | o | o | + | o | o | + | o | o | o | o |
| C | + | + | + | + | + | ++ | + | + | + | + | + | + |

(fortgesetzt)

| | F (erfindungsgemäß) | | | | | | | | | | | |
|---|---|---|---|---|---|---|---|---|---|---|---|---|
| | F.01 | F.02 | F.03 | F.04 | F.05 | F.06 | F.07 | F.08 | F.09 | F.10 | F.11 | F.12 |
| D | - | - | o | - | o | o | o | o | + | - | - | o |

Tabelle 10

| | G (erfindungsgemäß) | | | | | | | | | | | |
|---|---|---|---|---|---|---|---|---|---|---|---|---|
| | G.01 | G.02 | G.03 | G.04 | G.05 | G.06 | G.07 | G.08 | G.09 | G.10 | G.11 | G.12 |
| A | o | o | o | o | - | - | - | - | - | o | - | - |
| B | + | + | + | o | + | o | + | o | o | + | - | - |
| C | + | + | + | + | + | + | + | + | + | + | + | + |
| D | - | o | - | - | o | o | - | o | o | - | o | o |

Tabelle 11

| | H (erfindungsgemäß) | | | | | | | | | | | |
|---|---|---|---|---|---|---|---|---|---|---|---|---|
| | H.01 | H.02 | H.03 | H.04 | H.05 | H.06 | H.07 | H.08 | H.09 | H.10 | H.11 | H.12 |
| A | o | - | - | o | o | o | + | + | - | - | o | o |
| B | + | + | o | o | o | o | o | o | - | - | o | o |
| C | ++ | + | + | + | + | ++ | ++ | + | + | + | + | + |
| D | o | o | o | - | - | o | o | o | o | - | o | o |

Die zweidimensionalen Schnittbilder der jeweiligen DVT-Bilder der gehärteten Dentalmaterialien A und E (nicht-erfindungsgemäß) zeigen vergleichsweise mehr, insbesondere ausgeprägtere Artefakte, verglichen mit den zweidimensionalen Schnittbildern der jeweiligen DVT-Bilder der gehärteten Dentalmaterialien B, C, D und F bis H (erfindungsgemäß), bei ansonsten identisch durchgeführten DVT-Messungen (vgl. oben Punkt IV.b).

V. Fließfähiges erfindungsgemäßes härtbares Dentalmaterial:

50 g des erfindungsgemäßen härtbaren Dentalmaterials C (vgl. Tabelle 1 oben im Text) wurden zusätzlich mit 5 g TEGDMA (Triethylenglycoldimethacrylat) und 1,5 g Aerosil R711 (Evonik) vermengt, anschließend auf dem Dreiwalzenstuhl Exakt 80 E (Exakt GmbH, Norderstedt) gewalzt und daran anschließend in einem Laborkneter mit Balkenrührern (PC Laborsystem, Magden, CH) für 10 min bei 50 U/min und -0,85 bar entlüftet. Es resultierte ein erfindungsgemäßes, unter Scherung fließfähiges härtbares Dentalmaterial ("Flow-Komposit") (härtbares Dentalmaterial J). Aus diesem fließfähigen, erfindungsgemäßen Dentalmaterial J wurden analog zu den Beispielen A - H Prüfkörper angefertigt. Für diese Prüfkörper wurde eine Transluzenz von 54,3 % bestimmt und eine Röntgenopazität von 3,6 mm Al. In der DVT wurden überwiegend artefaktarme Bilder erhalten.

**Patentansprüche**

1. Härtbares Dentalmaterial, herstellbar durch Mischen von Startmaterialien, wobei als zu mischende Startmaterialien ausschließlich eingesetzt werden:

  - ein, zwei, drei oder mehr als drei polymerisierbare Monomere, welche die Gesamtmenge (A) der polymerisierbaren Monomere im härtbaren Dentalmaterial bilden,
  - ein, zwei, drei oder mehr als drei partikuläre Füllstoffe, welche die Gesamtmenge (B) der partikulären Füllstoffe im härtbaren Dentalmaterial bilden,

sowie

- ein, zwei, drei oder mehr als drei Hilfsstoffe, welche die Gesamtmenge (C) der Hilfsstoffe im härtbaren Dentalmaterial bilden,

wobei

das härtbare Dentalmaterial eine der Gesamtmenge (B) zugehörige Teilmenge (B1) umfasst, bestehend aus der im härtbaren Dentalmaterial enthaltenen Gesamtmenge an

(a) nicht-aggregierten und nicht-agglomerierten Füllstoffpartikeln mit einer Partikelgröße im Bereich von 25 bis 120 nm aus Ytterbiumfluorid
und
(b) nicht-aggregierten und nicht-agglomerierten Füllstoffpartikeln mit einer Partikelgröße im Bereich von 25 bis 120 nm aus Bariumsulfat,

wobei

der Anteil der Teilmenge (B1)

- 2,5 Gew.-% oder größer als 2,5 Gew.-% ist, bezogen auf die Gesamtmasse des härtbaren Dentalmaterials

und

- 90 Gew.-% oder größer als 90 Gew.-% ist, bezogen auf die Gesamtmasse an Füllstoffpartikeln aus Ytterbiumfluorid und Bariumsulfat im härtbaren Dentalmaterial,

wobei die qualitative und quantitative Charakterisierung der Füllstoffpartikel erfolgt wie in der Beschreibung angegeben.

2. Härtbares Dentalmaterial nach Anspruch 1, wobei die Gesamtmenge (A) aus dem polymerisierbaren Monomer bzw. aus den polymerisierbaren Monomeren einen Brechungsindex $n_A$ im Bereich von 1,45 bis 1,55 besitzt, vorzugsweise einen Brechungsindex $n_A$ im Bereich von 1,48 bis 1,55.

3. Härtbares Dentalmaterial nach einem der vorangehenden Ansprüche, wobei der Anteil der Teilmenge (B1) in einem Bereich von 2,5 Gew.-% bis 20 Gew.-% liegt, vorzugsweise in einem Bereich von 5 Gew.-% bis 18 Gew.-%, weiter bevorzugt in einem Bereich von 10,1 Gew.-% bis 18 Gew.-%, besonders bevorzugt in einem Bereich von 10,1 Gew.-% bis 16 Gew.-%, bezogen auf die Gesamtmasse des härtbaren Dentalmaterials.

4. Härtbares Dentalmaterial nach einem der vorangehenden Ansprüche, wobei die Gesamtmenge (B) der partikulären Füllstoffe aus der Teilmenge (B1) und einer Teilmenge (B2) besteht, wobei die Gesamtmenge der partikulären Füllstoffe in der Teilmenge (B2) vorzugsweise einen Brechungsindex $n_{B2}$ im Bereich von 1,49 bis 1,62, besonders bevorzugt im Bereich von 1,50 bis 1,56 besitzt
und/oder
wobei der Anteil der Gesamtmenge (B) im Bereich von 72 bis 92 Gew-% liegt, vorzugsweise im Bereich von 75 bis 89,5 Gew.-% liegt, bezogen auf die Gesamtmasse der zu mischenden Startmaterialien.

5. Härtbares Dentalmaterial nach einem der vorangehenden Ansprüche, wobei 70 Gew.-% oder mehr, vorzugsweise 80 Gew.-% oder mehr, der partikulären Füllstoffe, welche die Gesamtmenge (B) der partikulären Füllstoffe im härtbaren Dentalmaterial bilden, organisch oberflächenmodifiziert sind, vorzugsweise umfasst das härtbare Dentalmaterial ausschließlich Füllstoffpartikel, die organisch oberflächenmodifiziert sind.

6. Härtbares Dentalmaterial nach einem der vorangehenden Ansprüche, wobei in der Teilmenge (B1), bestehend aus der im härtbaren Dentalmaterial enthaltenen Gesamtmenge an

(a) nicht-aggregierten und nicht-agglomerierten Füllstoffpartikeln mit einer Partikelgröße im Bereich von 25 bis 120 nm aus Ytterbiumfluorid
und
(b) nicht-aggregierten und nicht-agglomerierten Füllstoffpartikeln mit einer Partikelgröße im Bereich von 25 bis 120 nm aus Bariumsulfat,

nicht-aggregierte und nicht-agglomerierte Füllstoffpartikel aus Ytterbiumfluorid mit einer Partikelgröße im Bereich von 30 bis 100 nm, vorzugsweise im Bereich von 30 bis 80 nm, besonders bevorzugt im Bereich von 30 bis 60 nm, insbesondere vorzugsweise im Bereich von 35 bis 55 nm, enthalten sind
und/oder

nicht-aggregierte und nicht-agglomerierte Füllstoffpartikel aus Bariumsulfat mit einer Partikelgröße im Bereich von 30 bis 100 nm, vorzugsweise im Bereich von 30 bis 80 nm, besonders bevorzugt im Bereich von 30 bis 60 nm, insbesondere vorzugsweise im Bereich von 35 bis 55 nm, enthalten sind,
wobei die qualitative und quantitative Charakterisierung der Füllstoffpartikel erfolgt wie in der Beschreibung angegeben.

7. Härtbares Dentalmaterial nach einem der vorangehenden Ansprüche, herstellbar durch Mischen von Startmaterialien, wobei als zu mischende Startmaterialien ausschließlich eingesetzt werden:

- in einer Gesamtmenge im Bereich von 7,95 bis 27,95 Gew.-%, bezogen auf die Gesamtmasse der zu mischenden Startmaterialien, ein, zwei, drei oder mehr als drei polymerisierbare Monomere, welche die Gesamtmenge (A) der polymerisierbaren Monomere im härtbaren Dentalmaterial bilden und wobei die Gesamtmenge (A) aus dem polymerisierbaren Monomer bzw. aus den polymerisierbaren Monomeren einen Brechungsindex $n_A$ in einem Bereich von 1,45 bis 1,55 besitzt,
- in einer Gesamtmenge im Bereich von 72 bis 92 Gew-%, bezogen auf die Gesamtmasse der zu mischenden Startmaterialien, ein, zwei, drei oder mehr als drei partikuläre Füllstoffe, welche die Gesamtmenge (B) der partikulären Füllstoffe im härtbaren Dentalmaterial bilden,
- in einer Gesamtmenge im Bereich von 0,05 bis 2 Gew.-%, bezogen auf die Gesamtmasse der zu mischenden Startmaterialien, ein, zwei, drei oder mehr als drei Hilfsstoffe, welche die Gesamtmenge (C) der Hilfsstoffe im härtbaren Dentalmaterial bilden,

wobei
das härtbare Dentalmaterial eine der Gesamtmenge (B) zugehörige Teilmenge (B1) umfasst, bestehend aus der im härtbaren Dentalmaterial enthaltenen Gesamtmenge an

(a) nicht-aggregierten und nicht-agglomerierten Füllstoffpartikeln mit einer Partikelgröße im Bereich von 25 bis 120 nm aus Ytterbiumfluorid,

- wobei vorzugsweise die Teilmenge (B1), vorzugsweise zu mehr als 50 Gew.-%, nicht-aggregierte und nicht-agglomerierte Füllstoffpartikel aus Ytterbiumfluorid mit einer Partikelgröße im Bereich von 30 bis 100 nm enthält, besonders bevorzugt im Bereich von 30 bis 80 nm, insbesondere bevorzugt im Bereich von 30 bis 60 nm, ganz besonders bevorzugt im Bereich von 35 bis 55 nm

und
(b) nicht-aggregierten und nicht-agglomerierten Füllstoffpartikeln mit einer Partikelgröße im Bereich von 25 bis 120 nm aus Bariumsulfat,

- wobei vorzugsweise die Teilmenge (B1), vorzugsweise zu mehr als 50 Gew.-%, nicht-aggregierte und nicht-agglomerierte Füllstoffpartikel aus Bariumsulfat mit einer Partikelgröße im Bereich von 30 bis 100 nm enthält, besonders bevorzugt im Bereich von 30 bis 80 nm, insbesondere bevorzugt im Bereich von 30 bis 60 nm, ganz besonders bevorzugt im Bereich von 35 bis 55 nm,

wobei

- der Anteil der Teilmenge (B1)
- 2,5 Gew.-% oder größer als 2,5 Gew.-% ist, vorzugsweise in einem Bereich von 2,5 Gew.-% bis 20 Gew.-% liegt, besonders vorzugsweise in einem Bereich von 5 Gew.-% bis 18 Gew.-%, weiter bevorzugt in einem Bereich von 10,1 Gew.-% bis 18 Gew.-% liegt, besonders bevorzugt in einem Bereich von 10,1 Gew.-% bis 16 Gew.-% liegt, bezogen auf die Gesamtmasse des härtbaren Dentalmaterials

und

- 90 Gew.-% oder größer als 90 Gew.-% ist, bezogen auf die Gesamtmasse an Füllstoffpartikeln aus Ytterbiumfluorid und Bariumsulfat im härtbaren Dentalmaterial

und

- vorzugsweise die Gesamtmenge der partikulären Füllstoffe in der Teilmenge (B1) einen Brechungsindex $n_{B1}$ im Bereich von 1,55 bis 1,64 besitzt,

wobei die qualitative und quantitative Charakterisierung der Füllstoffpartikel erfolgt wie in der Beschreibung angegeben.

8. Härtbares Dentalmaterial nach einem der vorangehenden Ansprüche zur Anwendung in einem Verfahren zur chirurgischen oder therapeutischen Behandlung des menschlichen oder tierischen Körpers und/oder zur Anwendung in einem Diagnostizierverfahren, das am menschlichen oder tierischen Körper vorgenommen wird, vorzugsweise zur spezifischen Anwendung

- in einem therapeutischen Verfahren zum temporären oder permanenten Füllen einer dentalen Kavität, oder
- in einem therapeutischen Verfahren als

- Zahnfüllungsmaterial,
- Dentalzement,
- dentales Unterfüllungsmaterial,
- als fließfähiges Kompositmaterial (Flow-Material),
- als Kronenmaterial,
- als Inlay und/oder Onlay,
- als Brückenmaterial
- und/oder als Stumpfaufbaumaterial.

9. Gehärtetes Dentalmaterial, erhältlich durch Polymerisation polymerisierbarer Monomere in einem härtbaren Dentalmaterial nach einem der Ansprüche 1 bis 8.

10. Verwendung eines härtbaren Dentalmaterials nach einem der Ansprüche 1 bis 8 zur Herstellung eines dentalen Erzeugnisses, wobei die Herstellung nicht am menschlichen oder tierischen Körper erfolgt, vorzugsweise zur Herstellung eines dentalen Erzeugnisses ausgewählt aus der Gruppe bestehend aus künstlichen Zähnen, Inlays, Onlays, Kronen, Brücken, Fräsrohlingen, Implantaten und Zahnfertigteilen.

11. Verfahren zur Herstellung eines härtbaren Dentalmaterials nach einem der Ansprüche 1 bis 8 umfassend die folgenden Schritte:

(i) Herstellen oder Bereitstellen der Startmaterialien wie in einem der Ansprüche 1 bis 8 definiert, oder
Herstellen oder Bereitstellen von Teilmischungen aus den Startmaterialien wie in einem der Ansprüche 1 bis 8 definiert
(ii) Mischen der gemäß (i) hergestellten bzw. bereitgestellten Startmaterialien bzw. der gemäß (i) hergestellten bzw. bereitgestellten Teilmischungen, so dass jeweils das härtbare Dentalmaterial resultiert.

12. Kit, umfassend

- eine oder mehr als eine dentale Spritze

und

(i) ein, zwei oder mehr als zwei härtbare Dentalmaterialien nach einem der Ansprüche 1 bis 8
oder
(ii) eine, zwei oder mehr als zwei Basispasten und eine, zwei oder mehr als zwei Katalysatorpasten, wobei durch Mischen einer Basispaste und der dazugehörigen Katalysatorpaste ein härtbares Dentalmaterial nach einem der Ansprüche 1 bis 8 erhältlich ist, welches einen Polymerisationsinitiator umfasst.

13. Verwendung eines härtbaren Dentalmaterials nach einem der Ansprüche 1 bis 8 als Baumaterial in einem generativen, ein digitales Datenmodell verwendenden Fertigungsverfahren, vorzugsweise im 3D-Druck, vorzugsweise zur Herstellung eines dentalen Erzeugnisses, vorzugsweise zur Herstellung eines dentalen Erzeugnisses ausgewählt aus der Gruppe bestehend aus künstlichen Zähnen, Inlays, Onlays, Kronen, Brücken, Fräsrohlingen, Implantaten und Zahnfertigteilen.

14. Verfahren zur Herstellung eines dentalen Erzeugnisses mittels eines generativen, ein digitales Datenmodell verwendenden Fertigungsverfahrens umfassend die Schritte:

- Herstellen oder Bereitstellen eines härtbaren Dentalmaterials nach einem der Ansprüche 1 bis 8, vorzugsweise Herstellen nach einem Verfahren gemäß Anspruch 11,
- Verarbeiten des hergestellten bzw. bereitgestellten härtbaren Dentalmaterials in dem generativen, ein digitales Datenmodell verwendenden Fertigungsverfahren, so dass das dentale Erzeugnis oder eine Vorstufe des dentalen Erzeugnisses resultiert,
wobei das dentale Erzeugnis vorzugsweise ausgewählt ist aus der Gruppe bestehend aus künstlichen Zähnen, Inlays, Onlays, Kronen, Brücken, Fräsrohlingen, Implantaten und Zahnfertigteilen.

**Claims**

1. Curable dental material producible by mixing starting materials, wherein the starting materials to be mixed are exclusively:

- one, two, three or more than three polymerizable monomers which form the total amount (A) of the polymerizable monomers in the curable dental material,
- one, two, three or more than three particulate fillers which form the total amount (B) of the particulate fillers in the curable dental material,
and
- one, two, three or more than three auxiliaries which form the total amount (C) of the auxiliaries in the curable dental material,

wherein
the curable dental material comprises a component (B1) which forms part of the total amount (B), consisting of the total amount of the following that are present in the curable dental material:

(a) nonaggregated and nonagglomerated filler particles having a particle size in the range from 25 to 120 nm of ytterbium fluoride
and
(b) nonaggregated and nonagglomerated filler particles having a particle size in the range from 25 to 120 nm of barium sulfate,

wherein
the proportion of component (B1) is

- 2.5% by weight or greater than 2.5% by weight, based on the total mass of the curable dental material,
and
- 90% by weight or greater than 90% by weight, based on the total mass of filler particles of ytterbium fluoride and barium sulfate in the curable dental material,

wherein the qualitative and quantitative characterization of the filler particles is effected as specified in the description.

2. Curable dental material according to Claim 1, wherein the total amount (A) of the polymerizable monomer(s) has a refractive index $n_A$ in the range from 1.45 to 1.55, preferably a refractive index $n_A$ in the range from 1.48 to 1.55.

3. Curable dental material according to either of the preceding claims, wherein the proportion of component (B1) is within a range from 2.5% by weight to 20% by weight, preferably within a range from 5% by weight to 18% by weight, further preferably within a range from 10.1% by weight to 18% by weight, more preferably within a range from 10.1% by weight to 16% by weight, based on the total mass of the curable dental material.

4. Curable dental material according to any of the preceding claims, wherein the total amount (B) of the particulate fillers consists of component (B1) and a component (B2), wherein the total amount of the particulate fillers in component (B2) preferably has a refractive index $n_{B2}$ in the range from 1.49 to 1.62, more preferably in the range from 1.50 to 1.56,

and/or

wherein the proportion of the total amount (B) is in the range from 72% to 92% by weight, preferably in the range from 75% to 89.5% by weight, based on the total amount of the starting materials to be mixed.

5. Curable dental material according to any of the preceding claims, wherein 70% by weight or more, preferably 80% by weight or more, of the particulate fillers which form the total amount (B) of the particulate fillers in the curable dental material have been organically surface-modified, the curable dental material preferably comprising exclusively filler particles which have been organically surface-modified.

6. Curable dental material according to any of the preceding claims, wherein component (B1) consisting of the total amount of the following present in the curable dental material:

(a) nonaggregated and nonagglomerated filler particles having a particle size in the range from 25 to 120 nm of ytterbium fluoride

and

(b) nonaggregated and nonagglomerated filler particles having a particle size in the range from 25 to 120 nm of barium sulfate

includes nonaggregated and nonagglomerated filler particles of ytterbium fluoride having a particle size in the range from 30 to 100 nm, preferably in the range from 30 to 80 nm, more preferably in the range from 30 to 60 nm, especially preferably in the range from 35 to 55 nm,

and/or

includes nonaggregated and nonagglomerated filler particles of barium sulfate having a particle size in the range from 30 to 100 nm, preferably in the range from 30 to 80 nm, more preferably in the range from 30 to 60 nm, especially preferably in the range from 35 to 55 nm,

wherein the qualitative and quantitative characterization of the filler particles is effected as specified in the description.

7. Curable dental material according to any of the preceding claims, producible by mixing starting materials, wherein the starting materials to be mixed are exclusively:

- in a total amount in the range from 7.95% to 27.95% by weight, based on the total mass of the starting materials to be mixed, one, two, three or more than three polymerizable monomers which form the total amount (A) of the polymerizable monomers in the curable dental material, wherein the total amount (A) of the polymerizable monomer(s) has a refractive index $n_A$ within a range from 1.45 to 1.55,

- in a total amount in the range from 72% to 92% by weight, based on the total mass of the starting materials to be mixed, one, two, three or more than three particulate fillers which form the total amount (B) of the particulate fillers in the curable dental material,

- in a total amount in the range from 0.05% to 2% by weight, based on the total mass of the starting materials to be mixed, one, two, three or more than three auxiliaries which form the total amount (C) of the auxiliaries in the curable dental material,

wherein

the curable dental material comprises a component (B1) which forms part of the total amount (B), consisting of the total amount of the following that are present in the curable dental material:

(a) nonaggregated and nonagglomerated filler particles having a particle size in the range from 25 to 120 nm of ytterbium fluoride,

- where component (B1) preferably comprises, preferably to an extent of more than 50% by weight, nonaggregated and nonagglomerated filler particles of ytterbium fluoride having a particle size in the range from 30 to 100 nm, more preferably in the range from 30 to 80 nm, especially preferably in the range from 30 to 60 nm, most preferably in the range from 35 to 55 nm

and

(b) nonaggregated and nonagglomerated filler particles having a particle size in the range from 25 to 120 nm of barium sulfate,

- where component (B1) preferably comprises, preferably to an extent of more than 50% by weight, nonaggregated and nonagglomerated filler particles of barium sulfate having a particle size in the range from 30 to 100 nm, more preferably in the range from 30 to 80 nm, especially preferably in the range from 30 to 60 nm, most preferably in the range from 35 to 55 nm,

wherein

- the proportion of component (B1) is
- 2.5% by weight or greater than 2.5% by weight, preferably within a range from 2.5% by weight to 20% by weight, more preferably within a range from 5% by weight to 18% by weight, further preferably within a range from 10.1% by weight to 18% by weight, more preferably within a range from 10.1% by weight to 16% by weight, based on the total mass of the curable dental material
and
- 90% by weight or greater than 90% by weight, based on the total mass of filler particles of ytterbium fluoride and barium sulfate in the curable dental material
and
- the total amount of the particulate fillers in component (B1) preferably has a refractive index $n_{B1}$ in the range from 1.55 to 1.64,

wherein the qualitative and quantitative characterization of the filler particles is effected as specified in the description.

8. Curable dental material according to any of the preceding claims for use in a method for treatment of the human or animal body by surgery or therapy and/or for use in a diagnostic method practised on the human or animal body, preferably for specific use

- in a method of therapy for temporary or permanent filling of a dental cavity,
or
- in a method of therapy as

- tooth filling material,
- dental cement,
- dental lining material,
- as free-flowing composite material (flow material),
- as crown material,
- as inlay and/or onlay,
- as bridge material
- and/or as core build-up material.

9. Cured dental material obtainable by polymerizing polymerizable monomers in a curable dental material according to any of Claims 1 to 8.

10. Use of a curable dental material according to any of Claims 1 to 8 for production of a dental product, wherein the production is not effected in the human or animal body, preferably for production of a dental product selected from the group consisting of artificial teeth, inlays, onlays, crowns, bridges, mill blanks, implants and dentures.

11. Method for producing a curable dental material according to any of Claims 1 to 8, comprising the following steps:

(i) producing or providing the starting materials as defined in any of Claims 1 to 8
or
producing or providing partial mixtures of the starting materials as defined in any of Claims 1 to 8,
(ii) mixing the starting materials produced or provided in (i) or the partial mixtures produced or provided in (i), so as to result in the curable dental material in each case.

12. Kit comprising

- one or more than one dental syringe

and

(i) one, two or more than two curable dental materials according to any of Claims 1 to 8
or
(ii) one, two or more than two base pastes and one, two or more than two catalyst pastes, wherein a curable dental material according to any of Claims 1 to 8 is obtainable by mixing a base paste and the corresponding catalyst paste.

13. Use of a curable dental material according to any of Claims 1 to 8 as construction material in an additive manufacturing method that uses a digital data model, preferably in a 3D printing method, preferably for production of a dental product, preferably for production of a dental product selected from the group consisting of artificial teeth, inlays, onlays, crowns, bridges, mill blanks, implants and dentures.

14. Method for producing a dental product by means of an additive manufacturing method that uses a digital data model, comprising the steps of:

- producing or providing a curable dental material according to any of Claims 1 to 8, preferably producing by a method according to Claim 11,
- processing the curable dental material produced or provided in the additive manufacturing method that uses a digital data model, so as to result in the dental product or a precursor of the dental product, wherein the dental product is preferably selected from the group consisting of artificial teeth, inlays, onlays, crowns, bridges, mill blanks, implants and dentures.

**Revendications**

1. Matériau dentaire durcissable, pouvant être produit par mélange de matériaux de départ, dans lequel sont utilisés exclusivement en tant que matériaux de départ à mélanger :

- un, deux, trois ou plus de trois monomères polymérisables, lesquels forment la quantité totale (A) des monomères polymérisables dans le matériau dentaire durcissable,
- une, deux, trois ou plus de trois charges particulaires, lesquelles forment la quantité totale (B) des charges particulaires dans le matériau dentaire durcissable,
ainsi que
- un, deux, trois ou plus de trois adjuvants, lesquels forment la quantité totale (C) des adjuvants dans le matériau dentaire durcissable,

dans lequel
le matériau dentaire durcissable comprend une quantité partielle (B1) associée à la quantité totale (B), constituée de la quantité totale, contenue dans le matériau dentaire durcissable, de

(a) particules de charge non agrégées et non agglomérées avec une taille de particules dans la plage de 25 à 120 nm de fluorure d'ytterbium
et
(b) de particules de charge non agrégées et non agglomérées avec une taille de particules dans la plage de 25 à 120 nm de sulfate de baryum

dans lequel
le pourcentage de la quantité partielle (B1)

- est 2,5 % en poids ou supérieur à 2,5 % en poids, rapporté à la masse totale du matériau dentaire durcissable
et
- est 90 % en poids ou supérieur à 90 % en poids, rapporté à la masse totale de particules de charge de fluorure d'ytterbium et de sulfate de baryum dans le matériau dentaire durcissable,

dans lequel la caractérisation qualitative et quantitative des particules de charge s'effectue comme indiqué dans la description.

**2.** Matériau dentaire durcissable selon la revendication 1, dans lequel la masse totale (A) du monomère polymérisable ou des monomères polymérisables possède un indice de réfraction $n_A$ dans la plage de 1,45 à 1,55, de préférence un indice de réfraction $n_A$ dans la plage de 1,48 à 1,55.

**3.** Matériau dentaire durcissable selon l'une quelconque des revendications précédentes, dans lequel le pourcentage de la quantité partielle (B1) se situe dans une plage de 2,5 % en poids à 20 % en poids, de préférence dans une plage de 5 % en poids à 18 % en poids, plus préférablement dans une plage de 10,1 % en poids à 18 % en poids, de manière particulièrement préférée dans une plage de 10,1 % en poids à 16 % en poids, rapporté à la masse totale du matériau dentaire durcissable.

**4.** Matériau dentaire durcissable selon l'une quelconque des revendications précédentes, dans lequel la masse totale (B) des charges particulaires est constituée de la quantité partielle (B1) et d'une quantité partielle (B2), dans lequel la quantité totale des charges particulaires dans la quantité partielle (B2) possède de préférence un indice de réfraction $n_{B2}$ dans la plage de 1,49 à 1,62, de manière particulièrement préférée dans la plage de 1,50 à 1,56 et/ou
dans lequel le pourcentage de la quantité totale (B) se situe dans la plage de 72 à 92 % en poids, de préférence dans la plage de 75 à 89,5 % en poids, rapporté à la masse totale des matériaux de départ à mélanger.

**5.** Matériau dentaire durcissable selon l'une quelconque des revendications précédentes, dans lequel 70 % en poids ou plus, de préférence 80 % en poids ou plus, des charges particulaires, lesquelles forment la masse totale (B) des charges particulaires dans le matériau dentaire durcissable, sont à surface modifiée organiquement, de préférence le matériau dentaire durcissable comprend exclusivement des particules de charge qui sont à surface modifiée organiquement.

**6.** Matériau dentaire durcissable selon l'une quelconque des revendications précédentes, dans lequel, dans la quantité partielle (B1), constituée de la masse totale contenue dans le matériau dentaire durcissable, de

(a) particules de charge non agrégées et non agglomérées avec une taille de particules dans la plage de 25 à 120 nm de fluorure d'ytterbium
et
(b) particules de charge non agrégées et non agglomérées avec une taille de particules dans la plage de 25 à 120 nm de sulfate de baryum,

des particules de charge de fluorure d'ytterbium non agrégées et non agglomérées avec une taille de particules dans la plage de 30 à 100 nm, de préférence dans la plage de 30 à 80 nm, de manière particulièrement préférée dans la plage de 30 à 60 nm, en particulier de préférence dans la plage de 35 à 55 nm, sont contenues
et/ou
des particules de charge de sulfate de baryum non agrégées et non agglomérées avec une taille de particules dans la plage de 30 à 100 nm, de préférence dans la plage de 30 à 80 nm, de manière particulièrement préférée dans la plage de 30 à 60 nm, en particulier de préférence dans la plage de 35 à 55 nm, sont contenues
dans lequel la caractérisation qualitative et quantitative des particules de charge s'effectue comme indiqué dans la description.

**7.** Matériau dentaire durcissable selon l'une quelconque des revendications précédentes, pouvant être produit par mélange de matériaux de départ, dans lequel sont utilisés exclusivement comme matériaux de départ à mélanger :

- dans une quantité totale dans la plage de 7,95 à 27,95 % en poids, rapporté à la masse totale des matériaux de départ à mélanger, un, deux, trois ou plus de trois monomères polymérisables, lesquels forment la quantité totale (A) des monomères polymérisables dans le matériau dentaire durcissable et dans lequel la quantité totale (A) du monomère polymérisable ou des monomères polymérisables possède un indice de réfraction $n_A$ dans une plage de 1,45 à 1,55,
- dans une quantité totale dans la plage de 72 à 92 % en poids, rapporté à la masse totale des matériaux de départ à mélanger, une, deux, trois ou plus de trois charges particulaires, lesquelles forment la quantité totale (B) des charges particulaires dans le matériau dentaire durcissable,
- dans une quantité totale dans la plage de 0,05 à 2 % en poids, rapporté à la masse totale des matériaux de départ à mélanger, un, deux, trois ou plus de trois adjuvants, lesquels forment la quantité totale (C) des adjuvants dans le matériau dentaire durcissable,

dans lequel
le matériau dentaire durcissable comprend une quantité partielle (B1) associée à la quantité totale (B), constituée de la quantité totale, contenue dans le matériau dentaire durcissable, de

(a) particules de charge non agrégées et non agglomérées avec une taille de particules dans la plage de 25 à 120 nm de fluorure d'ytterbium,

- dans lequel de préférence la quantité partielle (B1) contient, de préférence à plus de 50 % en poids, des particules de charge non agrégées et non agglomérées de fluorure d'ytterbium avec une taille de particules dans la plage de 30 à 100 nm, de manière particulièrement préférée dans la plage de 30 à 80 nm, en particulier de préférence dans la plage de 30 à 60 nm, mieux encore dans la plage de 35 à 55 nm, et

(b) particules de charge non agrégées et non agglomérées avec une taille de particules dans la plage de 25 à 120 nm de sulfate de baryum,

- dans lequel de préférence la quantité partielle (B1) contient, de préférence à plus de 50 % en poids, des particules de charge non agrégées et non agglomérées de sulfate de baryum avec une taille de particules dans la plage de 30 à 100 nm, de manière particulièrement préférée dans la plage de 30 à 80 nm, en particulier de préférence dans la plage de 30 à 60 nm, mieux encore dans la plage de 35 à 55 nm, dans lequel
- le pourcentage de la quantité partielle (B1)
- est 2,5 % en poids ou supérieur à 2,5 % en poids, de préférence dans une plage de 2,5 % en poids à 20 % en poids, de manière particulièrement préférée dans une plage de 5 % en poids à 18 % en poids, plus préférablement dans une plage de 10,1 % en poids à 18 % en poids, en particulier de préférence dans une plage de 10,1 % en poids à 16 % en poids, rapporté à la masse totale du matériau dentaire durcissable et
- est 90 % en poids ou supérieur à 90 % en poids, rapporté à la masse totale de particules de charge de fluorure d'ytterbium et de sulfate de baryum dans le matériau dentaire durcissable et
- de préférence la quantité totale des charges particulaires dans la quantité partielle (B1) possède un indice de réfraction $n_{B1}$ dans la plage de 1,55 à 1,64, dans lequel la caractérisation qualitative et quantitative des particules de charge s'effectue comme indiqué dans la description.

8. Matériau dentaire durcissable selon l'une quelconque des revendications précédentes à utiliser dans un procédé pour le traitement chirurgical ou thérapeutique du corps humain ou animal et/ou à utiliser dans un procédé de diagnostic, qui est réalisé sur le corps humain ou animal, de préférence à utiliser de manière spécifique

- dans un procédé thérapeutique pour le remplissage temporaire ou permanent d'une cavité dentaire, ou
- dans un procédé thérapeutique en tant que

-- matériau d'obturation dentaire,
-- ciment dentaire,
-- matériau de fond de cavité,
-- en tant que matériau composite coulant (matériau fluide),
-- en tant que matériau de couronne,
-- en tant qu'inlay et/ou onlay,
-- en tant que matériau de bridge
-- et/ou en tant que matériau de reconstitution coronaire.

9. Matériau dentaire durci, pouvant être obtenu par polymérisation de monomères polymérisables dans un matériau dentaire durcissable selon l'une quelconque des revendications 1 à 8.

10. Utilisation d'un matériau dentaire durcissable selon l'une quelconque des revendications 1 à 8 pour la production d'un produit dentaire, dans laquelle la production ne s'effectue pas sur le corps humain ou animal, de préférence pour la production d'un produit dentaire choisi dans le groupe constitué des dents artificielles, inlays, onlays, couronnes, bridges, ébauches à fraiser, implants et pièces finies dentaires.

11. Procédé pour fabriquer un matériau dentaire durcissable selon l'une quelconque des revendications 1 à 8 comprenant les étapes suivantes :

(i) la production ou fourniture des matériaux de départ comme défini dans une des revendications 1 à 8, ou
la production ou fourniture de mélanges partiels composés des matériaux de départ comme défini dans une des revendications 1 à 8
(ii) le mélange des matériaux de départ produits ou fournis selon (i) ou des mélanges partiels produits ou fournis selon (i), de sorte qu'il en résulte respectivement le matériau dentaire durcissable.

12. Kit, comprenant

- une ou plus d'une seringue dentaire

et

(i) un, deux ou plus de deux matériaux dentaires durcissables selon l'une quelconque des revendications 1 à 8 ou
(ii) une, deux ou plus de deux pâtes de base et une, deux ou plus de deux pâtes de catalyseur, dans lequel, par mélange d'une pâte de base et de la pâte de catalyseur associée, un matériau dentaire durcissable selon l'une quelconque des revendications 1 à 8 peut être obtenu, lequel comprend un initiateur de polymérisation.

13. Utilisation d'un matériau dentaire durcissable selon l'une quelconque des revendications 1 à 8 en tant que matériau de construction dans un procédé de fabrication générative utilisant un modèle de données numériques, de préférence dans l'impression 3D, de préférence pour la production d'un produit dentaire, de préférence pour la production d'un produit dentaire choisi dans le groupe constitué des dents artificielles, inlays, onlays, couronnes, bridges, ébauches à fraiser, implants et pièces finies dentaires.

14. Procédé pour produire un produit dentaire au moyen d'un procédé de fabrication générative utilisant un modèle de données numériques, comprenant les étapes :

- de production ou fourniture d'un matériau dentaire durcissable selon l'une quelconque des revendications 1 à 8, de préférence de production d'après un procédé selon la revendication 11,
- de traitement du matériau dentaire durcissable produit ou fourni dans le procédé de fabrication générative utilisant un modèle de données numériques, de sorte qu'il en résulte le produit dentaire ou un premier stade du produit dentaire,

dans lequel le produit dentaire est choisi de préférence dans le groupe constitué des dents artificielles, inlays, onlays, couronnes, bridges, ébauches à fraiser, implants et pièces finies dentaires.

# Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 8201006 A1
- DE 3502594 A1
- DE 19617931 A1
- DE 102004017124 A1
- EP 2436363 A2
- DE 2121480 A1
- DE 4111914 A1
- EP 0685454 A1
- US 2012082954 A1
- DE 2458380 A1
- DE 2347591 A1
- US 3801344 A
- US 3808170 A
- EP 0011735 A2
- EP 0189540 A2
- EP 2548546 A1
- WO 2005011621 A1
- DE 3609038 A1
- DE 19846556 A1
- US 8476338 B2
- DE 2419887 A1
- DE 2406557 A1
- DE 2931926 A1
- DE 3522005 A1
- DE 3522006 A1
- DE 3703120 A1
- DE 102005021332 A1
- DE 102005053775 A1
- DE 102006060983 A1
- DE 69935794 T2
- DE 102007034457 A1
- DE 19903177 A1
- DE 4416857 C1
- EP 0980682 B1
- EP 0948955 A1
- DE 102006019092 A1
- DE 3941629 C2
- DE 60116142 T2
- EP 1720506 A1
- EP 0783880 B1
- DE 10119831 A1
- EP 1563821 A1
- EP 1839640 A2
- DE 1495520 A1
- WO 02092021 A1
- WO 02092023 A1
- EP 0059451 A1

**In der Beschreibung aufgeführte Nicht-Patentliteratur**

- **R. SCHULZE et al.** Artefacts in CBCT: a review. *Dentomaxillofacial Radiology,* 2011, vol. 40, 265-273
- **RIWOTZKI et al.** Liquid-Phase Synthesis of Doped Nanoparticles: Colloids of Luminescing LaPO4:Eu and CePO4:Tb Particles with a Narrow Particle Size Distribution. *J. Phys. Chem. B,* 2000, vol. 104, 2824-2828
- **DEBORAH HUCK-JONES ; RENATE HESSEMANN.** Chemische Identität einzelner Partikel. *Nachrichten aus der Chemie,* September 2014, vol. 62, 886, , 887